# EUROPEAN PATENT APPLICATION

(11) **EP 4 592 382 A1**
(43) Date of publication of application: **30.07.2025**
(21) Application number: 23868585.3
(22) Date of filing: 20.09.2023
(51) Int. Cl.: C12N 5/071, C12N 5/077, A61K 35/28, A61K 35/38, A61P 1/04, A61K 47/42, A61K 47/34

(54) **HIGH-PERFORMANCE MATURE INTESTINAL ORGANOID REGENERATIVE THERAPEUTIC AGENT DERIVED FROM PLURIPOTENT STEM CELLS**

(30) Priority: 21.09.2022 KR 20220119270
(71) Applicant: Korea Research Institute of Bioscience and Biotechnology, Daejeon 34141 (KR)
(72) Inventor: SON, Mi-Young, Daejeon 34141 (KR); LEE, Hana, Daejeon 34141 (KR); CHOI, Eunho, Daejeon 34141 (KR); PARK, Sohee, Daejeon 34141 (KR); HUH, Yubin, Daejeon 34141 (KR)
(74) Representative: Symbiosis IP Limited
(86) International application number: PCT/KR2023/014267
(87) International publication number: WO 2024/063533

(57) **Abstract**

The present disclosure relates to a high-performance mature human intestinal organoid regenerative therapeutic agent derived from pluripotent stem cells. The high-performance mature human intestinal organoid regenerative therapeutic agent derived from pluripotent stem cells according to the present disclosure includes peristromal cell layers containing mesenchymal stromal cells, derived from the intestine, as the microenvironment around the intestine. That is, the high-performance mature human intestinal organoid regenerative therapeutic agent derived from pluripotent stem cells according to the present disclosure can exhibit high engraftment ability when transplanted together with peripheral cell layers around intestinal organoids and can exhibit superior regenerative ability through intestinal stem cells present in mature intestinal organoids, and therefore is likely to be useful as a regenerative therapeutic agent.

## Description

### [Technical Field]

The present disclosure relates to a high-performance mature intestinal organoid derived from pluripotent stem cells, comprising stromal cells around an intestinal organoid (hereinafter, also referred to as peripheral stromal cells or peristromal cells) and a peristromal cell layer while resembling an adult intestine through a maturation process, a method for producing the same, and use thereof.

### [Background Art]

The development of efficient large-scale organoid culture techniques can be integrated into various applications, including disease modeling, drug efficacy test, and organ replacement therapy. Organoids serve as a powerful model for personalized medicine and are expected to be more effective in testing the safety and efficacy of new drugs than two-dimensionally created cell tissues, which facilitates the selection of the most appropriate treatment for each patient or individual. Further, for therapeutic purposes, the organoids may be transplanted into organs that are damaged by disease or underdeveloped properly to improve their condition. The technology for producing these organoids is theoretically known to be capable of producing almost any type of organ using only stem cells. As a result, it has significant potential for treating various diseases, leading to an accelerated advancement of research in the field of regenerative medicine.

In 2009, Hans Clevers' laboratory became the first to successfully culture isolated intestinal stem cells in three dimensions using Matrigel, and demonstrated the ability of intestinal stem cells to self-form intestinal organoids having an intestinal crypt-villus structure similar to the in vivo intestine structure. In other words, it is known that normal adult or patient (tissue)-derived intestinal organoids have been successfully delivered to mice with experimental colitis, and that there are partial areas where cells are attached to the epithelium and integrated. In these studies, intestinal organoid growth and transplantation relied on the use of animal-derived matrices (e.g., Matrigel) for proliferation.

However, the most commonly used Matrigel^{®} for the establishment of in vitro organoid cultures is a gel derived from mouse sarcoma extract. This means that due to batch-to-batch variability and undefined composition of animal-derived matrices such as matrigel, the use of matrigel in humans is very limited. In particular, in order to transfer intestinal organoid transplantation therapies to human patients, several aspects of intestinal organoid culture need to be modified for clinical use. This means that it can be difficult or impossible to obtain regulatory approval due to its inability to be used in a clinical translational application.

Thus, a number of efforts are being made to develop xeno-free (i.e., free of animal-derived components) support matrices and culture media that are approved for human use and capable of achieving proliferation. In this regard, some success of this approach has been shown for proliferation and organoid formation from mouse cells, but its application to humans is still not sufficient.

In other words, eliminating the use of gels with undefined arbitrary media components for organoid establishment would be essential for clinical applications such as regenerative medicine, precision medicine, drug trials, or patient stratification.

Meanwhile, a method for producing mini-intestinal organs or intestinal tissues in vitro has been recently developed by producing three-dimensional human intestinal organoids (hIOs) using directed differentiation protocols from human pluripotent stem cells (hPSCs), such as human embryonic stem cells (hESCs) and human induced pluripotent stem cells (hiPSCs). However, it is very common for hIOs derived from hPSCs produced in this manner to have characteristics of immature fetal small intestine with insufficient digestive function, immune function, and expression of intestinal stem cell marker genes such as OLFM4. This is thought to be due to the limited time allowed for maturation in vitro and the lack of appropriate signals around the small intestine and/or key cell types such as immune, vascular, and enteric nervous system (ENS) progenitors that are critical for intestinal maturation.

The inventors have completed the present disclosure by producing intestinal organoids derived from pluripotent stem cells matured in vitro and confirming their applicability in fields such as cell therapeutics based on their biocompatibility and therapeutic efficacy.

### [Disclosure]

### [Technical Problem]

An object of the present disclosure is to provide a pluripotent stem cell-derived intestinal organoid comprising: a stromal cell layer around an intestinal organoid.

Another object of the present disclosure is to provide a transplant material comprising: the pluripotent stem cell-derived intestinal organoid comprising the stromal cell layer around the intestinal organoid.

Still another object of the present disclosure is to provide a pharmaceutical composition comprising: the pluripotent stem cell-derived intestinal organoid comprising the stromal cell layer around the intestinal organoid.

Still another object of the present disclosure is to provide a pharmaceutical composition for preventing or treating an intestinal disease, comprising: the pluripotent stem cell-derived intestinal organoid comprising the stromal cell layer around the intestinal organoid.

Still another object of the present disclosure is to provide a method for producing a pluripotent stem cell-derived intestinal organoid comprising a stromal cell layer around an intestinal organoid.

### [Technical Solution]

Various modifications can be made and various embodiments may be implemented in the present disclosure, and specific embodiments are illustrated in the drawings and described in detail. However, it should be understood that this is not intended to limit the present disclosure to specific embodiments, and comprises all modifications, equivalents, and substitutes included in the spirit and scope of the present disclosure.

Terms used in the present application are only used to describe specific embodiments and are not intended to limit the present disclosure. Singular expressions include plural expressions unless the context clearly indicates otherwise. In the present specification, terms such as "comprise" or "have" are intended to designate the presence of features, numbers, steps, operations, components, parts, or combinations thereof described in the specification and it should not be understood as precluding the possibility of the presence or addition of one or more other features, numbers, steps, operations, components, parts, or combinations thereof.

When amounts, concentrations, or other values or parameters herein are given as ranges, preferred ranges, or lists of upper desirable values and lower desirable values, it should be understood as specifically disclosing all ranges formed by any pair of any upper range limit or preferred value and any lower range limit or preferred value, regardless of whether the scope is separately disclosed.

When ranges of numerical values are stated herein, unless otherwise stated, it is intended that the endpoints of the range and the scope of the parent invention within the range are not limited to the specific values stated when defining the range.

### Pluripotent stem cell

As used herein, the term "pluripotent stem cell (PSC)" is commonly known as a PS cell, and includes any cell that can be differentiated into almost all cells, i.e., a cell derived from any of three germ layers (reproductive epithelium) including endoderm (internal gastric wall, gastrointestinal tract, lung), mesoderm (muscle, bone, blood, genitourinary), and ectoderm (epithelial tissue and nervous system). The PSC may be derived from embryonic stem cells (including embryonic germ cells) or may be a descendant of pluripotent cells obtained by inducing non-pluripotent cells, such as adult somatic cells, by forcing expression of specific genes.

As used herein, the term "induced pluripotent stem cell (iPSC)" is commonly abbreviated as an iPS cell, and refers to a type of pluripotent stem cells that are artificially induced from normally non-pluripotent cells, such as adult somatic cells, by inducing "forced" expression of specific genes.

As used herein, the term "embryonic stem cell (ESC)" is commonly abbreviated as an ES cell, and refers to a cell derived from the inner cell mass of blastocyst, which is a pluripotent early-stage embryo. For purposes of the present disclosure, the term "ESC" is sometimes used broadly, including embryonic germ cells.

### Basal medium

A differentiation medium used in the method of the present disclosure comprises a basal medium. The basal medium is any basal medium suitable for animal or human cells, subject to the limitations provided herein.

The basal medium for animal or human cell culture typically contains a number of components necessary to support maintenance of cultured cells. Combinations of suitable components may be easily formulated by a skilled person in consideration of the following contents. The basal medium for use in the present disclosure will generally include a nutrient solution containing standard cell culture components as described in more detail above and in the document, for example, amino acids, vitamins, lipid supplements, mineral salts, carbon energy sources, and buffers. In some embodiments, the culture medium is further supplemented with one or more standard cell culture components selected from, for example, amino acids, vitamins, liquid supplements, inorganic salts, carbon energy sources, and buffers.

The skilled person will understand, from the general knowledge of the art, the type of culture medium that may be used as the basal medium in the differentiation medium of the present disclosure. The potentially suitable cell culture medium is commercially available, and include, but is not limited to, Dulbecco's modified eagle medium (DMEM), minimum essential medium (MEM), knockout-DMEM (KO-DMEM), Glasgow's minimum essential medium (G-MEM), a basal medium eagle (BME), DMEM/Ham's F12, Advanced DMEM/Ham's F12, Iscove's modified dulbecco's medium, minimum essential medium (MEM), Ham's F-10, Ham's F-12, Medium 199, RPMI 1640 medium, and KnockOut Serum replacement XenoFree medium.

For example, the basal medium may be DMEM/F12, Advanced DMEM/F12, and/or RPMI 1640.

If needed, Advanced DMEM/F12 or Advanced RPMI that is optimized for serum-free culture and already contains insulin is used.

### Differentiation (or production method) into intestinal organoid including stromal cell layer around intestinal organoid

The differentiation into intestinal organoid from pluripotent stem cells may include 1) a differentiation process from pluripotent stem cells into definitive endoderm (DE) cells, 2) a differentiation process from definitive endoderm (DE) cells into hindgut (HG), and 3) a differentiation process from hindgut (HG) into (mature) intestinal organoids.

### 1) Differentiation from pluripotent stem cells into definitive endoderm (DE) cells

One or more growth factors are used in a differentiation process from pluripotent stem cells into definitive endoderm (DE) cells.

Specifically, growth factors that may affect Activin A/nodal signaling activation or phosphatidylinositol 3-kinase (PI3K) signaling inhibition may be used.

For example, one or more growth factors used in the differentiation process may include a growth factor in TGF-β superfamily. One or more growth factors may include Nodal/Activin and/or BMP subgroup of growth factors in TGF-beta super family.

In some aspects, one or more growth factors are selected from the group consisting of Nodal, Activin A, Activin B, BMP4, Wnt3a, bFGF or any combination of these growth factors. In some aspects, the pluripotent stem cells are treated with one or more growth factors for at least 6 hours; at least 12 hours; at least 18 hours; at least 24 hours; at least 36 hours; at least 48 hours; at least 60 hours; at least 72 hours; at least 84 hours; at least 96 hours; at least 120 hours; at least 150 hours; at least 180 hours; or at least 240 hours. In some aspects, the embryonic stem cells or iPSCs are treated with one or more growth factors at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more. In some aspects, the concentration of the growth factor is maintained at a constant level during the treatment process. The concentration of the growth factor may be varied during the treatment process.

That is, the method may include a step of treating pluripotent stem cells with any one or more selected from the group consisting of Nodal, Activin A, Activin B, BMP4, bFGF, and Wnt3a to perform differentiation into definitive endoderm cells. More specifically, the method may include a step of treating embryonic stem cells or induced pluripotent stem cells with any one selected from the group consisting of Activin A, BMP4, bFGF, and Wnt3a to perform differentiation into definitive endoderm cells. More specifically, the method may include a step of treating embryonic stem cells or induced pluripotent stem cells with Activin A to perform differentiation into definitive endoderm cells.

The factors may be used, for example, in an amount of 1 ng/ml to 1,000 ng/ml, preferably 10 ng/ml to 500 ng/ml, but the present disclosure is not limited thereto.

According to one embodiment of the present disclosure, Activin A may be used to differentiate pluripotent stem cells into definitive endoderm (DE) cells. In the differentiation, for example, 10 ng/ml to 500 ng/ml of Activin A may be used, but the present disclosure is not limited thereto. More specifically, 10, 20, 30, 40, 50, 60, 70 80, 90, 100, 200, 300, 400, or 500 ng/ml of Activin A may be used. More specifically, the culturing may be performed for 6 hours to 120 hours, or 12 hours to 100 hours, but the present disclosure is not limited thereto.

According to an embodiment of the present disclosure, the growth factor is suspended in a medium containing fetal bovine serum (FBS) with varying concentrations, as needed. For example, the medium may contain any fetal bovine serum (FBS or FCS) suitable for growth, such as 0.1%, 0.2%, 0.3%, 0.5%, 1.0%, 2.0%, 3.0%, 4.0%, or 5.0%, as well as 1x B-27^{™} Supplement, serum free.

If necessary, the culturing may be performed on micropatterned substrates/plates (e.g., Aggrewell, EZSPHERE) to produce aggregates (e.g., spheroids) and may be performed in contact with the extracellular matrix (ECM). The extracellular matrix may be applied to the present differentiation method, including all of the matters described herein.

### 2) Differentiation from definitive endoderm (DE) cells to hindgut (HG)

Definitive endoderm (DE) induced by activin, preferably activin A, may be subject to a differentiation step into hindgut (HG) by FGF/Wnt signaling.

FGF and Wnt ligands may induce differentiation from DE developed in iPSC or ESC, into the hindgut through direct differentiation.

The differentiation into Hindgut or Mid-hindgut may be performed through three-dimensional culture, spheroid culture, or the like, as needed. For example, the differentiation into Hindgut or Mid-hindgut may be performed on a micropatterned substrate/plate (e.g., Aggrewell, EZSPHERE) and formed into an aggregation (e.g., spheroid) form or may be performed through three-dimensional culture (e.g., suspension) within a bioreactor. As for serum-free differentiation, B-27 may be added instead of fetal bovine serum (FBS or FCS). In addition, the culture medium may further include any one or more selected from the group consisting of Activin A, FGF, bFGF, BMP-4, LY294002 (PI3K inhibitor), CHIR99021 (GSK3 inhibitor), and Retinoic acid, but is not limited thereto.

Altering the expression of any Wnt signaling protein in combination with any FGF ligand may result in direct differentiation as described herein.

Modulators/activators of the Wnt signaling pathway include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. In some aspects, the pathway may be modulated through the use of a small molecule modulator or a protein modulator that activates the above-described pathway or a protein that activates the pathway. Examples of the small molecule modulator of the Wnt pathway include, but are not limited to, lithium chloride; 2-amino-4,6-disubstituted pyrimidine(hetero)arylpyrimidine; IQ1; QS11; NSC668036; DCA-beta catenin; and 2-amino-4-[3,4-(methylenedioxy)-benzyl-amino]-6-(3-methoxyphenyl) pyrimidine. Exemplary natural inhibitors of Wnt signaling include, but are not limited to, Dkk1, SFRP protein, and FrzB. In some aspects, exogenous molecules include, but are not limited to, small molecules such as WAY-316606; SB-216763; or BIO(6-bromoindirubin-3'-oxime).

In addition, the exogenous molecules include GSK3-inhibiting molecules or proteins that activate the Wnt signaling pathway. Exemplary GSK3 inhibitors include, but are not limited to, LY2090314, BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII (α,4-dibromoacetophenone), L803-mts (Myr-N-GKEAPPAPPQSpP-NH₂), and CHIR99021.

Specifically, the GSK3 inhibitors include Chiron/CHIR99021 that inhibits GSK3. The GSK3 inhibitor may be treated in an amount of about 0.1 uM to about 100 uM, or about 0.2 uM to about 50 uM, or about 0.3 uM to about 10 uM.

A Fibroblast growth factor (FGF) is a group of growth factors associated with vascularization, wound healing, and embryo development. In some aspects, it will be understood by one of ordinary skill in the art that any FGF may be used together with a protein derived from the Wnt signal pathway.

In some embodiments, the FGF signaling pathway is activated in contact with cells with at least one molecule selected from the group consisting of FGF1, FGF2, FGF3, FGF4, FGF10, FGF11, FGF12, FGF13, FGF14, FGF15, FGF16, FGF17, FGF18, FGF19, FGF20, FGF21, FGF22, and FGF23.

In some aspects, the DE cells are treated with one or more modulators of the signaling pathways described herein for at least 6 hours; at least 12 hours; at least 18 hours; at least 24 hours; at least 36 hours; at least 48 hours; at least 60 hours; at least 72 hours; at least 84 hours; at least 96 hours; at least 120 hours; at least 150 hours; at least 180 hours; at least 200 hours; at least 240 hours; at least 270 hours; at least 300 hours; at least 350 hours; at least 400 hours; at least 500 hours; at least 600 hours; at least 700 hours; at least 800 hours; at least 900 hours; at least 1,000 hours; at least 1,200 hours; or at least 1,500 hours.

In some aspects, the DE cells are treated with one or more molecules of the FGF signaling pathway described herein at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more.

In some aspects, the concentration of signaling molecules remains constant during the course of treatment. In another aspect, the concentration of molecules in the signaling pathway is varied during the course of the treatment.

In some aspects, the signaling molecules according to the invention are suspended in a DMEM medium.

In some aspects, the signaling molecules according to the invention are suspended in a medium containing fetal bovine serum (fetal bovine serum, FBS or FCS).

Any known molecules of the signal pathway described herein, including any molecules of the Wnt and FGF signaling pathways, are applicable alone or in combination.

In other words, the method includes a step of differentiating the definitive endoderm cells into hindgut by treatment with FGF and Wnt ligands. More specifically, the method includes a step of differentiating the definitive endoderm cells into hindgut by treatment with FGF and CHIR99021.

According to an embodiment of the present disclosure, the differentiation from the definitive endoderm (DE) into the hindgut may be performed through the GSK3 inhibitor and the fibroblast growth factor. More specifically, the differentiation into the hindgut may be performed through three-dimensional culture by treatment with CHIR99021 and FGF4.

In the differentiation, for example, 100 ng/ml, 120 ng/ml, 150 ng/ml, 200 ng/ml, 500 ng/ml, 1,000 ng/ml; or 1,200 ng/ml of FGF4 may be used, but the present disclosure is not limited thereto. Specifically, FGF4 may be used at a concentration of 100 ng/ml to 1,200 ng/ml, more specifically, 120 to 1,000 ng/ml.

In addition, CHIR99021 may be used in an amount of about 0.3 uM to about 10 uM. As needed, it is suspended in a medium containing fetal bovine serum (FBS). For example, the medium may contain any fetal bovine serum (FBS or FCS) suitable for growth, such as 0.1, 0.2, 0.3, 0.5, 1.0, 2.0, 3.0, 4.0, or 5.0%.

More specifically, the medium may be cultured for 24 hours or longer; 36 hours or longer; 48 hours or longer; 60 hours or longer; 72 hours or longer; 84 hours or longer; 96 hours or longer; 120 hours or longer; 150 hours or longer; 180 hours or longer, but the present disclosure is not limited thereto. Preferably, the medium may be cultured for 24 hours to 360 hours, and more preferably for 36 hours to 240 hours.

If needed, the medium may be cultured in contact with the extracellular matrix (ECM). The extracellular matrix may be applied to the present differentiation method, including all of the matters described herein.

### 3-1) Differentiation from hindgut (HG) into intestinal organoid

Differentiation from hindgut (HG) to intestinal organoid essentially includes BMP inhibitors, Wnt agonists, and receptor tyrosine kinase ligands. In addition, one or more additional components selected from the group consisting of ROCK inhibitors, B27, N-acetylcysteine, N2, nicotinamide, Gastrin, IGF-1, heregulin-1β, bFGF, A-83-01, and/or SB202190 may be included.

The BMP inhibitor is an agent that binds to a BMP molecule to form a complex. The inhibitor may be an agent that binds to the BMP receptor and prevents binding of the BMP ligand to the receptor, such as an antibody that binds to the receptor. The BMP inhibitor may be a protein or a small molecule and may be natural, modified, and/or partially or entirely synthetic. The BMP inhibitor may be Noggin, DAN, or a DAN-like protein including Cerberus and Gremlin (R&D systems). A preferred BMP inhibitor is Noggin. Noggin may be used at any suitable concentration. The culture medium may include about 10 ng/ml to about 100 ng/ml of Noggin. For example, the culture medium may include about 10 ng/ml, 20 ng/ml 30 ng/ml, 40 ng/ml, or 50 ng/ml or more of Noggin. Preferably, the culture medium may include approximately 10 to 100 ng/ml of Noggin.

The Wnt agonist may preferably be R-spondin 1, R-spondin 2, R-spondin 3 or R-spondin 4. The culture medium may include the Wnt agonist at a concentration of 50 ng/ml, 100 ng/ml, 200 ng/ml, 300 ng/ml, 500 ng/ml, 600 ng/ml, 700 ng/ml, 800 ng/ml, 900 ng/ml, 1 ug/ml, 1.5 ug/ml, or 2 ug/ml or more. Preferably, the culture medium may contain approximately 50 to 800 ng/ml of R-spondin 1.

The receptor tyrosine kinase ligand is, for example, a mitogenic growth factor selected from the group consisting of an epidermal growth factor (EGF), a transforming growth factor-alpha (TGF-alpha), a basal fibroblast growth factor (bFGF), a brain-derived neurotrophic factor (BDNF), neuregulin (NRG 1-4), a hepatocyte growth factor (HGF), and keratinocyte growth factor (KGF).

Preferably, the receptor tyrosine kinase ligand is the EGF. The EGF is a potent mitogenic factor for a variety of cultured ectodermal and mesodermal cells and has sufficient effects on differentiation of certain cells in vivo and in vitro and some fibroblasts in cell cultures. Preferred concentrations thereof are at least 10, 20, 25, 30, 40, 45, 50 ng/ml, 100 ng/ml, 200 ng/ml, 300 ng/ml, 500 ng/ml, 600 ng/ml, and 700 ng/ml. More preferred concentrations are more than or equal to 50 ng/ml and less than or equal to 300 ng/ml.

In addition to the essential composition of the BMP inhibitor, the WNT agonist, and the receptor tyrosine kinase ligand as described above, additional factors may be interpreted as further including addition of any known factor of the intestinal organoid. Preferably, depending on the purpose of differentiation, one or more additional components selected from the group consisting of ROCK inhibitors, B27, N-acetylcysteine, N2, nicotinamide, Gastrin, IGF-1, heregulin-1β, bFGF, A-83-01, and SB202190 may be further included.

The ROCK inhibitor is preferably selected from R-(+)-trans-4-(1-aminoethyl)-N-(4-pyridyl)cyclohexanecarboxamide dihydrochloride monohydrate (Y-27632, Sigma-Aldrich), 5-(1,4-diazepan-1-ylsulfonyl)isoquinoline (fasudil or HA1077, Cayman Chemical), and (S)-(+)-2-methyl 1-[(4-methyl-5-isoquinolinyl)sulfonyl]-hexahydro-1H-1,4-diazepinedihydrochloride (H-1152, Tocris Bioscience).

B27, N-acetylcysteine, N2, nicotinamide, Gastrin, IGF-1, heregulin-1β, bFGF, A-83-01, and/or SB202190 may be added as needed, for example, to improve the culture efficiency and lifespan of organoids, to modulate the proliferation of cells, to aid in the stability of DNA, etc.

Preferably, B27 may be further included. More specifically, the B27 supplement is referred to as "B27 supplement minus vitamin A" (also referred to herein as "B27 without vitamin A" or "B27 wo VitA"; and it may be obtained in Invitrogen (Located in Carlsbad, Califonia, USA); www.invitrogen.com; current catalog number 12587010; PAA Laboratories GmbH (Located in Pasching, Austria); www.paa.com; catalog number F01-002; and document [Brewer et al. (1993) J Neurosci Res.35(5):567-76]). In some embodiments, the B27 supplement may be replaced with a generic formulation including one or more of the components selected from the following list: biotin, cholesterol, linoleic acid, linolenic acid, progesterone, putrescine, retinyl acetate, sodium selenite, tri-iodothyronine (T3), DL-alpha-tocopherol (vitamin E), albumin, insulin, and transferrin.

If necessary, the culturing may be performed in contact with the extracellular matrix (ECM) during differentiation from the Hindgut into the intestinal organoid.

In some embodiments, the extracellular matrix is a three-dimensional matrix. In some embodiments, the hindgut is embedded in the extracellular matrix. The culture medium of the present disclosure may diffuse into the three-dimensional extracellular matrix.

Matters regarding the ECM disclosed in documents [Polymer Hydrogels to Guide Organotypic and Organoid Cultures (Adv Funct Mater, 2020, Valentina Magno et al.) and Engineering the Extracellular Matrix for Organoid Culture (Int J Stem Cells. 2022 Feb 28; 15(1): 60-69)] are incorporated herein by reference.

The extracellular matrix includes fibrin, laminin, collagen, and/or alginate, but is not limited thereto. Examples of extracellular matrix-producing cells include chondrocytes, which mainly produce collagen and proteoglycans; fibroblasts, which mainly produce type IV collagen, laminin, interstitial procollagen, and fibronectin; and colon myofibroblasts, which mainly produce collagen (types I, III, and V), chondroitin sulfate proteoglycans, hyaluronic acid, fibronectin, and tenascin-C. These are "naturally produced extracellular matrices". Naturally produced extracellular matrix may be commercially provided. Examples of commercially available extracellular matrices include extracellular matrix proteins (Invitrogen) and basement membrane preparations from Engelbreth-Holm-Swarm (EHS) murine sarcoma cells (e.g., Cultrex^{®} basement membrane extracts (Trevigen, Inc.), type I collagen (Invitrogen), VitroGel^{®} (TheWell Bioscience Inc.), Regenix^{®} (Cellartgen Inc.), Geltrex (ThermoFisher), or Matrigel^{®} (BD Biosciences)).

### 3-2) Differentiation from Hindgut (HG) into mature intestinal organoid (mature hIO)

As used herein, the term "maturation" or "mature" intestinal organoid (mature hIO), as opposed to an immature intestinal organoid, refers to an intestinal organoid in which genes necessary for digestive functions, transport systems, immune functions, and host defense of the small intestine of an adult are expressed. Specifically, the mature small intestine has unique characteristics including enhancement of expression of genes necessary for small intestine stem cell marker genes, digestive functions, transport systems, a wide range of immune functions, and host defense. In particular, proper expression and activity of transporters involved in physiological and pharmacokinetic roles is a prerequisite for normal small intestinal functions, such as absorption, distribution and excretion of drugs. Particularly, the mature intestinal organoid according to the present disclosure has improved efficacy in terms of engraftment. More specifically, this may be due to high expression characteristics of vascular endothelium-related factors in the stromal cell layer present around the intestinal epithelial cell layer constituting the mature intestinal organoid, and various secretory factors that are characteristics of stromal cells.

In addition, the mature intestinal organoid may exhibit characteristics of budding structure development present on the intestinal organoid. Further, the organoid exhibits higher development of goblet cells, thereby exhibiting high efficiency in terms of differentiation ability of intestinal stem cells.

The differentiation from hindgut (HG) into mature intestinal organoid is used in co-culture to mimic the *in vivo* intestinal environment, or essentially involves treatment with cytokines secreted by T-lymphocyte co-culture (that is, treatment with cytokines secreted by T-lymphocyte), and/or treatment with STAT3 and mTOR signaling pathway activators, in addition to the above-described factors for differentiation into the intestinal organoid.

More specifically, the cytokine secreted by T-lymphocyte may be one or more selected from the group consisting of IL-2 (interleukin-2), IL-22 (interleukin-22), IL-6 (interleukin-6), IL-1β (interleukin-1β), IL-11 (interleukin-11), EGF (epidermal growth factor), OSM (oncostatin M), and IL-10 (interleukin-10), and more specifically, may be IL-2.

The differentiation from hindgut to mature intestinal organoid may be performed to enable maturation of intestinal organoid by treatment with STAT3 and mTOR signaling pathway activators, in addition to the above-described factors for differentiation into the intestinal organoid. For example, the maturation of intestinal organoid may be performed by treatment with colivelin.

In addition, the maturation of intestinal organoid may be performed by treatment with NRG-1 (Neuregulin-1).

More preferably, in the differentiation from hindgut (HG) into mature intestinal organoid, the differentiation from hindgut (HG) into intestinal organoid, starting from the step of treatment with extracellular matrix (ECM), is used in co-culture to mimic the *in vivo* intestinal environment, or essentially includes treatment with cytokines secreted by T-lymphocyte co-culture (that is, treatment with cytokines secreted by T-lymphocyte), treatment with NRG-1, and/or treatment with STAT3 and mTOR signaling pathway activators.

In other words, the degree of maturation of the organoid may be greatly enhanced by immediate treatment of the hindgut with at least any one cytokine selected from the group consisting of IL-2, IL-22, IL-6, IL-1β, IL-11, EGF, OSM, and IL-10, NRG-1, and/or colivelin, as a medium component, for intestinal maturation.

Namely, any one or more cytokines selected from the group consisting of IL-2, IL-22, IL-6, IL-1β, IL-11, EGF, OSM, and IL-10, NRG-1, and/or colivelin may be included as a medium component. Preferred concentrations thereof are at least 0.1, 0.2, 0.3, 0.4, 0.5, 0.6, 0.7, 0.8, 0.9, 1.0, 2.0, and 3.0 ng/ml. More preferably, the concentration may be 0.3 to 2.0 ng/ml.

As used herein, the term immature intestinal organoid refers to an intestinal organoid without the use in co-culture to mimic the in vivo intestinal environment or without the treatment with cytokines secreted by T-lymphocyte co-culture, or without the treatment with STAT3 and mTOR signaling pathway activators.

In other words, the present disclosure may include a step of maturing the intestinal organoid by treating the hindgut with a BMP inhibitor; a Wnt agonist; a receptor tyrosine kinase ligand; and cytokine secreted by T-lymphocyte, NRG-1 or colivelin. More specifically, the present disclosure may include a step of maturing the intestinal organoid by treating the hindgut with Noggin as a BMP inhibitor;

at least any one Wnt agonist selected from the group consisting of R-spondin 1, R-spondin 2, R-spondin 3, and R-spondin 4;

an epidermal growth factor (EGF) as a receptor tyrosine kinase ligand; and

at least any one cytokine secreted by T-lymphocytes, selected from the group consisting of IL-2, IL-22, IL-6, IL-1β, IL-11, EGF, OSM, and IL-10, NRG-1 and/or colivelin.

According to an embodiment of the present disclosure, the differentiation from hindgut into mature intestinal organoid may essentially include Noggin, EGF and R-spondin 1, and the culturing may be performed by adding cytokines, NRG-1 and/or colivelin thereto, for differentiation into the mature intestinal organoid. The addition of these cytokines, NRG-1 and/or colivelin may be achieved within 24 hours, within 20 hours, within 16 hours, within 12 hours, within 8 hours, within 4 hours, within 2 hours, within 1 hour, or simultaneously, at the time of medium exchange in the culture stage of intestinal organoid after culturing the hindgut. The rapid addition of cytokines, as described above, may have greater benefits for maturation of intestinal organoids, particularly the enhancement of vascular endothelial factor expression to promote engraftment.

Further, one or more additional components selected from the group consisting of ROCK inhibitors, B27, N-acetylcysteine, N2, nicotinamide, Gastrin, IGF-1, heregulin-1β, bFGF, A-83-01, and SB202190 as described above, may be further included.

The differentiation into intestinal organoid may require different culture days depending on their morphological and molecular biological characteristics of the organoid. However, for production of the desired mature intestinal organoid, the organoid may be cultured for approximately 3 to 30 days, preferably 5 to 28 days. In addition, intestinal organoids that have reached a certain level of maturity at this culture stage may be maintained through subculture (passage culture).

### 4) Method for producing pluripotent stem cell-derived mature intestinal organoids comprising stromal cell layer around intestinal organoid

The present disclosure provides a method for producing a pluripotent stem cell-derived mature intestinal organoid comprising a stromal cell layer around an intestinal organoid, the method comprising:
(a) culturing pluripotent stem cells in a medium containing any one or more selected from the group consisting of Nodal, Activin A, Activin B, BMP4, Wnt3a, and bFGF to perform differentiation into definitive endoderm;
(b) culturing the definitive endoderm in a medium containing: any one or more GSK3 inhibitors selected from the group consisting of BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(α,4-dibromoacetophenone), L803-mts(Myr-N-GKEAPPAPPQSpP-NH₂), Wnt3a, and CHIR99021; and a fibroblast growth factor (FGF), to perform differentiation into hindgut; and
(c) culturing the hindgut in a medium containing Noggin; epidermal growth factor (EGF); any one or more Wnt agonists selected from the group consisting of R-spondin 1, R-spondin 2, R-spondin 3, and R-spondin 4; and any one or more factors selected from the group consisting of IL-2, IL-22, IL-6, IL-1β, IL-11, EGF, OSM, NRG-1, IL-10, and colivelin to produce a mature intestinal organoid.

The method for producing a pluripotent stem cell-derived mature intestinal organoid comprising a stromal cell layer around an intestinal organoid according to the present disclosure is characterized in that, after producing the hindgut in Step (b), in Step (c), any one or more factors selected from the group consisting of IL-2, IL-22, IL-6, IL-1β, IL-11, EGF, OSM, IL-10, NRG-1, and colivelin are immediately added. The rapid addition of the cytokines secreted by T-lymphocytes and/or colivelin may rapidly achieve maturation of intestinal organoids and impart favorable characteristics for the engraftment of the peristromal cell layer.

Specifically, by rapidly treating the cytokines secreted by T-lymphocytes and/or colivelin while maintaining the peristromal cells formed in the differentiation process from the early stage of culture (e.g., P0), intestinal organoids with improved transplantation characteristics may be produced. In particular, in order to maintain the peristromal cells, a blade or chopper may be used for isolation into a certain size or larger.

Step (a) above is described in detail as follows.

In Step (a) of culturing the pluripotent stem cells in a medium containing any one or more selected from the group consisting of Nodal, Activin A, Activin B, BMP4, Wnt3a, and bFGF to perform differentiation into definitive endoderm, the culturing may be performed for 6 to 120 hours, preferably 12 to 100 hours.

More specifically, any one selected from the group consisting of the above Activin A, BMP4, bFGF and Wnt3a, may be used. More specifically, Activin A may be used.

More specifically, the above pluripotent stem cells may be embryonic stem cells or induced pluripotent stem cells.

The factors may be used, for example, in an amount of 1 ng/ml to 1,000 ng/ml, preferably 10 ng/ml to 500 ng/ml, but the present disclosure is not limited thereto.

Any of the basic culture media described above may be used as the culture medium. Preferably, the culture medium may be RPMI1640. If necessary, the medium can further contain various concentrations of fetal bovine serum (FBS). For example, the medium may contain any fetal bovine serum (FBS or FCS) suitable for growth, such as 0.1, 0.2, 0.3, 0.5, 1.0, 2.0, 3.0, 4.0, or 5.0%.

If needed, the medium may be cultured in contact with the extracellular matrix (ECM). The extracellular matrix may be applied to the present differentiation method, including all of the matters described herein.

According to a specific embodiment of the present disclosure, the present disclosure may include (a) culturing pluripotent stem cells in a medium containing 10 ng/ml to 500 ng/ml Activin A and 0.1 to 4.0% FBS for 6 to 120 hours to perform differentiation into definitive endoderm.

Step (b) above is described in detail as follows.

In Step (b) of culturing the definitive endoderm in a medium containing: any one or more GSK3 inhibitors selected from the group consisting of BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(α,4-dibromoacetophenone), L803-mts(Myr-N-GKEAPPAPPQSpP-NH₂), and CHIR99021; and a fibroblast growth factor (FGF), to perform differentiation into hindgut, the culturing may be performed for 24 to 360 hours, preferably 36 to 240 hours.

More specifically, the GSK3 inhibitor may be CHIR99021. The GSK3 inhibitor may be treated in an amount of about 0.1 uM to about 100 uM, or about 0.2 uM to about 50 uM, or about 0.3 uM to about 10 uM.

More specifically, the FGF may be at least one selected from the group consisting of FGF1, FGF2, FGF3, FGF4, FGF10, FGF11, FGF12, FGF13, FGF14, FGF15, FGF16, FGF17, FGF18, FGF19, FGF20, FGF21, FGF22, and FGF23, and specifically, may be FGF4. Specifically, it may be used at a concentration of 100 ng/ml to 1,200 ng/ml, more specifically, 120 to 1,000 ng/ml.

Any of the basic culture media described above can be used as the culture medium. Preferably, the culture medium may be DMEM. If necessary, the medium can further contain various concentrations of fetal bovine serum (FBS). For example, the medium may contain any fetal bovine serum (FBS) suitable for growth, such as 0.1, 0.2, 0.3, 0.5, 1.0, 2.0, 3.0, 4.0, or 5.0%.

If needed, the medium may be cultured in contact with the extracellular matrix (ECM). The extracellular matrix may be applied to the present differentiation method, including all of the matters described herein.

According to a specific embodiment of the present disclosure, the present disclosure may include (b) culturing the definitive endoderm in a medium containing 0.1 uM to 100 uM CHIR99021; and 100 ng/ml to 1,200 ng/ml FGF4 for 24 to 360 hours to perform differentiation into hindgut.

Step (c) above is described in detail as follows.

In Step (c) of culturing the hindgut in a medium containing Noggin; epidermal growth factor (EGF); any one or more Wnt agonists selected from the group consisting of R-spondin 1, R-spondin 2, R-spondin 3, and R-spondin 4; and any one or more factors selected from the group consisting of IL-2, IL-22, IL-6, IL-1β, IL-11, EGF, OSM, NRG-1, IL-10, and colivelin to produce a mature intestinal organoid, the culturing may be performed for about 3 to 30 days, preferably about 5 to 28 days.

The production of the mature intestinal organoid according to the present disclosure essentially includes four components: Noggin; epidermal growth factor (EGF); Wnt agonist; and cytokine secreted by T-lymphocyte, NRG-1, or colivelin. In particular, the cytokine secreted by T-lymphocyte, NRG-1 or colivelin is added from the beginning stage of intestinal maturation by changing the medium from the hindgut differentiation stage, leading to rapid maturation of the intestine and significant growth of the stromal cell layer around the intestinal organoid.

In other words, after differentiation into Hindgut in Step (b), in Step (c), the cytokine secreted by T-lymphocytes, NRG-1 or colivelin, is immediately added.

More specifically, the noggin may be treated at a concentration of about 10 ng/ml to about 100 ng/ml.

The epidermal growth factor (EGF) may be treated at a concentration of 50 ng/ml to 300 ng/ml.

The Wnt agonist is preferably R-spondin 1. The R-spondin 1 may be treated at a concentration of 50 to 800 ng/ml.

The cytokine secreted by T-lymphocyte or colivelin is preferably IL-2. IL-2 may be treated at a concentration of 0.3 to 2.0 ng/ml.

Any of the basic culture media described above may be used as the culture medium. Preferably, the culture medium may be Advanced DMEM/F12.

If needed, the medium may be cultured in contact with the extracellular matrix (ECM). The extracellular matrix may be applied to the present differentiation method, including all of the matters described herein. The step of producing a mature intestinal organoid according to the present disclosure may be performed under Xeno-free conditions. More specifically, a Xeno-free polymer hydrogel (e.g., VitroGel), laminin (e.g., laminin-111), fibrin, or fibrin/laminin matrix may be used. More specifically, a mature intestinal organoid may be produced by applying Xeno-free VitroGel.

In particular, Step (c) above may comprise production of a mature intestinal organoid by applying Xeno-free VitroGel.

According to a specific embodiment of the present disclosure, Step (c) may include a step of culturing the hindgut in a medium containing 10 ng/mL to 100 ng/mL Noggin; 50 ng/mL to 300 ng/mL epidermal growth factor (EGF); 50 to 800 ng/mL R-spondin 1; and 0.3 to 2.0 ng/mL IL-2 to produce a mature intestinal organoid.

The extracellular matrix in this culture step may be VitroGel under xeno-free conditions.

Furthermore, the subculture may be performed as needed, and for example, the subculture may be performed from 12 hours to 7 days, preferably from 1 day to 5 days. The pluripotent stem cell-derived intestinal organoid comprising the stromal cell layer around the intestinal organoid of the present disclosure may be subcultured after being chopped to a certain size or larger. For example, the intestinal organoid of the present disclosure may be subcultured after being chopped to a length of 100 µm, 200 µm, 300 µm, 400 µm, 500 µm, 600 µm or more. For example, if it is subcultured after being chopped to the above-described certain size or larger, it has the advantage of maximally maintaining the characteristics of mature intestinal organoid, including the peristromal cell layer.

### Pluripotent stem cell-derived intestinal organoid comprising stromal cell layer around intestinal organoid

The present disclosure provides a pluripotent stem cell-derived intestinal organoid comprising a stromal cell layer around an intestinal organoid, wherein the stromal cell layer around the intestinal organoid comprises mesenchymal stromal cells.

As used herein, the term "maturation" or "mature" intestinal organoid (mature hIO) is an intestinal organoid in which genes necessary for digestive functions, transport systems, immune functions, and host defense of the small intestine of an adult are expressed. Specifically, the mature small intestine has unique characteristics including small intestine stem cell marker genes, digestive functions, transport systems, a wide range of immune functions, and enhancement of expression of genes necessary for host defense. In particular, proper expression and activity of transporters involved in physiological and pharmacokinetic roles is a prerequisite for normal small intestinal functions, such as absorption, distribution and excretion of drugs.

The mature intestinal organoid according to the present disclosure is produced from an intestinal organoid derived from a pluripotent stem cell (also referred to as pluripotent stem cell-derived intestinal organoid).

The pluripotent stem cell-derived intestinal organoid according to the present disclosure is characterized by having a stromal cell layer around the intestinal organoid epithelial layer. Therefore, the intestinal organoid has high expression levels of markers of mesenchymal stromal cells, including but not limited to αSMA, VIM, DESMIN, etc. The αSMA is able to function not only as a marker of mesenchymal stromal cells but also as a marker of pericytes.

Conventional adult stem cell-derived intestinal organoids or patient (tissue)-derived intestinal organoids do not have a peristromal cell layer when inducing differentiation into intestinal organoids. On the other hand, the pluripotent stem cell-derived intestinal organoid according to the present disclosure exhibits properties that differ from previously known adult-derived intestinal organoids by having the stromal cell layer around the intestinal organoid.

The mature intestinal organoid differentiated by treatment with cytokines or colivelin for maturation according to the present disclosure has improved efficacy in terms of engraftment compared to immature intestinal organoids derived from non-mature pluripotent stem cells. More specifically, the stromal cell layer around the mature intestinal organoid may exhibit high expression of vascular endothelial-related factors.

More specifically, the mature intestinal organoid is a mature intestinal organoid comprising a peristromal cell layer (i.e., a stromal cell layer around an intestinal organoid), wherein the peristromal cell layer comprises intestine-derived mesenchymal stromal cells.

The mature intestinal organoid is derived from pluripotent stem cells (PSCs).

The mature intestinal organoid is characterized by higher expression of any one or more factors selected from the group consisting of VIL1, KRT20, CHGA, MUC13, LYZ, OLFM4, SI, LCT, and DPP4, compared to pluripotent stem cell-derived immature intestinal organoids. More specifically, the mature intestinal organoid may exhibit a 2-fold or greater difference in the level expression of genes selected from the group consisting of KRT20, CHGA, MUC13, LYZ, and OLFM4 compared to pluripotent stem cell-derived immature intestinal organoids.

The mature intestinal organoid according to the present disclosure may have good maintenance and differentiation of intestinal organoid through cell spheroid formation and low cell-matrix interaction, especially under xeno-free conditions, thereby providing an intestinal organoid suitable for clinical applications.

The mature intestinal organoid according to the present disclosure may exhibit high expression of angiogenic factors, vascular endothelial-related factors, and/or vascular endothelial cell-related factors. For example, the mature intestinal organoid according to the present disclosure may have high expression levels of any one or more angiogenic factors, vascular endothelial-related factors and/or vascular endothelial cell-related factors selected from the group consisting of hCD31 (PECAM-1), CDH5 (VE-CAD), ECSCR, LYVE1, IGF2, and VEGF (e.g., VEGF-A).

The mature intestinal organoid according to the present disclosure is characterized in that the expression of any one or more selected from the group consisting of vascular markers hCD31, ANGPT1 and KDR is higher than that of adult stem cell-derived organoids or pluripotent stem cell-derived immature intestinal organoids.

The mature intestinal organoid according to the present disclosure may have high expression levels of small intestine-specific markers. The small intestine-specific markers may include, but are not limited to, VIL1, KRT20, CHGA, LYZ, DEFA5, OLFM4, MUC2, MUC13, DPP4, LCT, SLC5A1, CREB3L3, etc

The mature intestinal organoid according to the present disclosure comprises mesenchymal stromal cells in the stromal cell layer around the intestinal organoid, and particularly comprises a large number of cells exhibiting characteristics of vascular cells (hCD31⁺). In addition, compared to adult stem cell-derived organoids or pluripotent stem cell-derived immature intestinal organoids, the mature intestinal organoid according to the present disclosure has very high expression levels of groups related to vascular development, mesenchyme development, epithelial cell proliferation, and/or muscle tissue development.

In particular, it is characterized by a high expression level of hCD31⁺ together with high expression levels of markers related to mesenchymal stromal cells.

In addition, the mature intestinal organoid is characterized in that the stromal cell layer around the intestinal organoid and/or the mesenchymal stromal cells present therein may have endothelial cell type characteristics.

In addition, in the mature intestinal organoid, the stromal cell layer around the intestinal organoid and/or the mesenchymal stromal cells present therein may exhibit high expression levels of angiogenic factors, vascular endothelial-related factors or vascular endothelial cell-related factors, compared to adult stem cell-derived intestinal organoids or pluripotent stem cell-derived immature intestinal organoids. These angiogenic factors, vascular endothelial-related factors, or vascular endothelial cell-related factors are as described above.

As described above, the mature intestinal organoid having the stromal cell layer around the intestinal organoid may exhibit excellent therapeutic efficacy through high engraftment ability and excellent regeneration ability of intestinal stem cells within the organoid by transplanting the peripheral cell layer around the organoid together. Therefore, the mature intestinal organoid of the present disclosure may exhibit excellent performance in intestinal transplantation, and thus can be utilized as a high-performance intestinal organoid regenerative therapeutic agent. The mature intestinal organoid according to the present disclosure is defined and used herein interchangeably with high-performance mature intestinal organoid or matured intestinal organoid.

The present disclosure provides a pluripotent stem cell-derived intestinal organoid comprising a stromal cell layer around an intestinal organoid,

wherein the stromal cell layer around the intestinal organoid comprises mesenchymal stromal cells. The stromal cell layer around the intestinal organoid comprises, but is not limited to, a large amount of mesenchymal stromal cells, and thus has high expression levels of related markers capable of exhibiting mesenchymal stromal cell characteristics, such as αSMA, VIM, DESMIN, etc

More specifically, the stromal cell layer around the intestinal organoid may further comprise at least any one cell selected from the group consisting of smooth muscle cells, fibroblasts, myofibroblasts, telocytes, and pericytes.

The pluripotent stem cell-derived intestinal organoid comprising the stromal cell layer around the intestinal organoid according to the present disclosure is preferably a mature intestinal organoid treated with Cytokines secreted by T-lymphocytes, NRG-1 and/or colivelin, at the intestinal organoid differentiation stage from the hindgut. More specifically, Cytokines secreted by T-lymphocytes, NRG-1 and/or colivelin are added when converting from Hindgut to intestinal organoid differentiation medium. Specifically, the pluripotent stem cell-derived intestinal organoid is cultured by treatment with at least any one factor selected from the group consisting of IL-2, IL-22, IL-6, IL-1β, IL-11, EGF, OSM, IL-10, NRG-1, and colivelin.

More specifically, the cytokine secreted by T-lymphocyte and/or colivelin may be IL-2.

The treatment with at least any one factor selected from the group consisting of IL-2, IL-22, IL-6, IL-1β, IL-11, EGF, OSM, IL-10, NRG-1, and colivelin described above may be performed during differentiation into the intestinal organoid from the hindgut.

The pluripotent stem cell-derived mature intestinal organoid comprising the stromal cell layer around the intestinal organoid according to the present disclosure may have higher expression of any one or more factors selected from the group consisting of VIL1, KRT20, CHGA, MUC13, LYZ, OLFM4, SI, LCT, and DPP4, compared to pluripotent stem cell-derived immature intestinal organoids.

Further, the pluripotent stem cell-derived mature intestinal organoid comprising the stromal cell layer around the intestinal organoid (referred to as the matured intestinal organoid) may have higher expression of any one or more factors selected from the group consisting of IGF2, FOXF1, CXCL14, BMP4, IGFBP3, APOA1, PDGFRA, FOXF2, PTCH1, VEGFA NKX2-3, BMP5, ITGA8, NRG1, ADAM28, COL4A5, and DLL1 in the stromal cells, compared to pluripotent stem cell-derived immature intestinal organoids.

In addition, the pluripotent stem cell-derived mature intestinal organoid comprising the stromal cell layer around the intestinal organoid (referred to as the matured intestinal organoid) may have higher expression of any one or more factors selected from the group consisting of intestinal stem cell markers ASCL2, AXIN2, LGR5, and OLFM4, compared to pluripotent stem cell-derived immature intestinal organoids. More specifically, the expression levels of any one or more markers selected from the group consisting of ASCL2, AXIN2, LGR5, and OLFM4 is enhanced. More specifically, the expression characteristics of the markers may be higher compared to pluripotent stem cell-derived immature intestinal organoids.

In particular, the pluripotent stem cell-derived mature intestinal organoid comprising the stromal cell layer around the intestinal organoid according to the present disclosure may have high levels of vascularization-related markers. The vascularization-related markers may be, for example, any one or more angiogenic factors, vascular endothelial-related factors and/or vascular endothelial cell-related factors selected from the group consisting of hCD31 (PECAM-1), CDH5 (hVE-Cad), ECSCR, LYVE1, IGF2, VEGF (e.g., VEGF-A), ANGPT1 and KDR. More specifically, the expression level of any one or more markers selected from the group consisting of hCD31, CDH5, ECSCR, LYVE1, IGF2, VEGF (e.g., VEGF-A), ANGPT1, and KDR is improved. More specifically, the expression characteristics of the markers may be higher compared to pluripotent stem cell-derived immature intestinal organoids.

Further, the pluripotent stem cell-derived mature intestinal organoid comprising the stromal cell layer around the intestinal organoid (referred to as the matured intestinal organoid) may have higher expression of any one or more factors selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, compared to pluripotent stem cell-derived immature intestinal organoids. More specifically, the expression level of at least any one marker selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1 is improved. More specifically, the expression characteristics of the markers may be higher compared to pluripotent stem cell-derived immature intestinal organoids.

These results show that, compared to intestinal organoids produced by conventionally known methods, the mature intestinal organoid produced by the method according to the present disclosure has many MSCs with enhanced engraftment ability around the organoid, excellent intestinal stem cell characteristics, and significantly increased expression of markers related to vascularization.

Here, the term immature intestinal organoid refers to an intestinal organoid without the use in co-culture to mimic the in vivo intestinal environment or without the treatment with cytokines secreted by T-lymphocyte co-culture, or without the treatment with STAT3 and mTOR signaling pathway activators.

The pluripotent stem cell-derived mature intestinal organoid comprising the stromal cell layer around the intestinal organoid according to the present disclosure may have high expression levels of small intestine-specific markers. The small intestine-specific markers may include, but are not limited to, VIL1, KRT20, CHGA, LYZ, DEFA5, OLFM4, MUC2, MUC13, DPP4, LCT, SLC5A1, CREB3L3, etc. More specifically, the pluripotent stem cell-derived mature intestinal organoid comprising the stromal cell layer around the intestinal organoid may have high expression levels of any one or more intestinal stem cell markers selected from the group consisting of ASCL2, AXIN2, OLFM4, and LGR5 and/or any one or more vascular-specific markers selected from the group consisting of hCD31, CDH5, ECSCR, LYVE1, IGF2, VEGF (e.g., VEGF-A), ANGPT1, and KDR.

The stromal cell layer around the intestinal organoid of the pluripotent stem cell-derived mature intestinal organoid comprising the stromal cell layer around the intestinal organoid according to the present disclosure comprises vascular cells (hCD31⁺). In other words, it is characterized by a high expression level of hCD31⁺ together with high expression levels of markers related to mesenchymal stromal cells. More specifically, the peristromal cell layer of the mature intestinal organoid may comprise hCD31⁺ vascular cells.

The mesenchymal stromal cells included in the stromal cell layer around the intestinal organoid of the pluripotent stem cell-derived mature intestinal organoid comprising the stromal cell layer around the intestinal organoid described above, exhibit at least one characteristic selected from the following:
(a) an enhanced expression level of hCD31 (PECAM1) compared to adult-derived mesenchymal stem cells, pluripotent stem cell-derived mesenchymal stem cells and/or immature intestinal organoid-derived mesenchymal stromal cells;
(b) enhanced expression levels of factors related to cellular vascular endothelial stimulation, angiogenesis, cell adhesion, and differentiation, compared to adult-derived mesenchymal stem cells, pluripotent stem cell-derived mesenchymal stem cells, and/or immature intestinal organoid-derived mesenchymal stromal cells;
(c) enhanced expression levels of endothelial cell types, compared to adult-derived mesenchymal stem cells, pluripotent stem cell-derived mesenchymal stem cells, and/or immature intestinal organoid-derived mesenchymal stromal cells;
(d) enhanced expression levels of endothelial cell characteristic factors on gene ontology (GO) term enrichment, compared to adult-derived mesenchymal stem cells, pluripotent stem cell-derived mesenchymal stem cells, and/or immature intestinal organoid-derived mesenchymal stromal cells;
(e) enhanced expression levels of angiogenic factors, vascular endothelial-related factors or vascular endothelial cell-related factors, compared to adult-derived mesenchymal stem cells, pluripotent stem cell-derived mesenchymal stem cells, and/or immature intestinal organoid-derived mesenchymal stromal cells;
   wherein the angiogenic factor, vascular endothelial-related factor and/or vascular endothelial cell-related factor is any one or more selected from the group consisting of hCD31, CDH5, ECSCR, LYVE1, IGF2, VEGF (such as VEGF-A), ANGPT1 and KDR,
(f) enhanced expression levels of any one or more selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, compared to adult-derived mesenchymal stem cells, pluripotent stem cell-derived mesenchymal stem cells, and/or immature intestinal organoid-derived mesenchymal stromal cells; and
(g) maintained expression levels of any one or more selected from the group consisting of αSMA, VIM and DESMIN at the time of subculture.

### Mature intestinal organoid-derived peristromal cell layer

Existing intestinal organoid technologies have not adequately addressed the necessity of the surrounding environment of intestinal organoids, interactions with stromal cells/immune cells, etc.

In particular, in adult stem cell-derived organoid technologies, where significant research and development are ongoing, the peristromal stromal cell layer is not generated or is insufficient in the environment around the organoid. In addition, even when utilizing pluripotent stem cells, techniques that aims to control this surrounding microenvironment, especially the peristromal cell layer have not been identified or reported.

The present disclosure may exhibit high engraftment ability by controlling the function and characteristics of the peristromal cell layer derived from the mature intestinal organoid through the production process of the above-described mature intestinal organoid

The peristromal cell layer comprises the above-described mesenchymal stromal cells. Further, the peristromal cell layer, including the mesenchymal stromal cells, may have high expression levels of vascular endothelial- or vascular endothelial cell-related factors.

Further, the peristromal cell layer may further comprise at least any one cell selected from the group consisting of smooth muscle cells, fibroblasts, myofibroblasts, telocytes, and pericytes.

This peristromal cell layer may support the engraftment of intestinal organoid and angiogenesis, and exhibit rapid recovery and therapeutic efficacy through interactions with immune cells, stromal cells, etc.

In particular, the mesenchymal stromal cells included in the mature intestinal organoid-derived peristromal cell layer according to the present disclosure may exhibit the characteristics described below, and the description of these characteristics encompasses the characteristics of the mature intestinal organoid-derived peristromal cell layer and the mesenchymal stromal cells included therein. In other words, the characteristics of the mature intestinal organoid-derived mesenchymal stromal cells described below may also be interpreted as referring to the characteristics of the mature intestinal organoid-derived peristromal cell layer.

### Mature intestinal organoid-derived mesenchymal stromal cells and isolation and culture thereof

The present disclosure provides mesenchymal stromal cells isolated (derived) from the mature intestinal organoid-derived peristromal cell layer and a method for isolating and/or culturing the same.

The mesenchymal stromal cells according to the present disclosure are mesenchymal stromal cells derived from the peristromal cell layer of mature intestinal organoids differentiated from pluripotent stem cells (PSCs).

A method for culturing mesenchymal stromal cells derived from the peristromal cell layer of mature intestinal organoids differentiated from pluripotent stem cells (PSCs) may be performed through the following steps:
(a) isolating a mature intestinal organoid into single cells or small cell clumps;
(b) seeding the isolated single cells or the small cell clumps in a culture medium coated with any one selected from the group consisting of gelatin, fibrin, and collagen I; and
(c) obtaining only cells that are adhered and cultured from the seeded cells.

In Step (a) above, the mature intestinal organoid refers to an intestinal organoid exhibiting the above-described mature characteristics. The intestinal organoid is isolated into single or small cell clumps by commonly known single cell isolation methods. According to an embodiment of the present disclosure, the mature intestinal organoid is isolated from an extracellular matrix, and cells are obtained from a cell layer existing around the intestinal organoid, followed by treatment with Trypsin-EDTA, TrypLE TM, etc., to isolate single cells.

In Step (b) above, the isolated single cells or small cell clumps may be seeded on a coating medium selected from the group consisting of gelatin, fibrin, and collagen I, and then cultured.

In Step (c) above, when single cells or small cell clumps are cultured in a coating medium selected from the group consisting of gelatin, fibrin, and collagen I, only cells exhibiting the characteristics of mesenchymal stromal cells are adhered to and cultured in the coating medium.

These adhered and cultured cells exhibit characteristics as mesenchymal stromal cells, and the above-described properties of mature intestinal organoid-derived mesenchymal stromal cells are maintained and expressed. A commonly known culture medium may be used for culturing the mature intestinal organoid-derived mesenchymal stromal cells. Preferably, the culturing may be performed in a medium containing B27, EGF, or both.

In particular, the medium composition according to the present disclosure exhibits excellent effects on the growth and maintenance of mesenchymal stem cells even under xeno-free conditions.

Further, the method for culturing mesenchymal stromal cells derived from the peristromal cell layer of the mature intestinal organoid differentiated from the pluripotent stem cell (PSC) may further comprise, after Step (c), (d) subculturing the adhered and cultured cells.

The above mature intestinal organoid may be produced, for example, through the following steps:
1) differentiating pluripotent stem cells into definitive endoderm cells by treatment with at least any one agent selected from the group consisting of Nodal, Activin A, Activin B, BMP4, and Wnt3a;
2) differentiatiating the definitive endoderm cells into a hindgut by treatment with FGF and Wnt ligands; and
3) maturing the intestinal organoid by treating the hindgut with a BMP inhibitor, a Wnt agonist, a receptor tyrosine kinase ligand, and cytokine secreted by T-lymphocyte.

More specifically, Step 1) above may include a step of differentiating embryonic stem cells or induced pluripotent stem cells into definitive endoderm cells by treatment with Activin A.

More specifically, Step 2) above may include a step of differentiating the definitive endoderm cells to the hindgut by treatment with FGF and CHIR99021.

More specifically, Step 3) above may include a step of maturing the intestinal organoid by treating the hindgut with Noggin as a BMP inhibitor, at least any one selected from the group consisting of R-spondin 1, R-spondin 2, R-spondin 3, and R-spondin 4 as Wnt agonists, an epidermal growth factor (EGF) as a receptor tyrosine kinase ligand, and at least any one cytokine selected from the group consisting of IL-2, IL-22, IL-6, IL-1β, IL-11, EGF, OSM, and IL-10 as cytokines secreted by T-lymphocytes.

More specifically, the cytokine secreted by T-lymphocyte may be IL-2.

The present disclosure also provides mesenchymal stromal cells derived from the stromal cell layer around the mature intestinal organoid, produced through the above culture process.

The present disclosure also provides mesenchymal stromal cells derived from the stromal cell layer around the mature intestinal organoid. More preferably, the present disclosure provides mesenchymal stromal cells derived from the stromal cell layer around the mature intestinal organoid, obtained through the above culture process.

The mesenchymal stromal cells derived from the stromal cell layer around the mature intestinal organoid exhibit at least one or more characteristics selected from the following:
(a) an enhanced expression level of hCD31 (PECAM1) compared to adult-derived mesenchymal stem cells, pluripotent stem cell-derived mesenchymal stem cells and/or immature intestinal organoid-derived mesenchymal stromal cells;
(b) enhanced expression levels of factors related to cellular vascular endothelial stimulation, angiogenesis, cell adhesion, and differentiation, compared to adult-derived mesenchymal stem cells, pluripotent stem cell-derived mesenchymal stem cells, and/or immature intestinal organoid-derived mesenchymal stromal cells;
(c) enhanced expression levels of endothelial cell types, compared to adult-derived mesenchymal stem cells, pluripotent stem cell-derived mesenchymal stem cells, and/or immature intestinal organoid-derived mesenchymal stromal cells;
(d) enhanced expression levels of endothelial cell characteristic factors on gene ontology (GO) term enrichment, compared to adult-derived mesenchymal stem cells, pluripotent stem cell-derived mesenchymal stem cells, and/or immature intestinal organoid-derived mesenchymal stromal cells;
(e) enhanced expression levels of angiogenic factors, vascular endothelial-related factors or vascular endothelial cell-related factors, compared to adult-derived mesenchymal stem cells, pluripotent stem cell-derived mesenchymal stem cells, and/or immature intestinal organoid-derived mesenchymal stromal cells;
   wherein the angiogenic factor, vascular endothelial-related factor and/or vascular endothelial cell-related factor is any one or more selected from the group consisting of hCD31, CDH5, ECSCR, LYVE1, IGF2, VEGF (such as VEGF-A), ANGPT1 and KDR,
(f) enhanced expression levels of any one or more selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, compared to adult-derived mesenchymal stem cells, pluripotent stem cell-derived mesenchymal stem cells, and/or immature intestinal organoid-derived mesenchymal stromal cells; and
(g) maintained expression levels of any one or more selected from the group consisting of αSMA, VIM and DESMIN at the time of subculture.

The mature intestinal organoid-derived mesenchymal stromal cells that constitute the stromal cell layer around the mature intestinal organoid may additionally exhibit at least one of the following characteristics: enhanced expression levels of any one or more vascular secretory factors selected from the group consisting of VEGF, Angiogenin, DKK1, EGF, Osteopontin, ICAM-1, VCAM-1, IGFBP3, MCP-1 and Activin A;
enhanced expression levels of any one or more intestinal-related promoting factors selected from the group consisting of CD34, RSPO3, DLL1, WNT2B, HGF, FOXL1, NOG, GLI1, WNT9A, WNT3, GLI2, and DLL3, compared to adult-derived mesenchymal stem cells;
enhanced expression levels of any one or more vascular-related promoting factors selected from the group consisting of VWF, PECAM1, ANGPTL1, CD34, ICAM1, and FLT1, compared to adult-derived mesenchymal stem cells;
enhanced expression levels of any one or more intestinal-related promoting factors selected from the group consisting of CD34, RSPO3, DLL1, WNT2B, HGF, FOXL1, NOG, WNT9A, WNT3, GLI2, and DLL3, compared to pluripotent stem cell-derived mesenchymal stem cells;
enhanced expression levels of any one or more vascular-related promoting factors selected from the group consisting of VWF, PECAM1, ANGPTL1, CD34, ICAM1 and FLT1,
compared to pluripotent stem cell-derived mesenchymal stem cells;
enhanced expression levels of the intestinal-related promoting factors, NOG and/or WNT9A, compared to immature intestinal organoid-derived mesenchymal stem cells; and
enhanced expression levels of any one or more vascular-related promoting factors selected from the group consisting of PECAM1, CDH5, LYVE1 and ECSCR, compared to immature intestinal organoid-derived mesenchymal stem cells.

On the other hand, the immature intestinal organoid-derived mesenchymal stromal cells are cells derived from intestinal organoids without the use in co-culture to mimic the in vivo intestinal environment or without the treatment with cytokines secreted by T-lymphocyte co-culture, or without the treatment with NRG-1 and/or with STAT3 and mTOR signaling pathway activator, colivelin. More specifically, unlike mature intestinal organoid-derived mesenchymal stromal cells, the immature intestinal organoid-derived mesenchymal stromal cells refer to mesenchymal stromal cells derived from intestinal organoids to which neither any one nor more selected from the group consisting of IL-2, IL-22, IL-6, IL-1β, IL-11, EGF, OSM, and IL-10 are added, nor are NRG-1 or colivelin added, as medium components.

Adult-derived mesenchymal stem cells refer to mesenchymal stem cells that exist in any tissue of an adult, such as bone marrow, umbilical cord blood, and adipose-derived mesenchymal stem cells.

Mesenchymal stem cells derived from pluripotent stem cells refer to cells that are differentiated into mesenchymal stem cells through the differentiation process of pluripotent stem cells.

As described above, the mesenchymal stromal cells derived from the stromal cell layer around the mature intestinal organoid have the following advantages and thus have high potential for use as a therapeutic agent for various diseases.

The mesenchymal stromal cells of the present disclosure correspond to mesenchymal stromal cells isolated from pluripotent stem cell-derived intestinal organoids, which can be used for allogeneic transplantation. Specifically, the mesenchymal stromal cells isolated from pluripotent stem cell-derived intestinal organoids with a reduced expression level of human leukocyte antigen (HLA), which are responsible for immune rejection during cell, tissue, and organ transplantation, may be utilized for therapeutic purposes as a cell source that does not cause immune rejection.

The mesenchymal stromal cells of the present disclosure may have high expression levels of angiogenic factors and vascular secretory factors and exhibit Gene Ontology of endothelial cells, especially vascular endothelial cells, thereby enhancing an early engraftment rate including vascularization during intestinal transplantation.

The mesenchymal stromal cells of the present disclosure may continuously show equivalent characteristics without losing the characteristics of mesenchymal stromal cells during passage culture, and thus they can be maintained for a long period of time without changing cell characteristics and can be subjected to passage culture.

For use as a therapeutic agent, it is preferable to use the mesenchymal stromal cells at a passage level of 1 to 10, and more preferably at a passage level of 2 to 6.

In particular, the present disclosure has a high potential for use as a therapeutic agent by continuously maintaining the characteristics of stem cells that are usually changed or lost under freezing and thawing conditions.

Under the above advantages, the mesenchymal stromal cells according to the present disclosure may have a high potential for use as an excellent regenerative therapeutic agent in combination with mesenchymal stromal cells themselves, other intestinal organoids or intestinal stem cells based on their high engraftment capacity.

### Medicinal Use

The present disclosure provides a transplant material comprising the pluripotent stem cell-derived intestinal organoid comprising the stromal cell layer around the intestinal organoid as described above.

The present disclosure also provides a pharmaceutical composition comprising: the pluripotent stem cell-derived intestinal organoid comprising the stromal cell layer around the intestinal organoid as described above.

The present disclosure also provides a pharmaceutical composition for preventing or treating an intestinal disease, comprising: the pluripotent stem cell-derived intestinal organoid comprising the stromal cell layer around the intestinal organoid as described above.

The pluripotent stem cells (PSCs) may be embryonic stem cells or induced pluripotent stem cells.

The pluripotent stem cell-derived intestinal organoid comprising the stromal cell layer around the intestinal organoid of the present disclosure may be superior for restoring cell or tissue function in tissue transplantation since it includes the mature peristromal cell layer around the intestinal organoid that is similar to the human intestinal environment and exhibits improved features such as vascularization and enhanced engraftment.

For example, the pluripotent stem cell-derived intestinal organoid comprising the stromal cell layer around the intestinal organoid, may be utilized as a transplant material itself and may have high value as a pharmaceutical composition. In particular, the pluripotent stem cell-derived intestinal organoid of the present disclosure may be applied to the treatment of various intestinal diseases and may be considered for use as a material for regeneration and reconstruction of injured (including dysfunctional) intestinal tissue. In particular, it is utilized as an intestinal transplant material.

Thus, the pharmaceutical composition according to the present disclosure may be a tissue therapeutic agent.

In the present disclosure, the intestinal disease may be any one or more intestinal diseases selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

A prerequisite for transplantation efficiency and regenerative treatment efficiency is that blood is supplied to transplanted cells or tissues through angiogenesis by rapidly engrafting on a transplanted site. Conventional small intestinal organoids, large intestinal organoids (colon organoids) or intestinal stem cells have a low engraftment rate and have insufficient vascularization at the time of intestinal transplantation, so that an initial transplantation efficiency is very low.

The pluripotent stem cell-derived intestinal organoid comprising the stromal cell layer around the intestinal organoid according to the present disclosure may show excellent effectiveness in improving the engraftment rate and vascularization efficiency at the time of intestinal transplantation, thereby solving the problem of engraftment, which is one of the biggest obstacles in intestinal transplantation.

In such transplantation, the pluripotent stem cell-derived intestinal organoid comprising the stromal cell layer around the intestinal organoid according to the present disclosure may be used in transplantation together with at least one biodegradable support selected from the group consisting of fibrin, laminin, collagen, gelatin, chitosan, alginate, hyaluronic acid, dextran, polylactic acid, poly(glycolic acid) (PGA), poly(lactic-co-glycolic acid) (PLGA), poly-ε-(caprolactone), polyanhydrides, polyorthoesters, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof. In other words, the transplant material or the pharmaceutical composition may further comprise a biodegradable support.

In other words, the organoid of the above transplant material may be utilized for transplantation as it is or by being embedded in the support described above.

In addition, dimethylsulfoxide (DMSO) and the like may be added for the purpose of protecting cells, an antibiotic substance and the like may be added for the purpose of blocking incorporation of bacteria, and various components (vitamins, cytokines, growth factors, steroids, and the like) may be added to the transplant material of the present disclosure for the purpose of activating cells, proliferation, or inducing differentiation.

The transplant material of the present disclosure is also available for the construction of in vivo test system. For example, the above-described transplant material may be transplanted into experimental animals such as mice, rats, guinea pigs, hamsters, pigs, cynomolgus monkeys, rhesus monkeys, chimpanzees, and the like, to produce humanized animals (human intestinal models). Such humanized animals are particularly useful for experiments such as pharmacokinetics and toxicity tests, and are expected to contribute to studies such as the effect of initial passing effects on oral drugs or pharmaceutical enteritis.

The term "prevention" herein means all actions that inhibit or delay the onset of intestinal diseases by administration of the composition, and the term "treatment" herein means all actions that improve or benefit the symptoms of intestinal diseases by administration of the composition.

The pharmaceutical composition of the present disclosure may be formulated into various formulations such as a liquid formulation, a suspension, and the like, according to the conventional method.

The pluripotent stem cell-derived intestinal organoid comprising the stromal cell layer around the intestinal organoid for such formulation, may be formulated as the pluripotent stem cell-derived intestinal organoid itself or as a mixture thereof after being chopped to a certain size, including the stromal cell layer around the intestinal organoid. For example, the certain size may be chopped to have a length of 100 µm, 200 µm, 300 µm, 400 µm, 500 µm, 600 µm, 700 µm, 800 µm, 900 µm, 1000 µm, or more. The length chopped to the certain size can vary depending on the subject to be administered. Preferably, the intestinal organoid may be chopped to a size of 200 to 500 µm.

The pharmaceutical composition of the present disclosure may be administered while being formulated into a unit dosage form of a pharmaceutical formulation suitable for administration into the body of a patient according to the conventional method in the pharmaceutical field, and the formulation includes an effective dosage by one or several administrations. The preferred formulations suitable for this purpose include parenteral formulations such as injections, infusions, and transplants. In addition, the pharmaceutical composition may include a pharmaceutically acceptable common inert carrier and diluent. The pharmaceutically acceptable carrier and diluent may be biologically and physiologically friendly to the mesenchymal stromal cells and recipients to be transplanted with the mesenchymal stromal cell. The diluent may include saline, a water-soluble buffer, a solvent and/or a dispersion medium, but the present disclosure is not limited thereto. In addition, for example, the injection may further include a preservative, a painless agent, a solubilizer, a stabilizer, or the like, and the preparation for topical administration may further include a base, an excipient, a lubricant, a preservative, or the like.

The composition of the present disclosure may be used unfrozen or may be frozen for later use. When the composition is to be frozen, a standard cryopreservative (e.g., DMSO, glycerol, and Epilife^{®} cell freezing medium (Cascade Biologics)) may be added to the cell population prior to freezing. The pluripotent stem cell-derived intestinal organoid comprising the stromal cell layer around the intestinal organoid according to the present disclosure is suitable for direct use as a therapeutic agent in that it retains its properties without degradation due to freezing and thawing.

In addition, the pluripotent stem cell-derived intestinal organoid of the present disclosure may be transplanted and administered using an administration method commonly used in the art, and preferably, may be directly engrafted or transplanted into a disease site of the patient in need of treatment, but the present disclosure is not limited thereto. In addition, the administration may be performed by both non-surgical administration using a catheter and surgical administration such as injection or transplantation after incision in the disease site. However, it should be understood that an actual dosage of an active component should be determined in light of various relevant factors such as a disease to be treated, severity of the disease, a route of administration, a weight, age and sex of the patient, and the like, and thus the dosage does not limit the scope of the present disclosure in any way.

The present disclosure also provides a pluripotent stem cell-derived intestinal organoid comprising a stromal cell layer around an intestinal organoid, as a transplant material.

The present disclosure also provides the use of the pluripotent stem cell-derived intestinal organoid comprising the stromal cell layer around the intestinal organoid, in the manufacture of a medicament for use as a transplant material.

There is also provided a method for treating an intestinal disease, comprising administering to a subject in need thereof the pluripotent stem cell-derived intestinal organoid comprising the stromal cell layer around the intestinal organoid.

The term "subject" refers to any animal that has developed or may develop an intestinal disease, including humans, monkeys, cows, horses, sheep, pigs, chickens, turkeys, quails, cats, dogs, mice, rats, rabbits, or guinea pigs.

The present disclosure also provides a pharmaceutical composition comprising: the pluripotent stem cell-derived intestinal organoid comprising the stromal cell layer around the intestinal organoid for use in preventing or treating an intestinal disease.

The present disclosure also provides the use of the pluripotent stem cell-derived intestinal organoid comprising the stromal cell layer around the intestinal organoid, in the manufacture of a medicament for use in preventing or treating an intestinal disease.

Unless otherwise defined, all technical terms used in the present disclosure are used in the same meaning as commonly understood by those skilled in the art in the related field of the present disclosure. In addition, a preferred method or sample is described in the present specification, but those similar or equivalent thereto are also included in the scope of the present disclosure. The contents of all referenced publications herein are incorporated by reference into the present disclosure.

### [Advantageous Effects]

The high-performance mature human intestinal organoid regenerative therapeutic agent derived from pluripotent stem cells according to the present disclosure includes peristromal cell layers containing mesenchymal stromal cells, derived from the intestine, as the microenvironment around the intestine. In other words, the present disclosure may exhibit high engraftment ability by transplantation together with peripheral cell layers around the intestinal organoid and may exhibit superior regeneration ability through intestinal stem cells present in mature intestinal organoid, and therefore is likely to be useful as a regenerative therapeutic agent.

### [Description of Drawings]

FIG. 1 shows a technique for differentiation and culture of high-performance mature intestinal organoids for transplantation derived from pluripotent stem cells.

FIG. 1a is a schematic diagram of a protocol for differentiation and subculture into mature intestinal organoids from pluripotent stem cells, which shows a protocol for maturation of differentiated human intestinal organoids (hIO) derived from pluripotent stem cells through daily treatment with 1 ng/ml rhIL-2 from P0 for maturation, wherein mature intestinal organoids are subcultured every 10 to 14 days, and especially from P1, a blade or chopper is used for subculture.

FIG. 1b shows cell morphology of immature or mature intestinal organoids (P2) differentiated from pluripotent stem cells (embryonic stem cells and induced pluripotent stem cells).

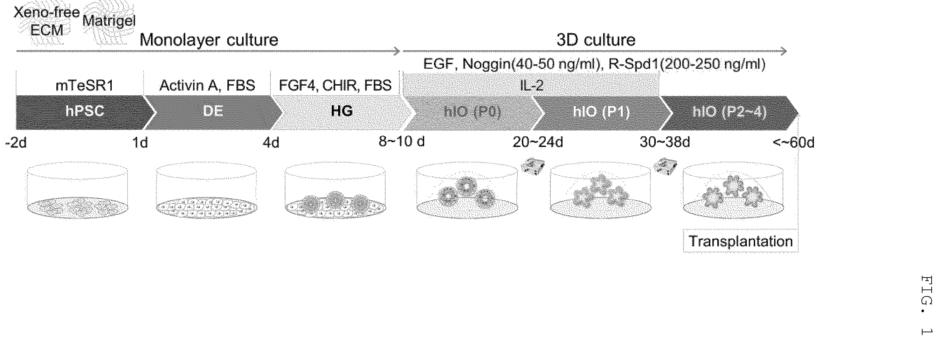

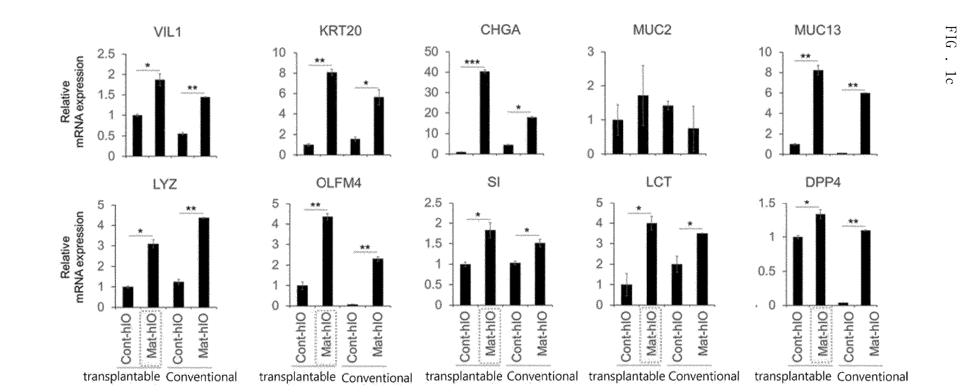

FIG. 1c shows qPCR analysis results of comparison between organoids (conventional) produced by the conventional method for producing mature organoids (rhIL-2 treatment from P1, subculture with a surgical blade, and containing 80-90 ng/ml Noggin and 400-450 ng/ml R-spondin1 in hIO medium) and organoids (transplantable) produced by a method for producing transplantable mature intestinal organoids (rhIL-2 treatment from P0, subculture with a blade or chopper from P1, and containing 40-50 ng/ml Noggin and 200-250 ng/ml R-spondin1 in hIO medium). Expression of intestinal cell type-specific markers (VIL1, KRT20, CHGA, MUC2, and LYZ) and intestinal maturation markers (MUC13, OLFM4, SI, LCT, and DPP4 and LCT) is shown.

FIG. 1d shows comparison of transplantable immature and mature organoids through qPCR analysis, which presents the expression of intestinal stem cell markers ASCL2, AXIN2, and OLFM4.

FIG. 1e shows comparison of transplantable immature and mature organoids through qPCR analysis, which presents the expression of vascular-specific markers CD31, CDH5, ECSCR, LYVE1, and IGF2.

FIG. 1f shows comparison of conventional mature organoids and transplantable mature intestinal organoids through qPCR analysis, which presents that expression of intestinal stem cell-specific markers (ASCL2, AXIN2, LGR5, and OLFM4) and vascular endothelium-related factors (CD31, CDH5, ECSCR, and LYVE1) is highly expressed in transplantable mature intestinal organoids.

FIG. 2 shows the level of specific genes at each differentiation stage by applying clinically applicable Xeno-free ECM (ECMatrix^{™}-511) during differentiation from pluripotent stem cells into intestinal organoids.

FIG. 2a shows the cell morphology of PSC, DE (Day 3), and HG (Day 2 and 4) differentiated from existing Matrigel and ECMatrix^{™}-511, respectively.

FIG. 2b shows that the mRNA levels of representative markers (OCT4, NANOG, FOXA2, SOX17, CDX2, and VIL1) of each differentiation stage are not significantly different between Matrigel and ECMatrix^{™}-511 by qPCR.

FIG. 3 shows organoids differentiated into intestinal organoids from pluripotent stem cells in ECMatrix^{™}-511, cultured using Matrigel and clinically applicable Xeno-free ECM (VitroGel #2, #3).

FIG. 3a shows the maintenance of the intestinal epithelial structure in VitroGel #2, #3, and the histological analysis through H&E staining and cell morphology after 5 days of culture.

FIG. 3b shows expression of small intestine-specific markers (CDX2, VIL1, LGR5, ASCL2, LYZ, MUC2, and MUC13) through qPCR.

FIG. 4 shows analysis of the characteristics of mesenchymal stromal cells around transplantable intestinal organoids by immunofluorescence staining.

FIG. 4a shows the presence of mesenchymal stromal cells in conventional intestinal organoids cultured using conventional hIO production and culture method and transplantable intestinal organoids produced using a transplantable hIO culture method, which presents that compared to conventional culture methods, the transplantable culture technique maintained three well-known mesenchymal stromal cells (smooth muscle cells, intestinal subepithelial myofibroblasts; ISEMF, and Fibroblasts) up to P6.

FIG. 4b is H&E staining images of conventional hIO P2 and transplantable hIO P2, which presents the distribution of mesenchymal stromal cells around the organoid epithelium, confirming that more cells were produced in the transplantable intestinal organoids.

FIG. 4c shows immunofluorescence staining of mesenchymal stromal cells isolated from immature (Cont-hIO) or high performance mature intestinal organoids (Mat-hIO), respectively, which presents expression of not only mesenchymal stem cell markers or pericyte markers (αSMA) but also vascular markers (hCD31) in Mat-hIO and the mesenchymal stromal cells isolated from it.

FIG. 5 shows identification of the constituent cells of intestinal organoids through single cell RNAseq of Cont-hIO and Mat-hIO, respectively.

FIG. 5a shows the UMAP of Cont-hIO and Mat-hIO, wherein the left panels (2 panels) show the UMAP of the entire intestinal organoid, and the right panel is a zoomed-in view of a group of mesenchymal stromal cells, which presents the grouping of Cont-hIO mesenchymal stromal cells and Mat-hIO mesenchymal stromal cells.

FIG. 5b shows the correlation between pseudo-bulk samples.

FIG. 5c shows the analysis of clusters in Gene Ontology biological process (GOBP) by selecting differentially expressed genes (DEGs) in Mat-hIO compared to Cont-hIO in the mesenchymal stromal cell group based on scRNAseq, which presents terms related to epithelial cell adhesion and cellular vascular endothelial stimulation.

FIG. 5d shows GOTERM_BP analysis of DEG in DAVID (https://david.ncifcrf.gov/summary.jsp), confirming increases in cell adhesion, angiogenesis, and differentiation in Mat-hIO compared to Cont-hIO.

FIG. 5e shows a volcano plot of DEGs in the mesenchymal stromal cell cluster of Mat-hIO , showing representative examples of the up-regulated genes in the mesenchymal stromal cells of Mat-hIO, including IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, etc.

FIG. 6 shows xenograft intestinal organoids observed 8 weeks after transplantation of immature or high-performance mature organoids into each kidney of the same immunodeficient mouse.

FIG. 6a shows a process of transplanting immature intestinal organoids (Cont-hIO) or mature intestinal organoids (Mat-hIO) derived from induced pluripotent stem cells into each kidney of the same immunodeficient mouse, followed by excision after 8 weeks.

FIG. 6b shows the cell morphology of immature or mature intestinal organoids derived from induced pluripotent stem cells before transplantation (P0).

FIG. 6c shows the kidneys excised from mice 8 weeks after transplantation. Even when transplanted into the same mouse, vascularization is observed around the Mat-hIO xenograft (n=6).

FIG. 6d shows histological analysis of structures such as adult small intestine and vascularization by H&E staining using sections of isolated kidneys and xenografts, which presents the vascularization in Mat-hIO. Right panel shows a graph showing the measurement of Villus length of Cont-hIO and Mat-hIO xenografts.

FIG. 6e shows immunofluorescence staining of xenografts obtained from the kidneys of mice excised 8 weeks after transplantation, confirming the small intestinal epithelial structure (hECAD), mesenchymal stromal cell and pericyte markers (αSMA), and vascular markers hVE-Cad, PECAM-1 (hCD31), and VEGF. Mat-hIO xenografts exhibit expression patterns similar to human small intestinal tissue.

FIG. 7 shows comparison of the genomes of in vitro cultured immature or high-performance mature organoids (P2) and renal xenograft immature intestinal organoids (Cont-hIO) and mature intestinal organoids (Mat-hIO) at 4 or 8 weeks from RNAseq data.

FIG. 7a shows 3D MSC plot using Elucidean distance to determine the migration path of in vitro organoids 4 and 8 weeks after transplantation.

FIG. 7b is a heatmap showing the data comparing the gene expression levels of each sample, wherein 8-week-old xenograft Mat-hIO and in vitro mature organoids exhibit gene expression more similar to human small intestinal tissue.

FIG. 7c shows visualization of the functionally grouped annotation network of selected terms of 538 genes in ClueGO of Cytoscape plugin. The size of the nodes (circles) reflects the statistical significance of the terms. Edges represent the degree of connectivity between terms. Each color represents a group of GO terms. The most important term showing the difference in Mat-hIO xenograft group compared to the xenografted Cont-hIO at 8 weeks is Vascular development.

FIG. 7d shows GOTERM_BP analysis of DEG in DAVID (https://david.ncifcrf.gov/summary.jsp), confirming increases in angiogenesis, blood vessel development, vasculogenesis, cell differentiation, etc., in Mat-hIO xenografts (8 weeks) compared to Cont-hIO.

FIG. 7e shows the mRNA levels, verified by qPCR, based on the RNAseq results, of vascular markers (hCD31, ANGPT1, and KDR), mesenchymal stromal cell markers (αSMA, VIM, and DESMIN), and intestine-specific markers (VIL1, KRT20, CHGA, LYZ, DEFA5, OLFM4, MUC2, MUC13, DPP4, LCT, SLC5A1, and CREB3L3). Vascular markers (hCD31, ANGPT1, and KDR) are increased in the 8-week Mat-hIO xenograft group, and mesenchymal stromal cell marker and pericyte marker (αSMA) is increased in the 4-week Mat-hIO xenograft group.

FIG. 8 shows induced pluripotent stem cell-derived immature intestinal organoids (Cont-hIOs) or mature intestinal organoids (Mat-hIOs) transplanted into DSS-induced inflammatory bowel disease immunodeficient mice and observed after 2 or 8 weeks to verify the transplantation and engraftment ability of high-performance mature intestinal organoids (Mat-hIO).

FIG. 8a is a schematic diagram showing the protocol for transplanting reporter intestinal organoids prepared to confirm engraftment ability, into the DSS-induced inflammatory bowel disease model.

FIG. 8b shows cell morphology, GFP fluorescence expression, and histological characteristics of reporter immature or mature intestinal organoids before transplantation, and shows chopped cells before transplantation.

FIG. 8c shows the engraftment of transplanted reporter intestinal organoids tracked by in vivo fluorescence imaging system (IVIS) at 2 and 8 weeks after transplantation.

FIG. 8d shows a fluorescence microscope image confirming the xenografted reporter intestinal organoids in colon tissue excised 8 weeks after transplantation.

FIG. 8e shows the engraftment ability confirmed by immunofluorescence staining with human-specific antibodies (hECAD), of colon tissue sections transplanted with immature intestinal organoids (Cont-hIO) or Mat-hIO at 2 or 8 weeks after transplantation.

FIG. 8f shows quantification of fluorescence expression of human-specific antibody (hECAD), confirming that both 2-week and 8-week Mat-hIO xenografts had significantly better engraftment ability than Cont-hIO.

FIG. 9 shows the initial transplantation efficacy of no treatment (NT), Matrigel, immature intestinal organoids (Cont-hIO), or high-performance mature intestinal organoids (Mat-hIO) derived from induced pluripotent stem cells into immunodeficient mice with EDTA-induced intestinal epithelial injury.

FIG. 9a is a schematic diagram of transplanting induced pluripotent stem cells-derived intestinal organoids into an EDTA-induced intestinal epithelial injury model.

FIG. 9b shows representative images confirmed by colonoscopy before transplantation, after intestinal epithelial injury, and on Day 1 after transplantation. The Mat-hIO transplantation group shows reduced bleeding on day 1 of transplantation.

FIG. 9c shows histological analysis using H&E and Mucin secretion functional test using AB-PAS, of the no treatment group (NT), Matrigel, Cont-hIO, and Mat-hIO transplantation groups.

FIG. 10 shows the efficacy of transplanting immature intestinal organoids (Cont-hIO) or mature intestinal organoids (Mat-hIO) derived from induced pluripotent stem cells into immunodeficient mice with EDTA-induced intestinal epithelial injury, by applying clinically applicable biomaterials (Fibrin) in addition to Matrigel. Two weeks after transplantation, H&E shows that both Matrigel and Fibrin had similar transplant efficacy.

FIG. 11 shows the transplantation ability and crypt depth after transplantation into immunodeficient mice with EDTA-induced intestinal epithelial injury to confirm the size of intestinal organoids suitable for transplantation.

FIG. 11a is a schematic diagram showing the protocol for transplanting organoids into the EDTA-induced intestinal epithelial injury model to confirm the transplantation ability according to the size of the intestinal organoids.

FIG. 11b shows the cell morphology and histological characteristics of Mat-hIO derived from induced pluripotent stem cells before transplantation, and shows chopped cells (100 µm and 200 µm) before transplantation.

FIG. 11c shows H&E results to confirm the histological characteristics of colon tissue sections excised 1 and 2 weeks after transplantation, which presents differences between groups in histological structures at the same location within 1 cm from the anus.

FIG. 11d shows quantification using ImageJ to confirm the increase in crypt depth, a characteristic of human cell xenografts, from H&E staining, which indicates that the crypt depth significantly increased in Mat-hIO (200 µm) xenografts.

FIG. 11e shows the size-specific culture growth rate in vitro and the presence or absence of mesenchymal stromal cells. The 200 µm-sized intestinal organoids were easily structured on Day 5, indicating that they were suitable for transplantation. In addition, it shows that the 500 µm-sized intestinal organoids used in the subculture had a uniform size and the mesenchymal stromal cells around the intestinal organoids were well maintained.

FIG. 12 shows the engraftment and regeneration efficacy of induced pluripotent stem cell-derived fluorescent reporter immature intestinal organoids (Cont-hIO) and mature intestinal organoids (Mat-hIO) in immunodeficient mice with EDTA-induced intestinal epithelial injury.

FIG. 12a is a schematic diagram showing the protocol for transplanting reporter intestinal organoids with biocompatible fibrin into the EDTA-induced intestinal epithelial injury model.

FIG. 12b shows the cell morphology and GFP fluorescence expression of reporter Cont-hIO or Mat-hIO before transplantation, and shows chopped cells (200 µm) before transplantation.

FIG. 12c is a fluorescence microscope image confirming GFP-expressing Mat-hIO in colon tissue excised 1 week after transplantation.

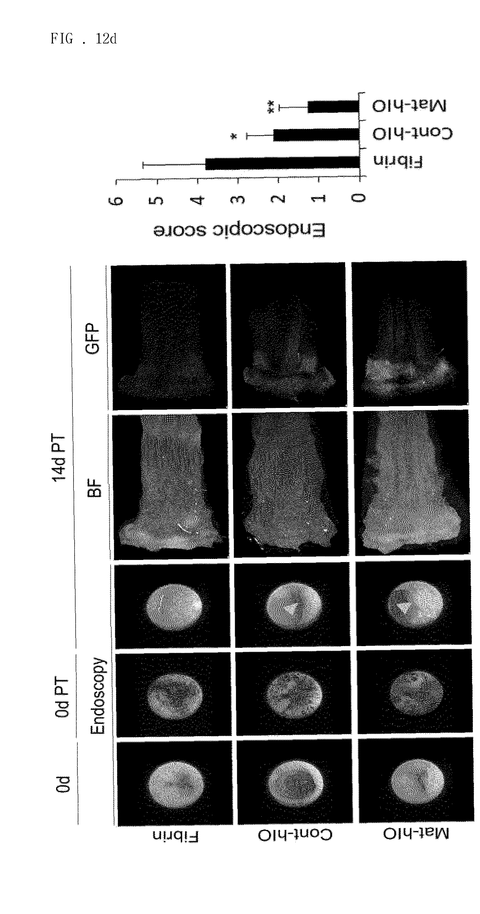

FIG. 12d shows the colonic environment of each group before transplantation, immediately after epithelial injury, and 2 weeks after transplantation, using a colonoscope for experimental animals. Quantitative assessment of injury performed through endoscopic scoring of an intestinal epithelial injury model, shows significant improvement in the Mat-hIO transplantation group, as observed by colonoscopy. In addition, it shows fluorescence dissecting microscope images confirming the status of the excised intestine and the engraftment confirmed by fluorescence expression using reporter intestinal organoids derived from induced pluripotent stem cells.

FIG. 12e shows the histological characteristics through H&E staining, and the functional properties of Goblet cells through AB-PAS staining, in the cell-transplanted rectum area. Compared to the Cont-hIO transplantation group, the Mat-hIO transplantation group shows structurally higher crypt depth and formation (restoration) rate, particularly, functionally active secretion of Mucin. Further, the successful transplantation of human intestinal organoids is demonstrated using a human-specific antibody (hCytokerain).

FIG. 12f is a graph showing quantification of the crypto depth using ImageJ. The Cont-hIO transplantation group is higher than the Fibrin (ECM only) transplantation group, and the Mat-hIO transplantation group shows a significant increase compared to the Fibrin and Cont-hIO transplantation groups.

FIG. 12g shows the transplantation site and non-transplantation site in the damaged (injured) region of the normal group (No injury), Fibrin group, and mature intestinal organoid (Mat-hIO) transplantation group, marked by H&E staining from longitudinal sections to confirm the overall pattern of crypt depth, which presents that the crypt depth of the transplanted rectal area in the Mat-hIO transplantation group is high.

FIG. 12h shows the Matrigel transplantation group, Cont-hIO transplantation group, and Mat-hIO transplantation group, confirmed through AB-PAS staining from Swiss roll sections to confirm the histological characteristics of the crypt and the functional properties of the goblet cells around the rectum. The rectum area of the Mat-hIO transplantation group shows that the intestinal epithelium is not collapsed and the secretory function of Mucin is also normal.

FIG. 13 demonstrates the engraftment and regeneration efficacy of high-performance mature intestinal organoids derived from embryonic stem cells differentiated from Xeno-free ECM in immunodeficient mice with EDTA-induced intestinal epithelial injury.

FIG. 13a is a schematic diagram showing the protocol for applying biocompatible fibrin and transplanting intestinal organoids differentiated from xeno-free ECM into an EDTA-induced intestinal epithelial injury model.

FIG. 13b shows cell morphology of immature intestinal organoids (Cont-hIO) or mature intestinal organoids (Mat-hIO) differentiated from Xeno-free ECM (hPSC to HG on the ECMatrixTM-511, hIO (P0 to P2) in Matrigel, hIO P3 in VitroGel #2 before transplantation) and chopped cells (200 µm) before transplantation.

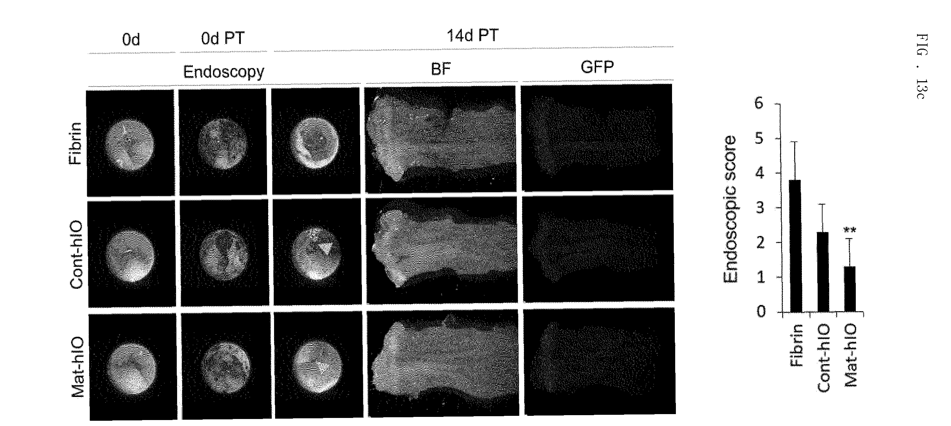

FIG. 13c shows the colonic environment of each group observed before transplantation, immediately after epithelial injury, and 2 weeks after transplantation, using a colonoscope for experimental animals. Quantitative assessment of injury as observed by colonoscopy, performed through endoscopic scoring of an intestinal epithelial injury model, shows significant improvement in the Mat-hIO transplantation group. In addition, it shows fluorescence dissecting microscope images confirming the status of the excised intestine and no fluorescence expression using embryonic stem cell-derived intestinal organoids, unlike the reporter intestinal organoids.

FIG. 13d shows the histological characteristics through H&E staining, and the functional properties of Goblet cells through AB-PAS staining, in the cell-transplanted rectum area. Compared to the Cont-hIO transplantation group, the Mat-hIO transplantation group shows structurally higher crypt depth and formation (restoration) rate, particularly, functionally active secretion of Mucin.

FIG. 13e is a graph showing quantification of the crypto depth using ImageJ. The Cont-hIO transplantation group is higher than the Fibrin (ECM only) transplantation group, and the Mat-hIO transplantation group shows a significant increase compared to the Fibrin and Cont-hIO transplantation groups.

FIG. 13f shows transplantation site and non-transplantation site in the damaged (injured) region of the Mat-hIO transplantation group from longitudinal sections to confirm the overall pattern of crypt depth.

FIG. 14 shows the engraftment and regeneration efficacy of reporter high-performance mature intestinal organoids derived from induced pluripotent stem cells in immunodeficient mice with DSS-induced inflammatory bowel disease.

FIG. 14a is a schematic diagram showing the protocol for transplanting reporter intestinal organoids with biocompatible fibrin into the DSS-induced intestinal epithelial injury model.

FIG. 14b is a graph showing the weight gain and loss compared to day 0 of transplantation, which indicates that the immature intestinal organoid (Cont-hIO) transplantation group had a weight gain compared to the Fibrin group, and in particular, the mature intestinal organoid (Mat-hIO) transplantation group exhibited an excellent increase from day 5.

FIG. 14c shows representative images observed under a dissecting microscope before transplantation to confirm the cell morphology and GFP fluorescence expression of fluorescent reporter immature intestinal organoids (Cont-hIO) or mature intestinal organoids (Mat-hIO) before transplantation, and shows chopped (200 µm) cells.

FIG. 14d is a fluorescence microscope image confirming GFP-expressing Mat-hIO in colon tissue excised 1 and 2 weeks after transplantation.

FIG. 14e shows colonoscopy for experimental animals to observe inflammation in the colon of each group before transplantation, 1 week, and 2 weeks after transplantation. It shows a significant decrease in bleeding and inflammation in the Mat-hIO transplantation group after 2 weeks. Quantitative assessment of inflammation-injury performed through endoscopic scoring of an intestinal epithelial injury model, shows significant improvement in the Mat-hIO transplantation group, as observed by colonoscopy.

FIG. 14f shows the histological characteristics through H&E staining, and the functional properties of Goblet cells through AB-PAS staining, from transverse or longitudinal sections of the cell-transplanted rectum area. Compared to the Cont-hIO transplantation group, the Mat-hIO transplantation group shows structurally higher crypt depth and formation (restoration) rate, particularly, functionally active secretion of Mucin.

FIG. 14g shows the transplantation sites of Fibrin group and mature intestinal organoid (Mat-hIO) transplantation group, marked by H&E staining from longitudinal sections to confirm the overall pattern of crypt depth, which indicates the generation of epithelial structure at the transplanted rectal site in the Mat-hIO transplantation group.

FIG. 14h shows the xenograft by immunofluorescence staining using human-specific antibody (hECAD).

FIG. 15 shows the engraftment and regeneration efficacy 4 weeks after transplantation of high-performance mature intestinal organoids derived from embryonic stem cells in immunodeficient mice with DSS-induced inflammatory bowel disease.

FIG. 15a is a schematic diagram showing the protocol for transplanting intestinal organoids with biocompatible fibrin into a DSS-induced inflammatory bowel disease model followed by excision after 6 weeks.

FIG. 15b is a graph showing the weight gain and loss at 4 weeks (day 28) compared to 1 day after transplantation, which indicates that the immature intestinal organoid (Cont-hIO) transplantation group and the mature intestinal organoid (Mat-hIO) transplantation group exhibited a significant increase compared to the fibrin group.

FIG. 15c shows colonoscopy for experimental animals to observe inflammation in the colon of each group before transplantation, and 4 week after transplantation, which presents a significant decrease in bleeding and inflammation in the Mat-hIO transplantation group after 4 weeks.

FIG. 15d shows the histological characteristics through H&E staining, and the functional properties of Goblet cells through AB-PAS staining, from longitudinal sections of the cell-transplanted rectum area. Compared to the Cont-hIO transplantation group, the Mat-hIO transplantation group shows structurally higher crypt depth and formation (restoration) rate, particularly, functionally active secretion of Mucin.

FIG. 15e shows the xenograft by immunofluorescence staining using human-specific antibody (hECAD), which presents high expression of the human-specific antibody in the Mat-hIO transplantation group.

FIG. 16 shows the inflammation level and safety 6 months after transplantation of high-performance mature intestinal organoids derived from induced pluripotent stem cells in immunodeficient mice with DSS-induced inflammatory bowel disease.

FIG. 16a is a schematic diagram showing the protocol for transplanting intestinal organoids with biocompatible fibrin into a DSS-induced inflammatory bowel disease model, followed by excision after 6 weeks.

FIG. 16b shows representative colonoscopic images for experimental animals observing the colonic environment of the control group (no injury), Fibrin transplantation group, Cont-hIO transplantation group, and Mat-hIO transplantation group, which indicates that no intestinal inflammation or abnormal tissue (mass) was found 6 months after transplantation.

FIG. 16c shows histological characteristics through H&E staining from longitudinal sections of the cell-transplanted rectum area, which presents similar patterns of epithelial tissue in Control, Fibrin, Cont-hIO, and Mat-hIO groups.

FIG. 16d shows the xenografts by immunofluorescence staining using human-specific antibody (hECAD), which indicates that there was no expression of the human-specific antibody (hECAD) in all transplantation groups, demonstrating no xenografts remained after 6 months.

FIG. 16e shows the excision of the 10 major organs (large intestine (referred to as colon), small intestine, spleen, kidney, lung, liver, stomach, heart, testis, and brain) including the transplanted large intestine. All groups showed no abnormalities upon visual observation.

FIG. 16f shows histological characteristics of the 10 major organs in the Cont-hIO and Mat-hIO transplantation groups, using H&E staining, which presents no ectopic tissue in any organ, and the same histological characteristics as the normal control group.

FIG. 16g is a graph showing the survival rate of the transplantation group after 6 months (190 days) after transplantation. The Fibrin transplantation group (n=16), Cont-hIO transplantation group (n=9), and Mat-hIO transplantation group (n=16) showed survival rates of 31.3% (Fibrin), 44.4% (Cont-hIO), and 56.3% (Mat-hIO), respectively.

FIG. 17 shows the isolation, culture, and freeze-thaw techniques of mesenchymal stromal cells from transplantable mature intestinal organoids derived from pluripotent stem cells.

FIG. 17a is a schematic diagram of the isolation, culture, and freeze-thaw protocol of mesenchymal stromal cells from the intestinal organoids, which presents maturation of the differentiated human intestinal organoids (hIOs) derived from the pluripotent stem cells through IL-2 treatment from P0 for maturation, wherein mesenchymal stromal cells (MSC^{hIO}) isolated from intestinal organoids P0-P2 are able to be cultured for 3-7 days, followed by subculture, freezing, and thawing.

FIG. 17b shows the phenotypes of immature intestinal organoids (Cont-hIO), mature intestinal organoids (Mat-hIO), differentiated from pluripotent stem cells, and mesenchymal stromal cells isolated therefrom (MSC^{Cont-hIO} and MSC^{Mat-hIO}), which presents representative images of subculture from P0 to P10 after isolation.

FIG. 17c shows the results of qPCR analysis by passage (P2, P3, P4, and P8) of markers (αSMA (ACTA2), Desmin, and Vimentin) of representative cell types of mesenchymal stromal cells, including smooth muscle cells or pericytes, intestinal subepithelial myofibroblasts (ISEMF), and fibroblasts, which indicates that markers of intestinal mesenchymal stromal cells are still maintained across generations.

FIG. 18 shows the genetic level characterization of immature intestinal organoid-derived mesenchymal stromal cells (MSC^{Cont-hIO}), mature intestinal organoid-derived mesenchymal stromal cells (MSC^{Mat-hIO}), mesenchymal stem cells differentiated from pluripotent stem cells (MSC^{PSC}), and commercially available human mesenchymal stromal cells.

FIG. 18a shows immunofluorescence staining results of mesenchymal stromal cell marker and pericyte marker (αSMA), and vascular marker (CD31), which presents high expression of αSMA and CD31 in MSC^{Cont-hIO}.

FIG. 18b is a heatmap showing the significant correlation between each sample through Spearman correlation, which indicates that the higher the similarity between samples, the closer it is to red, and that the closer it is to blue, the lower the similarity.

FIG. 18c shows the MDS plot confirmed through gene expression analysis of fold > 2 from RNAseq data, which indicates that MSC^{Mat-hIO} also has different characteristics from MSC^{Cont-hIO}.

FIG. 18d shows EnrichR and Ontology analysis confirming the Endothelial cell type in MSC^{Mat-hIO} compared to MSC^{Cont-hIO}.

FIG. 18e shows the gene expression of vascular endothelial-related factors (CD31 (PECAM1), CDH5, ECSCR, and LYVE1) in Fibroblast, iPSC, MSC^{Cont-hIO}, MSC^{Mat-hIO}, and hMSC, confirmed through qPCR, which presents a significant increase in the isolated MSC^{Mat-hIO}.

FIG. 18f shows the vascular endothelial-related factors (VEGF) secreted from MSC^{Cont-hIO} and MSC^{Mat-hIO} in the conditioned medium, confirmed through ELISA.

### [Best Mode]

The following exemplary embodiments are presented to help understanding of the present disclosure. These Examples and Preparation Examples are only provided to more easily understand the present disclosure, but the content of the present disclosure is not limited by these Examples.

### Experimental Example 1. Cell culture and iPSC production

Human pluripotent stem cells (hPSCs) including human embryonic stem cells (hESCs) and human induced pluripotent stem cells (hiPSCs) were cultured by the known method (Molecular carcinogenesis 55, 387-396 (2016), Proteomics 15, 2220-2229 (2015)). Non-integrating-hiPSCs were transfected by electroporation and reprogrammed using Episomal iPSC reprogramming vector (Cat. No. A14703. Invitrogen, Carlsbad, CA, USA) according to the known method.

After 5 days of electroporation, fibroblasts were plated in 1 x 10⁵/well on Matrigel (BD Biosciences, San Diego, CA, USA)-coated 6-well plates, and cultured in E8 medium (Stem Cell Technologies, Vancouver, Canada). After 3 weeks, hiPSC colonies were selected, and the cell number was increased for passage and subsequent characterization.

### Experimental Example 2. Differentiation from hPSC into intestinal organoid (hIO) for production of immature intestinal organoid (Cont-hIO)

Human intestinal organoids (hIOs) were produced using the known method (Nature 470, 105-109 (2011)). To induce definitive endoderm, hPSCs were plated on Matrigel or dishes coated with ECMatrix^{™}-511 and treated with 100 ng/ml of Activin A (R&D Systems, Minneapolis, MN, USA) in an RPMI 1640 medium having dialyzed fetal bovine serum (dFBS, HyClone, Thermo Fisher Scientific Inc., Waltham, MA, USA) at concentrations of 0%, 0.2% and 2% for 3 days. In addition, in order to perform differentiation into 3D hindgut spheroids, 500 ng/ml of FGF4 (R&D Systems) and 3 µM of CHIR99021 (TOCRIS) were treated together with the RPMI 1640 medium containing 2% of dFBS for 4 to 6 days. From day 4 after induction into the hindgut, spheroids were embedded in Matrigel (BD Biosciences), cultured in a hIO medium (2 mM of L-glutamine, 1% of penicillin-streptomycin, and 15 mM of HEPES buffer in Advanced DMEM F12) including 1X B27 (Invitrogen), 200 to 250 ng/ml of R-spondin 1 (R&D Systems), 100 ng/ml of EGF (R&D Systems), and 40 to 50 ng/ml of Noggin (R&D Systems), and subcultured every 10 to 14 days. At the time of subculture, the cells were cut into 500 um x 500 um pieces using a McIlwain Tissue Chopper and then cultured. After P2, specific cell passages (especially before transplantation) were performed with Vitrogel, and Xeno-free testing was performed.

### Experimental Example 3. Culture technique for producing high-performance mature intestinal organoid (Mat-hIO)

After the differentiation into the hindgut, interleukin-2 (hereinafter, rhIL-2 (R&D Systems)) at a concentration of 1 ng/ml (approximately 13 U/ml) was immediately added to the hIO medium daily at the hIO (P0) stage inserted into Matrigel. The hIO medium was treated with 1 ng/ml rhIL-2 every day until hIO P1, and subsequently, starting from hIO P2, the culture medium could be replaced every two days, with the option to treat with rhIL-2 or omit this step. In particular, in order to maintain the peripheral stromal cells, the cells were cut to a certain size level (500 um x 500 um) or more using a chopper, etc., and subcultured. Since the peripheral stromal cells were not isolated arbitrarily, the characteristics of high-performance mature intestinal organoids could be maintained. During the culture process, VitroGel was applied at specific passages (particularly before transplantation) to conduct the experiment.

### Experimental Example 4. Isolation and culture of mesenchymal stromal cells from immature (Cont-hIO) and mature intestinal organoid (Mat-hIO)

For mesenchymal stromal cell culture, 0.2% gelatin was coated on the culture dish for 1 hour. When subculturing intestinal organoids from immature (Cont-hIO) and mature intestinal organoids (Mat-hIO) P0~P2, the organoids were isolated from the Matrigel dome, and the mesenchymal stromal cells present in the Matrigel dome and the bottom were washed with ice-Advanced DMEM/F12. Afterwards, the cells were treated with Trypsin-EDTA (TE) or TrypLE^{™} (Gibco) at 37°C for 3-5 minutes to be isolated into single cells, followed by washing once with hIO medium. Cells collected by centrifugation were cultured in a pre-gelatin-coated culture dish with hIO medium containing 1x B27 and 100 ng/ml EGF. Mature intestinal organoid-derived mesenchymal stromal cells could be treated with rhIL-2 (1 ng/ml) while changing the culture medium once every two days from P0 to P1. Subculture was performed in the same manner at a ratio of 1 : 3 after 5-7 days.

### Experimental Example 5. Freezing and thawing of mesenchymal stromal cells

For freezing mesenchymal stromal cells, at 3-5 days after subculture, the cells were treated with TE at 37°C for 3 to 5 minutes in the same manner as the subculture process to be isolated into single cells, followed by washing once with hIO medium. Then, the cells were frozen in freezing medium (Recovery^{™} Cell Culture Freezing Medium, Gibco) and stored long-term in an LN₂ Tank. A warm medium (37°C) was prepared in advance to thaw the frozen mesenchymal stromal cells. After rapid thawing of the frozen cells, the cells were washed once with hIO medium, and cultured in hIO medium containing 1x B27 and 100 ng/ml EGF in a pre-gelatin-coated culture dish. Mature intestinal organoid-derived mesenchymal stromal cells could be treated with rhIL-2 (1 ng/ml) while changing the culture medium once every two days. Depending on the condition of the cells upon initial thawing, the cells were cultured for 3~7 days and then subcultured in the same manner.

### Experimental Example 6. Quantitative real-time RT-PCR (qRT-PCR)

Total RNA was extracted from cells using the RNeasy kit (Qiagen) and reverse transcribed using the Superscript III cDNA synthesis kit (Invitrogen). qRT-PCR was performed on the 7500 Fast Real-time PCR system (Applied Biosystems, Foster City, CA, USA) according to the known method (Cho et al., Oncotarget 6, 23837-23844, 2015). All experiments were repeated three times, and the CT value of each target gene was calculated using the software provided by the manufacturer. The base sequences of the primers used are shown in Table 1.

**[Table 1]**

| **Gene** | **Primer (Forward)** | **SEQ ID NO:** | **Primer (Reverse)** | **SEQ ID NO:** |
|---|---|---|---|---|
| *GAPDH* | GAA GGT GAA GGT CGG AGT C | 1 | GAA GAT GGT GAT GGG ATT TC | 2 |
| *VIL1* | AGC CAG ATC ACT GCT GAG GT | 3 | TGG ACA GGT GTT CCT CCT TC | 4 |
| *KRT20* | TGG CCT ACA CAA GCA TCT GG | 5 | TAA CTG GCT GCT GTA ACG GG | 6 |
| *CHGA* | TGA CCT CAA CGA TGC ATT TC | 7 | CTG TCC TGG CTC TTC TGC TC | 8 |
| *MUC2* | TGT AGG CAT CGC TCT TCT CA | 9 | GAC ACC ATC TAC CTC ACC CG | 10 |
| *MUC13* | CGG ATG ACT GCC TCA ATG GT | 11 | AAA GAC GCT CCC TTC TGC TC | 12 |
| *LYZ* | AAA   ACC   CCA   GGA GCA GTT AAT | 13 | CAA CCC TCT TTG CAC AAG CT | 14 |
| *OLFM4* | ACC TTT CCC GTG GAC AGA GT | 15 | TGG ACA TAT TCC CTC ACT TTG GA | 16 |
| *SI* | GGT AAG GAG AAA CCG GGA AG | 17 | GCA CGT CGA CCT ATG GAA AT | 18 |
| *LCT* | TCC TCG CCT ACA ACC TCA AC | 19 | GCT GGG ACA CTC CAT TCT CT | 20 |
| *DPP4* | CAG AAG ACT GGG AAC ATT TGA | 21 | CAC ACT TGA ACA CGC CAC TT | 22 |
| *DEFA5* | CCT   TTG   CAG   GAA ATG GAC TC | 23 | GGA CTC ACG GGT AGC ACA AC | 24 |
| *SLC5A1* | GTG CAG TCA GCA CAA AGT GG | 25 | ATG CAC ATC CGG AAT GGG TT | 26 |
| *CREB3L3* | ATC   TCC   TGT   TTG ACC GGC AG | 27 | GTC GTC AGA GTC GGG GTT TG | 28 |
| *PECAM1 (CD31)* | TGC GGC CAA ATG TTA TTT TC | 29 | TGC ATG GTT TCT GAC ATC GT | 30 |
| *CDH5* | CAG CCC AAA GTG TGT GAG AA | 31 | CGG TCA AAC TGC CCA TAC TT | 32 |
| *ECSCR* | GAG CAT GAG GGA AGA TGC GAC | 33 | TGA CCA CAC CAA CTA GGA TGA | 34 |
| *LYVE1* | GCT TTC CAT CCA GGT GTC AT | 35 | ACC ACG AAT CCA TCT CCA AC | 36 |
| *OCT4* | GCT CGA GAA GGA TGT GGT CC | 37 | CGT TGT GCA TAG TCG CTG CT | 38 |
| *NANOG* | GCA GAA GGC CTC AGC ACC TA | 39 | AGG TTC CCA GTC GGG TTC A | 40 |
| *FOXA2* | TGG GAG CGG TGA AGA TGG AAG GGC AC | 41 | TCA TGC CAG CGC CCA CGT ACG ACG AC | 42 |
| *SOX1 7* | CGC TTT CAT GGT GTG GGC TAA GGA CG | 43 | TAG TTG GGG TGG TCC TGC ATG TGC TG | 44 |
| *CDX2* | CTG GAG CTG GAG AAG GAG TTT C | 45 | ATT TTA ACC TGC CTC TCA GAG AGC | 46 |
| *LGR5* | TGC TCT TCA CCA ACT GCA TC | 47 | CTC AGG CTC ACC AGA TCC TC | 48 |
| *ASCL2* | CGTGAAGCTGGTGAACTT GG | 49 | GGATGTACTCCACGGCTGAG | 50 |
| *AXIN2* | GAG TGG ACT TGT GCC GAC TTC A | 51 | GGT GGC TGG TGC AAA GAC ATA G | 52 |
| *ANGPT1* | GGG GGA GGT TGG ACT GTA AT | 53 | GAA TAG GCT CGG TTC CCT TC | 54 |
| *KDR* | CCC   ACG   TTT   TCA GAG TTG GT | 55 | CGG CTC TTT CGC TTA CTG TT | 56 |
| *ACTA2 (αSMA)* | TTC AAT GTC CCA GCC ATG TA | 57 | GAA GGA ATA GCC ACG CTC AG | 58 |
| *VIM* | AGA ACG TGC AGG AGG CAG AAG AAT | 59 | TTC CAT TTC ACG CAT CTG GCG TTC | 60 |
| *DES* | CAG TGG CTA CCA GGA CAA CA | 61 | CTC AGA ACC CCT TTG CTC AG | 62 |
| *IGF2* | GTG GCA TCG TTG AGG AGT G | 63 | CAC GTC CCT CTC GGA CTT G | 64 |

### Experimental Example 7. Cell and tissue immunofluorescence

Immunofluorescence was performed according to the known method (Kwak et al., Biochemical and biophysical research communications 457, 554-560, 2015). Specifically, hPSCs and definitive endoderm cells were fixed with 4% paraformaldehyde (PFA) and permeabilized with PBS containing 0.1% Triton X-100.

hIO and tissues were fixed, cryoprotected with sucrose, and frozen using optimal cutting temperature (OCT) compound (Sakura Finetek, Tokyo, Japan). Then, frozen sections were cut into 10 µm using a cryostat microtome at -20°C and permeabilized with PBS containing 0.1% Triton X-100 for immunofluorescence analysis. Alternatively, the fixed whole organoids were permeabilized with PBS containing 0.1% Triton X-100 and then proceeded to the next step.

Specifically, after blocking with 4% BSA, the cells were reacted with the primary antibodies overnight at 4°C. Then, the cells were reacted with the secondary antibodies for 1 hour at room temperature. The primary antibodies used are listed in Table 2. DAPI was added to visualize nuclei. The slides were observed using an EVOS FL Auto2 (ThermoFisher) and an Axiovert 200M microscope (Carl Zeiss, Gottingen, Germany) or a fluorescence microscope (IX51, Olympus, Japan).

**[Table 2]**

| **Antibody** | **Catalog No.** | **Manufacturer** | **Dilution Ratio** |
|---|---|---|---|
| anti-E-Cadherin | 610182 | BD Biosciences | 1:200 for IHC |
| anti-E-Cadherin | AF648 | R&D systems | 1:500 for IHC |
| anti-E-Cadherin | Ab1416 | abcam | 1:100 for IHC |
| anti-Cytokeratin | 349205 | BD Biosciences | 25 µg/mL |
| anti-α-SMA | A5228 | Sigma | 1:500 for IHC |
| anti-Desmin | ab907 | Chemicon | 1:50 for IHC |
| anti-Vimentin | 5741S | CST | 1:100 for IHC |
| anti-hCD31 | MA5-15336 | Thermo Scientific | 1:400 for IHC |
| anti-PECAM-1 | sc-376764 | Santa Cruz | 1:50 for IHC |
| anti-VE-Cadherin | AF938 | R&D systems | 5-15 µg/mL |
| anti-VEGF | sc-7269 | Santa Cruz | 1:50 for IHC |

| | | | |
|---|---|---|---|
| *IHC: Immunohistochemistry | | | |

### Experimental Example 8. RNA sequencing and RNA quantification

For RNA sequence determination and quantification, first, RNA samples were prepared with an RNA Integrity Number (RIN) value of 7.5 or more through the Agilent 2100 Bioanalyzer system (Agilent Biotechnologies, Palo Alto, USA), and mRNA libraries were prepared through the Illumina TruSeq kit, followed by sequencing using Illumina HiSeq2500 machines (Illumina, San Diego, CA, USA). A sequencing quality was determined through a FastQC package, and a trimmed read length of 50 bases or less was excluded. Then, mapping was performed through HISAT2 (v2.0.5), with reference to the hg19 for the human genome information. Differentially expressed genes (DEGs) between samples were analyzed using Cuffquant and Cuffnorm (Cufflinks v2.2.1).

### Experimental Example 9. Bioinformatic analysis

Bioinformatic analysis was performed using IPA analysis software (Ingenuity systems, Redwood City, CA, USA), protein analysis through evolutionary relationships (PANTHER, http://www.pantherdb.org) database, and DAVID bioinformatics resource 6.7 (http://david.abcc.ncifcrf.gov). Functionally grouped Gene Ontology (GO)/pathways were analyzed using the Cytoscape software platform (version 3.3.0, http://www.cytoscape.org/what is cytoscape.html) in conjunction with ClueGO plug-in (Version 2.2.5, http://apps.cytoscape.org/apps/cluego). Additionally, Gene Ontology (GO)/pathway to identify cell types was analyzed using EnrichR (https://maayanlab.cloud/Enrichr/).

### Experimental Example 10. Single cell RNA sequencing and analysis

Sequencing data were processed using the 10x Genomics pipeline CellRanger (version 7.0.0) and a filtered gene-barcode matrix was generated using default parameters. The filtered gene-barcode matrix was used as input for downstream analysis using the R package Seurat (version 4.1.1). Cells with at least 200 genes and 9000 or more genes and cells with a mitochondrial fraction greater than 25% were removed to eliminate potential low-quality cells and doublets. Afterwards, SCtransform (version 0.3.3) was performed to normalize each dataset, and sample integration was prepared via standard correlation analysis to identify the largest source of variation preserved across all samples. Highly variable genes (HVGs) were identified using the FindVariableFeatures function, and then multiple sample data were integrated using the FindIntegrationAnchors and Integrateddata functions. After integrating the samples, primary components (PCs) were determined, and the first 50 PCs were used for cell clustering using the K-nearest neighbor graph with a clustering resolution of 0.8. Cell clusters were visualized using UMAP. To determine differentially expressed genes between conditions in each cluster, the FindMarkers function was used with the Wilcoxon Rank Sum test (min.pct=0.25, logfc.threshold=0.25) and p-value adjustment by Bonferroni correction.

### Experimental Example 11. Enzyme-linked immunosorbent assay (ELISA)

Mesenchymal stromal cells isolated from immature or mature intestinal organoids were cultured for 5 days, and the conditioned medium was collected to measure factors secreted from the cells. The level of VEGF, a vascular secretory factor, was measured according to the published experimental method using the VEGF Huma ELISA Kit (R&D Systems), and the measured value was normalized to the total protein concentration of the total cells.

### Experimental Example 12. Histological (Hematoxylin & Eosin, H&E) staining experiment

For histopathological analysis, colon tissues were fixed with 10% formalin in transverse, Swiss-roll, and longitudinal forms, and cultured in 10%, 20%, and 30% sucrose solutions in sequence for cryopreservation. Then, the tissues were embedded in Tissue-Tek^{®} O.C.T compound (Sakura^{®} Finetek, Tokyo, Japan), cryosectioned at 5 - 10 µm thickness, and adhered to slide glasses, followed by H&E staining according to the known method. The slides were observed through an optical microscope (BX53F, Olympus, Japan).

### Experimental Example 13. PAS/Mucicarmine staining experiment

Mature functional goblet cells were identified in mature intestinal organoids using a technique that stains mucin secreted by mature goblet cells. First, the control group and mature intestinal organoids were fixed with 4% paraformaldehyde and then further fixed with 10%, 20%, and 30% sucrose solutions. Then, the sample tissues were frozen using OCT, cryosectioned at 10 µm thickness using a cryostat microtome, and adhered to slide glasses, followed by staining using the Alcian Blue PAS Stain Kit. The slides were observed through an optical microscope (BX53F, Olympus, Japan).

### Experimental Example 14. Renal capsule transplantation

NSG mice (8-12 weeks old) were housed in a standard animal maintenance facility. For xenotransplantation, organoids (P1) cultured for 10 days post passage in hIO medium containing 1X B27, 100 ng/ml EGF were isolated from matrigel and washed with cold hIO medium. The organoids were stored in cold hIO medium. Before transplantation, mice were fed a standard diet without fasting and were anesthetized with 2% isoflurane (Butler Schein) by inhalation. The posterior ribs on both sides of the mouse were cleaned with isopropyl alcohol and povidine-iodine, and then incised. The kidneys were exposed and one Cont-hIO and one Mat-hIO were transplanted subrenally in each kidney. The kidney was then returned to the abdominal cavity, and the double layer of skin was closed with suture. After 8 weeks of engraftment, the mice were humanely euthanized, and the renal tissue xenograft was observed using an inverted fluorescence dissecting microscope (SZX16, Olympus).

### Experimental Example 15. EDTA-induced intestinal epithelial injury model

EDTA-induced intestinal epithelial injury model was created using the knkown method (Cell Stem Cell 22(2):171-176 (2018). NSG or NIG mice (male, 6-12 weeks old) were fed a standard diet without fasting, and maintained at a constant temperature (20-22°C) on a 12-hour day/night cycle. The mice were anesthetized with an intraperitoneal injection of 250-500 mg/kg Tribromoethanol (Avertin). The colon of mice was washed with PBS by inserting a thin catheter to remove the contents in the intestine. Then, 200 ul of 50°C 250 mM EDTA/PBS was slowly injected into the intestinal lumen for 2 minutes to injure the anal margin, rectum. After 2 minutes of exposure to EDTA/PBS, the intestinal lumen was washed with PBS for 1 minute. Then, an electric toothbrush equipped with a soft interdental brush (EW-DL22, EW0945, Panasonic Holdings Corp., Japan) was used to create an epithelial abrasion for 1 minute. Here, the brush head was inserted 1.5 cm into the colon to scrape the intestinal epithelial surface in a circular motion, and after 1 minute, success was confirmed if an isolated crypt was observed when washing the brush with PBS. The intestinal lumen was then washed again with PBS.

### Experimental Example 16. Inflammatory bowel disease model (DSS-induced colitis model)

NSG or NIG mice (male, 6-12 weeks old) were fed a standard diet without fasting, and maintained at a constant temperature (20-22°C) on a 12-hour day/night cycle. The DSS-induced colitis model was administered drinking water containing 3 (initial experiment) to 5% (w/v) DSS (36-50 kDa; MP Biomedicals, Hampton, NH, USA) for 5-7 days. Mice were administered drinking water to remove DSS during the 2 (initial experiment) to 3-day digestion period and prepared for transplantation. The DSS-induced inflammatory bowel model was subjected to daily measurements of body weight, stool consistency, and bleeding parameters to assess disease activity index (DAI).

### Experimental Example 17. Colonic transplantation

For xenotransplantation, organoids grown in Matrigel for 10 days were isolated from Matrigel and washed with cold hIO medium. 3D cells, hIO, were cut into a certain size, 200 um x 200 um, using a chopper before transplantation, and suspended in Matrigel/Advanced DMEM/F12 (1 : 10) or Fibrin. To determine size-dependent transplantation ability, cells were cut to a size (100 um x 100 um or 200 um x 200 um) and suspended in Matrigel/Advanced DMEM/F12 (1 : 10). A 100 ul suspension contained 1 to 2 x 10⁶ cells. Prior to transplantation, mice were not fasted, fed a standard diet, and anesthetized with an intraperitoneal injection of 250-500 mg/kg Tribromoethanol (Avertin). The colon of mice was washed with PBS through a thin catheter to remove the contents in the intestine. A 200 ul pipette, a soft catheter, or an endoscopic catheter was used to conduct injection into the lumen of the colon of the diseased mouse. In the Matrigel transplantation group, the anal verge was attached with adhesive (Vetbond Tissue Adhesive) (3M, USA) for 8-12 hours to promote the retention of transplanted cells in the intestine after injection. For the fibrin graft group, the process of attaching the anal verge was omitted. At each observation period, the mice were humanely euthanized and the xenografts were isolated for analysis. To confirm the xenografts in the colonic tissue, the xenografts were observed using an inverted fluorescence dissecting microscope (SZX16, Olympus) or an Axiovert 200M microscope (Carl Zeiss, Göttingen, Germany).

### Experimental Example 18. Colonoscopy

To determine the degree of inflammation and environment in the intestine, mice were anesthetized with intraperitoneal injection of Avertin before endoscopy. The colon of mice was washed with PBS through a thin catheter to remove the contents in the intestine. Medical gas was administered using an air pump, and a rigid HOPKINS endoscope (Karl Storz) was carefully inserted into the rectum, allowing for the recording of endoscopic images. The quantitative evaluation of colonoscopy was based on different models of intestinal injury, and the scoring criteria are shown in Tables 3, 4, 5, and 6.

**[Table 3]**

| EDTA-induced intestinal epithelial injury (Injury score, Day0 PT) | | | | |
|---|---|---|---|---|
| Endoscopic score | | | | |
| EDTA injury | None | Mild | Moderate | Severe |
| Erythema | 0 | 1 | 2 | 3 |
| Bleeding | 0 | 1 | 2 | 3 |
| Intestinal epithelial damage (erosion) | 0 | 1 | 2 | 3 |
| Epithelial damage area | 0 | 1 | 2 | 3 |
| Total score | | | | |

**[Table 4]**

| EDTA-induced intestinal epithelial injury (post-transplantation, PT) | | | | |
|---|---|---|---|---|
| Endoscopic score | | | | |
| Transplantation | None | Mild | Moderate | Severe |
| Erythema and bleeding | 0 | 1 | 2 | 3 |
| Intestinal epithelial damage (erosion) | 0 | 1 | 2 | 3 |
| Inflammation from wound | 0 | 1 | 2 | 3 |
| Total score | | | | |

**[Table 5]**

| DSS-induced colitis (Injury score, Day0 PT) | | | | |
|---|---|---|---|---|
| Endoscopic score | | | | |
| Injury | None | Mild | Moderate | Severe |
| Erythema | 0 | 1 | 2 | 3 |
| Bleeding | 0 | 1 | 2 | 3 |
| Mucosal changes | 0 | 1 | 2 | 3 |
| Erosion | 0 | 1 | 2 | 3 |
| Ulceration | 0 | 1 | 2 | 3 |
| Ulcer area | 0 | 1 | 2 | 3 |
| Total score | | | | |

**[Table 6]**

| DSS-induced colitis (post-transplantation, PT) | | | | |
|---|---|---|---|---|
| Endoscopic score | | | | |
| Transplantation | None | Mild | Moderate | Severe |
| Erythema and bleeding | 0 | 1 | 2 | 3 |
| Mucosal changes | 0 | 1 | 2 | 3 |
| Erosion | 0 | 1 | 2 | 3 |
| Ulceration | 0 | 1 | 2 | 3 |
| Ulcer area | 0 | 1 | 2 | 3 |
| Total score | | | | |

### Experimental Example 19. In vivo fluorescence imaging

To confirm the transplantation of hIO, reporter hIO derived from iPS cells were transplanted into the colon of mice. For in vivo fluorescence imaging, 2 or 8 weeks after transplantation, the transplanted mice were humanely euthanized and the xenografts were isolated for analysis. The intestinal tissue was placed in a sealed chamber with a charge-coupled device camera attached. The fluorescence intensity of each region of interest was measured using in vivo imaging system (IVIS Lumina II, Xenogen Corp., Alameda, CA, USA) with emission at 780 nm and excitation at 750 nm.

### Experimental Example 20. Statistical analysis

All in vitro experimental results are expressed as mean ± standard error of the mean (s.e.m.), with at least three replicates. P-values were determined using two-tailed Student's t-test or one-way ANOVA. Analysis of the length of crypt depth was determined using Welch's t-test. Analysis of endoscopic scoring between groups was determined using the two-tailed non-parametric Mann-Whitney U test. All analyses of statistical significance were calculated relative to the control group unless otherwise noted.

### Example 1. hPSC-derived hIO production and culture for transplantation

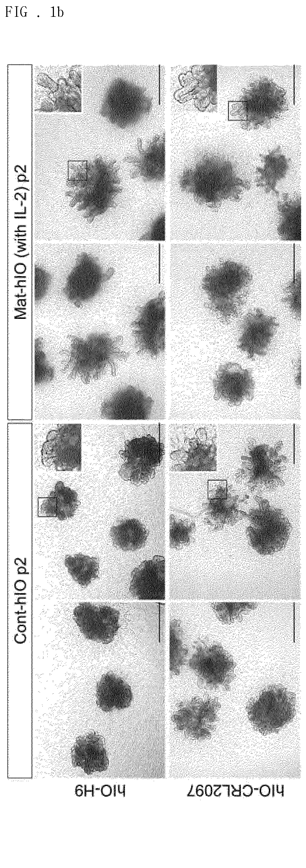

As described above, hIOs were produced from hPSCs containing hESC lines or non-integrating-hiPSC lines, using the hIO differentiation protocol. Then, the culture process of mature and immature organoids produced by treatment with 1 ng/ml rhIL-2 from P0 (immediately after hind gut production) was shown as a schematic diagram (FIG. 1a). Transplantable immature and high-performance mature intestinal organoids derived from embryonic stem cells or induced pluripotent stem cells were produced through passage culture using a surgical blade or McIlwain Tissue Chopper. Then, the expression levels of small intestine-specific genes (VIL1, KRT20, CHGA, MUC2, MUC13, LYZ, OLFM4, SI, LCT, and DPP4) were compared with those of immature and mature intestinal organoids subcultured using conventional methods, confirming small intestine-specific gene expression (FIGS. 1b and 1c).

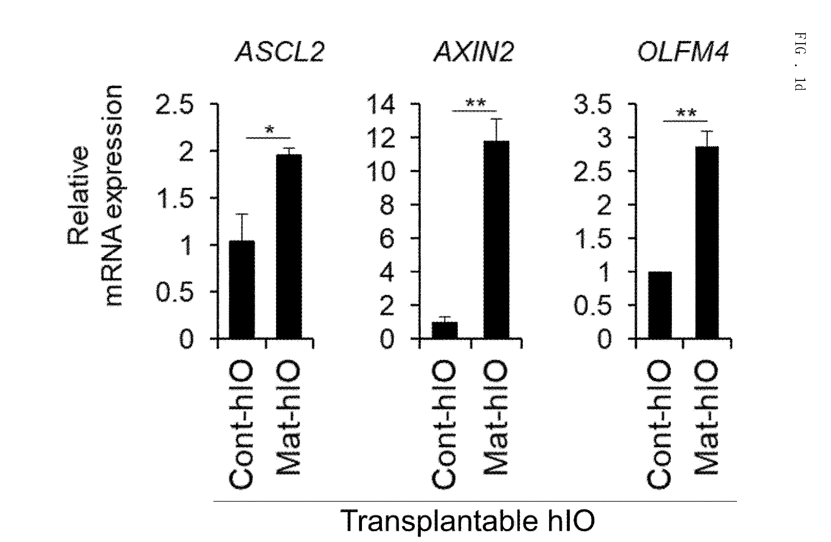

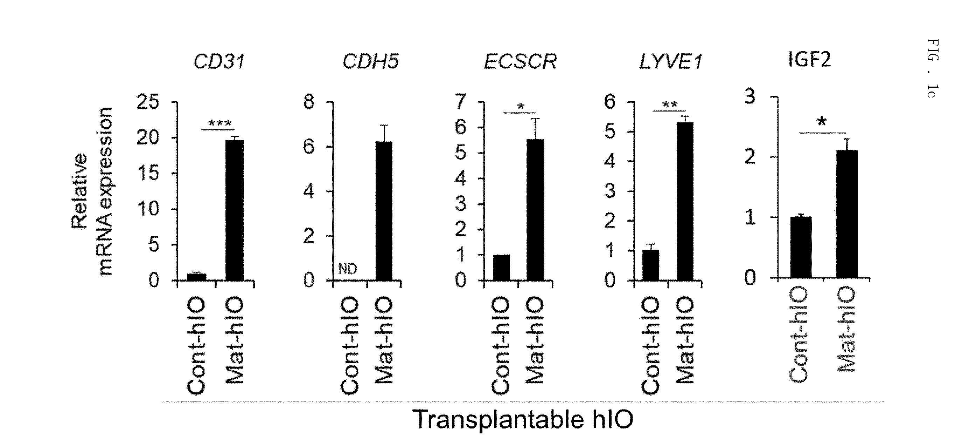

However, upon comparing the expression of intestinal stem cell marker genes (ASCL2, AXIN2, OLFM4) and vascular-specific marker genes (CD31, CDH5, ECSCR, LYVE1, IGF2) in the immature and mature organoids produced for transplantation, it was confirmed that the expression was significantly higher in the mature organoids (FIGS. 1d and 1e).

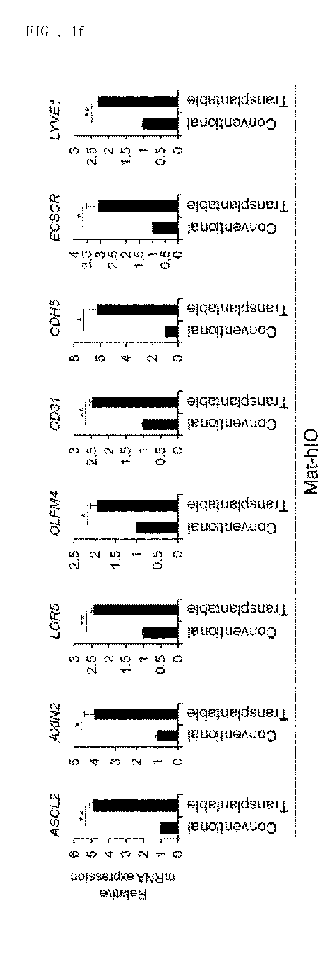

Furthermore, upon comparing the conventional mature intestinal organoids with the transplantable mature intestinal organoids, it was confirmed that the expression of intestinal stem cell-specific markers (ASCL2, AXIN2, LGR5, and OLFM4) and vascular endothelial-related factors (CD31, CDH5, ECSCR, and LYVE1) was highly expressed in the transplantable mature intestinal organoids (FIG. 1f).

These results demonstrated that the transplantable mature intestinal organoids exhibited characteristics different from those of intestinal organoids obtained by conventional methods, particularly, high levels of intestinal stem cell-specific markers, vascular-specific markers, and vascular endothelial-related factors, confirming their potential to be used as organoids suitable for transplantation.

### Example 2. Application of Xeno-free ECM for clinically applicable regenerative therapeutic agent

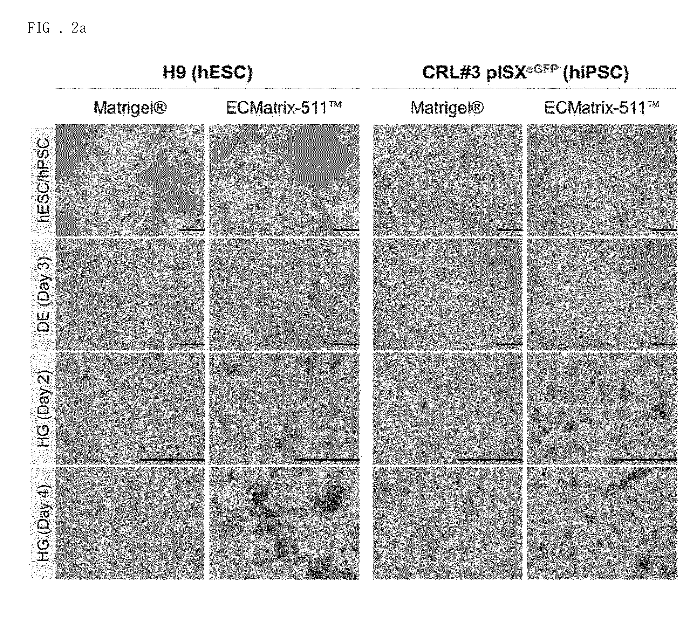

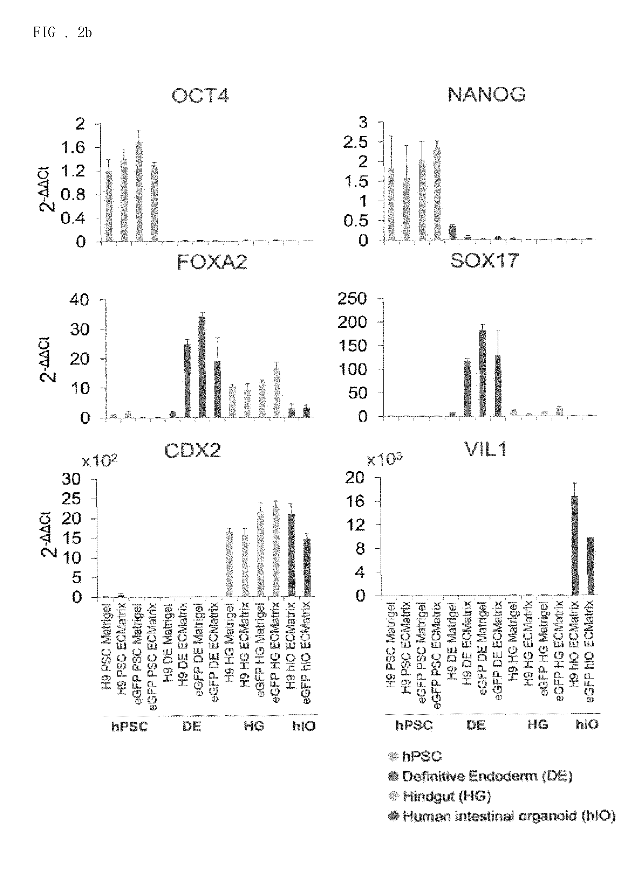

Using the previously presented differentiation protocol, embryonic stem cells and induced pluripotent stem cells were subjected to endoderm (DE) and hindgut (HG) differentiation on dishes coated with Matrigel and ECMatrix^{™}-511. In particular, the hindgut differentiated on ECMatrix^{™}-511 began to form from day 2 and showed a high differentiation rate (HG formation rate) (FIG. 2a). Furthermore, the expression of differentiation markers (OCT4, NANOG, FOXA2, SOX17, CDX2, VIL1) at each differentiation stage confirmed that the differentiation pattern was similar to that on conventional matrigel (FIG. 2b).

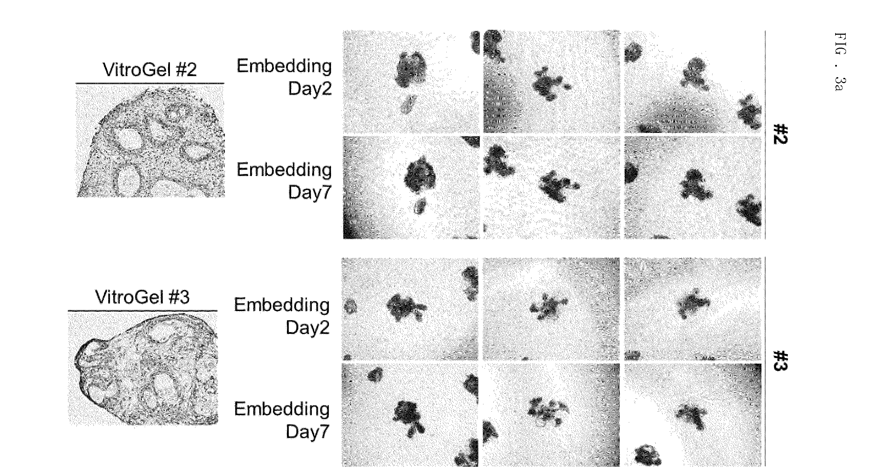

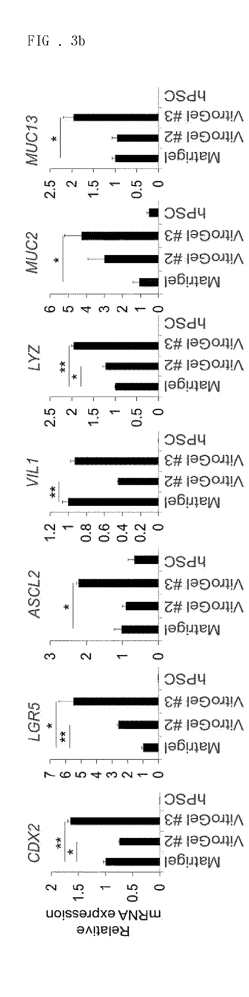

It was confirmed that the structure of the intestinal epithelium was maintained in Vitrogel #2 and #3 types, which were proposed to replace Matrigel for 3D culture, and that Matrigel, VitroGel #2, and VitroGel #3 showed some marker-specific differences in the expression of intestinal-specific markers, but the expression of all markers remained higher than that of undifferentiated pluripotent stem cells, confirming that the differentiation into intestinal organoids using clinically applicable ECMatrix^{™}-511, VitroGel, is the same as that using Matrigel (FIG. 3).

### Example 3. Comparison of the characteristics of mesenchymal stromal cells around immature or high-performance mature hIOs

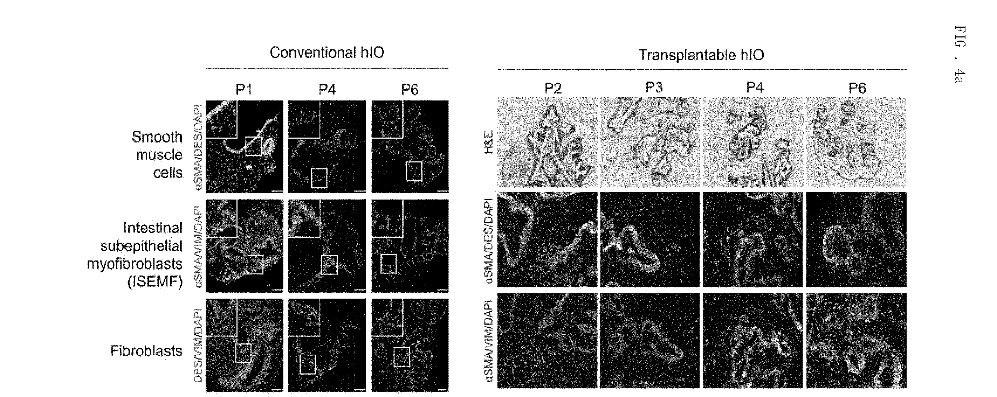

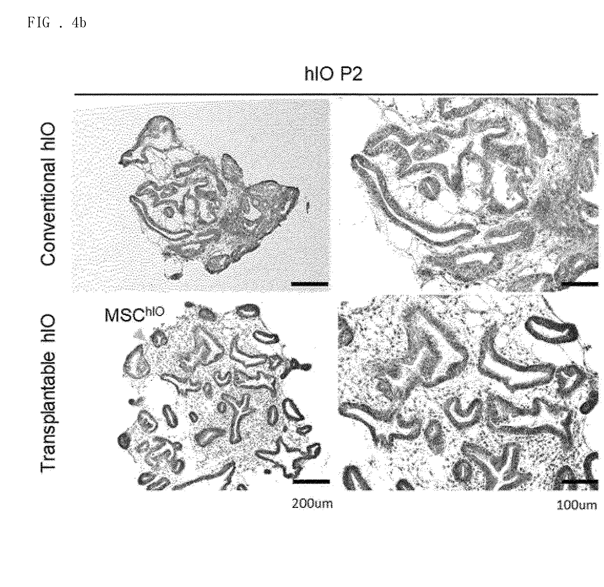

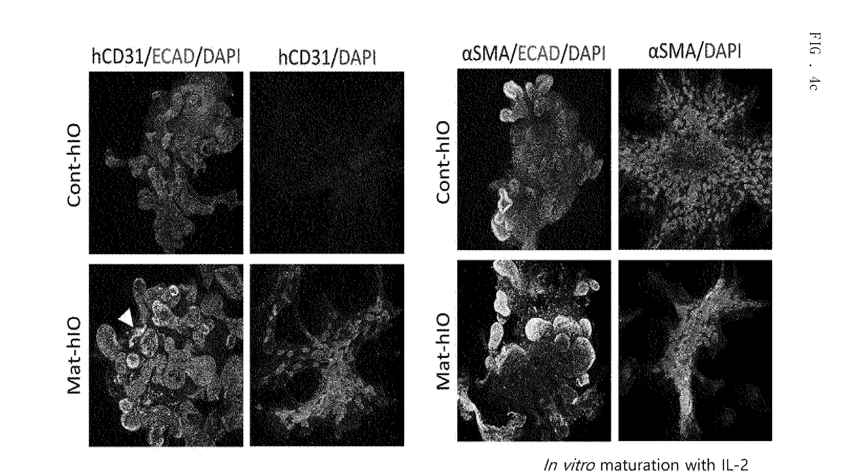

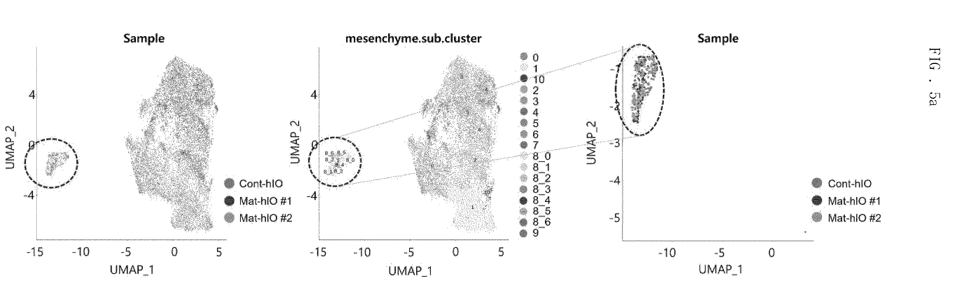

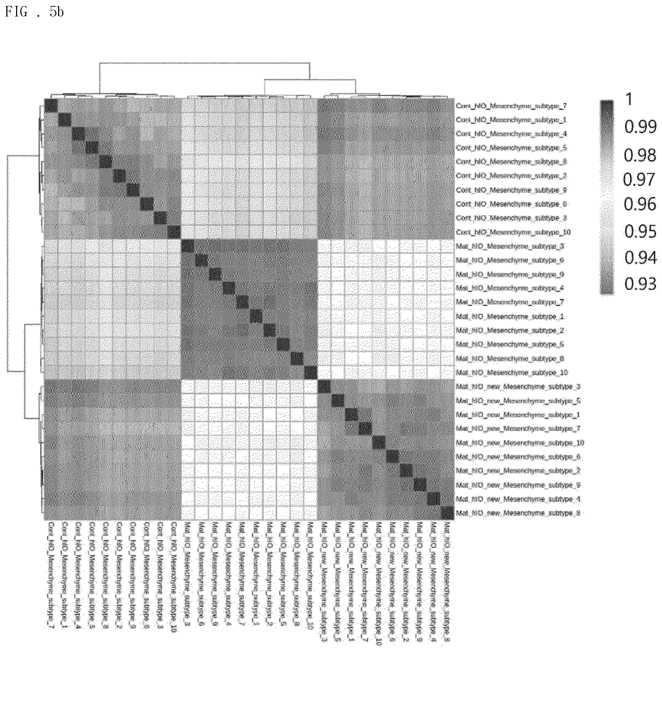

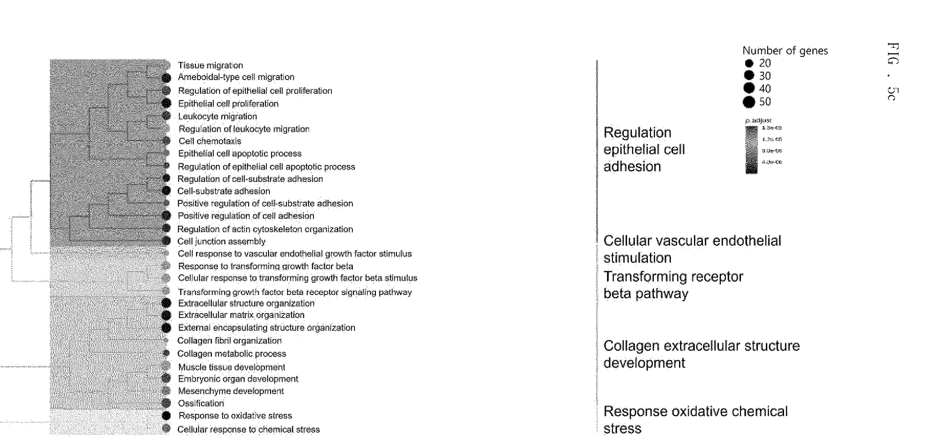

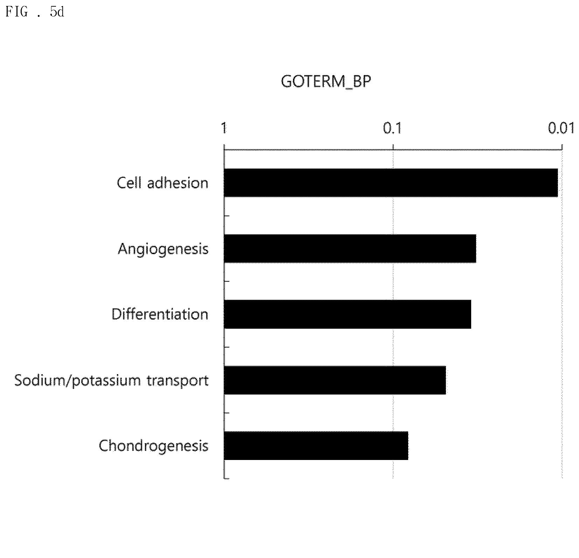

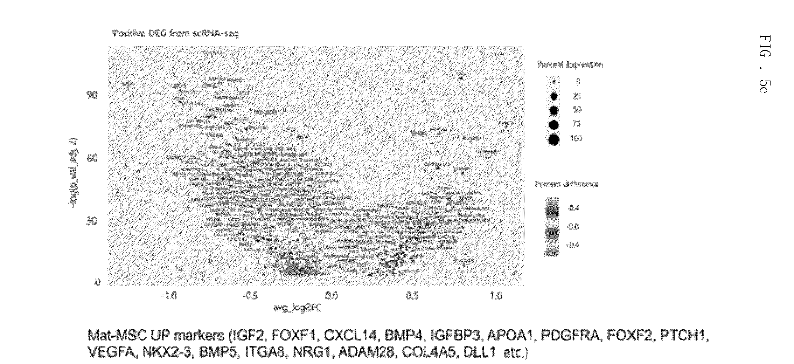

It was confirmed that the survival period of mesenchymal stromal cells around intestinal organoids cultured by conventional intestinal organoid production and culture methods was different from that of intestinal organoids cultured by the proposed transplantable intestinal organoid production technique (FIG. 4a). Compared to conventional culture methods, the transplantation culture technique maintained three well-known mesenchymal stromal cells (smooth muscle cells, intestinal subepithelial myofibroblasts; ISEMF, and Fibroblasts) up to P6, as shown by H&E and immunofluorescence staining (αSMA+, DES+, ViM+). In addition, H&E staining showed a greater distribution of mesenchymal stromal cells (MSC^{hIO}) around epithelium cells in the transplantable organoids compared to conventional organoids (FIG. 4b). Staining of whole organoids and immunofluorescence staining of mesenchymal cells isolated from organoids revealed the presence of vascular cells (hCD31+) in the mesenchymal cells (FIG. 4c). For a closer analysis at the gene level, single cell RNAseq was performed to analyze one kind of Cont-hIO and two kinds of Mat-hIO, identifying that the Mesenchyme cluster was composed of a total of seven groups (8_0, 8_1, 8_2, 8_3, 8_4, 8_5, and 8_6), where the remaining groups except for 8_0 and 8_6 confirmed the mesenchymal cell characteristics of Mat-hIO (FIG. 5a). Differentially Expressed Genes (DEGs) were selected from the Mesenchyme group of Mat-hIO compared to Cont-hIO, and the up-regulated Gene Ontology biological process (GOBP) included terms related to Epithelial cell adhesion or cellular vascular endothelial stimulation (FIG. 5c). In addition, GOTERM_BP analysis of DEGs in DAVID (https://david.ncifcrf.gov/summary.jsp) also confirmed that cell adhesion, angiogenesis, differentiation, etc., were increased in Mat-hIO compared to Cont-hIO (FIG. 5d). In addition, the volcano plot of DEGs in the mesenchymal stromal cell cluster of Mat-hIO showed that the up-regulated genes in the mesenchymal stromal cells of Mat-hIO included IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, as representative examples (FIG. 5e). The detailed gene list is shown in Table 7.

**[Table 7]**

| | **p_val** | **avg_log2FC** |
|---|---|---|
| **IGF2.1** | 9.66E-28 | 1.059351744 |
| **FOXF1** | 1.50E-25 | 0.839701803 |
| **CXCL14** | 7.19E-08 | 0.798390905 |
| **BMP4** | 4.03E-17 | 0.781237745 |
| **IGFBP3** | 9.76E-11 | 0.659753907 |
| **APOA1** | 1.09E-26 | 0.642983424 |
| **PDGFRA** | 6.20E-17 | 0.642415099 |
| **FOXF2** | 1.60E-14 | 0.56758628 |
| **PTCH1** | 1.54E-12 | 0.551034596 |
| **VEGFA** | 1.33E-10 | 0.491855898 |
| **NKX2-3** | 1.03E-13 | 0.487365795 |
| **BMP5** | 9.30E-09 | 0.473039399 |
| **COL9A1** | 2.67E-08 | 0.432872887 |
| **ITGA8** | 1.39E-06 | 0.419933776 |
| **NRG1** | 6.00E-08 | 0.415831905 |
| **ADAM28** | 1.17E-08 | 0.414366444 |
| **COL4A5** | 2.14E-06 | 0.397617201 |
| **DLL1** | 8.21E-08 | 0.381424539 |

### Example 4. Comparison of post-transplantation characteristics between immature and high-performance mature hIOs via subrenal transplantation

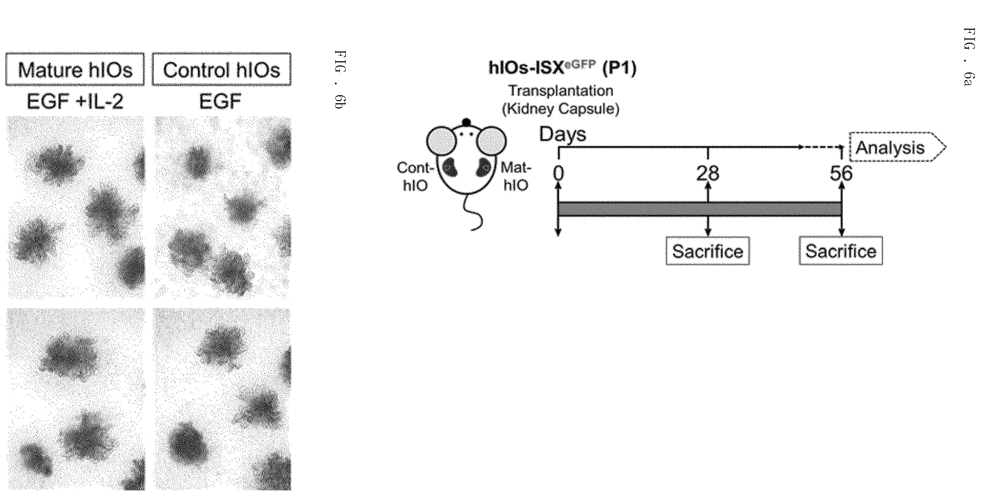

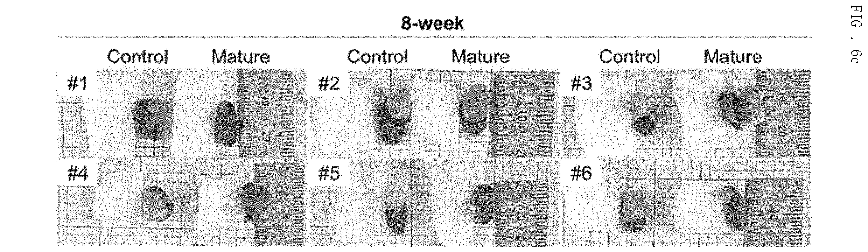

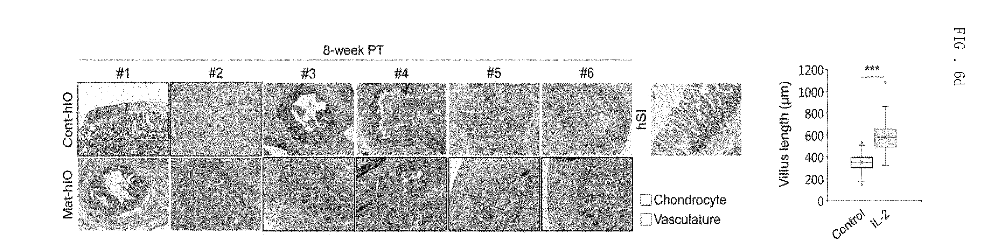

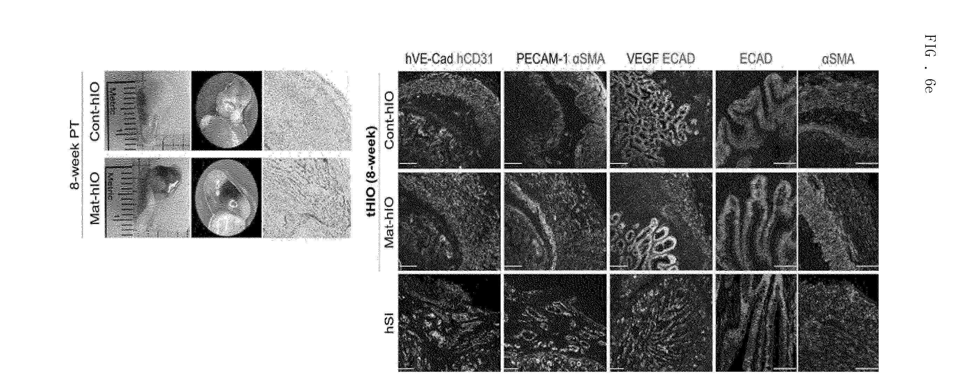

To minimize differences between mice after transplantation, Cont-hIO and Mat-hIO were transplanted into both kidneys of the same immunodeficient mouse and observed after 4 and 8 weeks, respectively (FIGS. 6a and 6b). After 8 weeks, the xenografts from both kidneys were excised and observed, and prominent vascularization was confirmed around the mature organoid xenografts compared to the immature organoids (FIG. 6c). This was supported by histological analysis using H&E, which showed that the blood vessels formed around the transplanted intestinal organoids and the villus length of the intestinal organoid structure developed similarly to that of adult human small intestine tissue (FIG. 6d). The expression of vascular markers (hVE-Cad, PECAM-1, and VEGF) using immunofluorescent staining confirmed the vascularization and structure around the xenograft organoid tissue and validated the development of the villus structure (hECAD) and mesenchymal stromal cells (αSMA) of the intestinal organoids in vivo (FIG. 6e).

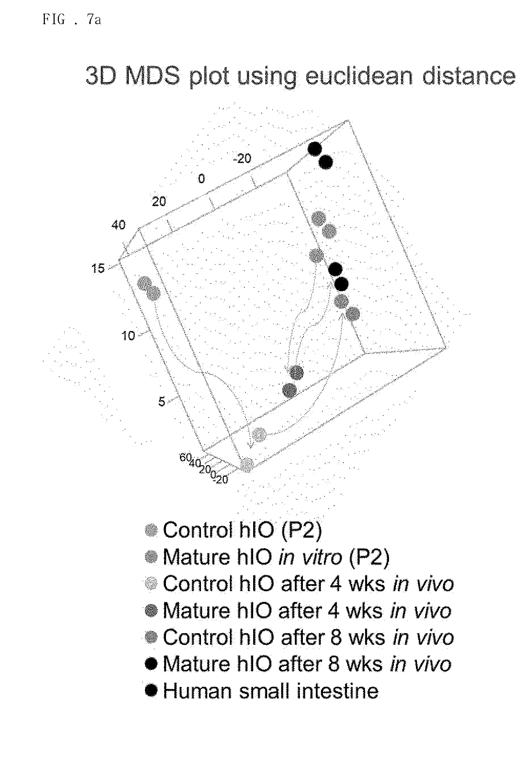

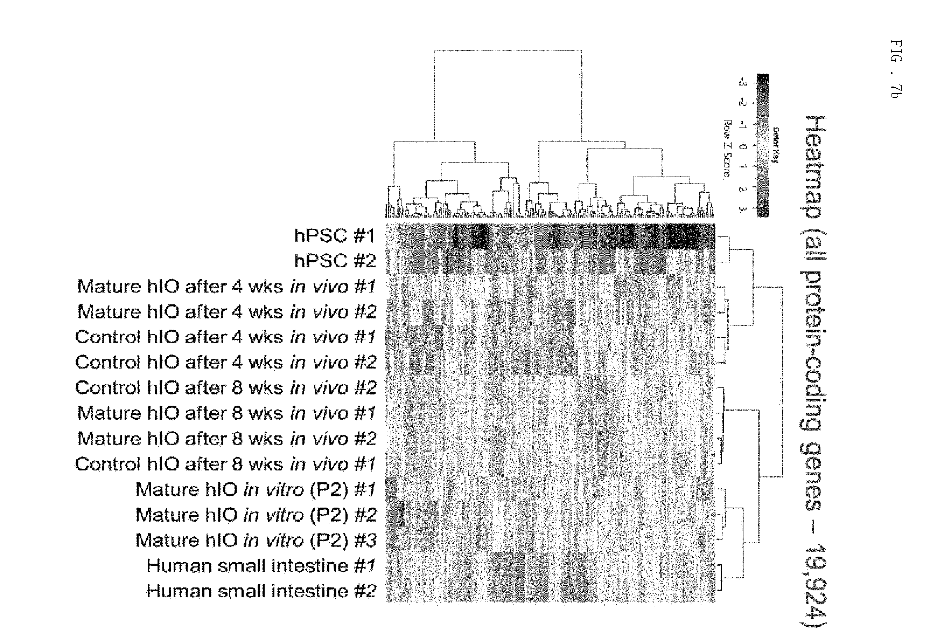

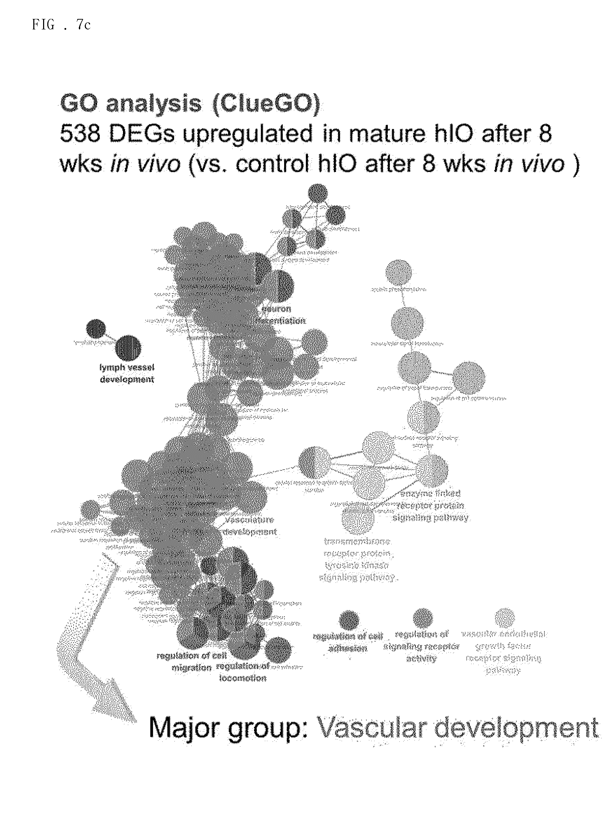

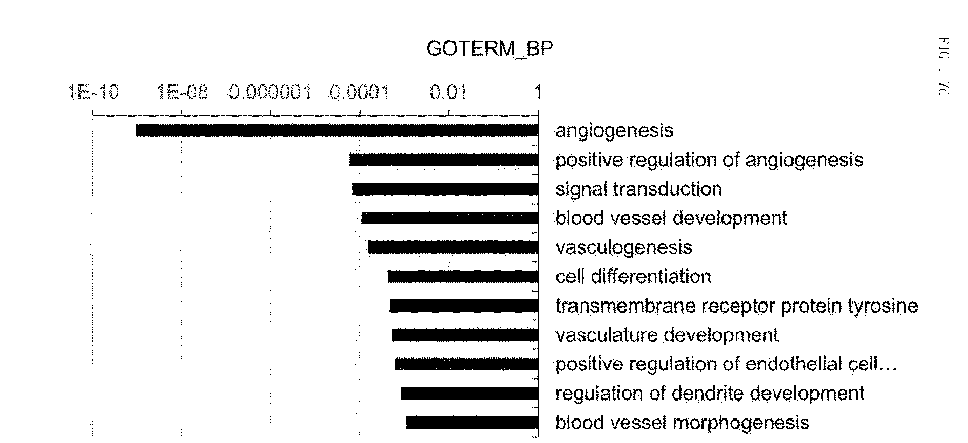

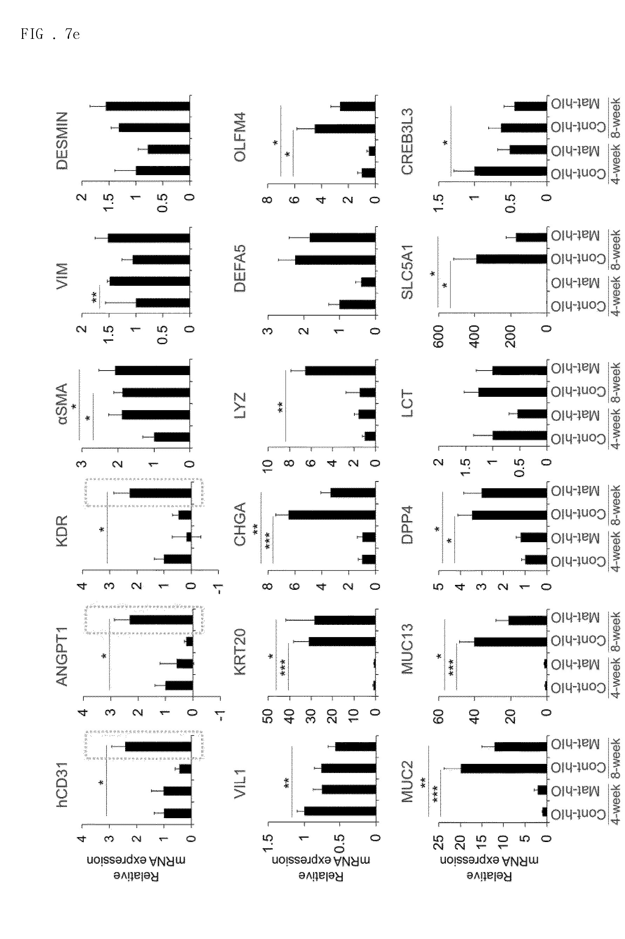

Genomic analysis via RNAseq confirmed the previously known maturation pathway of intestinal organoids upon subrenal transplantation, by comparing immature intestinal organoids, 4- and 8-week xenograft intestinal organoids, in vitro mature intestinal organoids, and human small intestinal tissue (FIG. 7a). In particular, GO analysis of 538 DEGs that were different between the Cont-hIO transplantation group and the Mat-hIO transplantation group 8 weeks after transplantation identified that vascular development was the major group (FIGS. 7b, 7c, and 7d). To validate the RNAseq genomic analysis results, qPCR confirmed that vascular markers (hCD31, ANGPT1, and KDR), representative markers of mesenchymal stromal cells (αSMA, VIM, and DESMIN) and most of the small intestine-specific markers were increased in the 8-week xenograft Mat-hIO compared to Cont-hIO (FIG. 7e).

### Example 5. Comparison of the intestinal transplantation engraftment ability of immature or high-performance mature hIOs

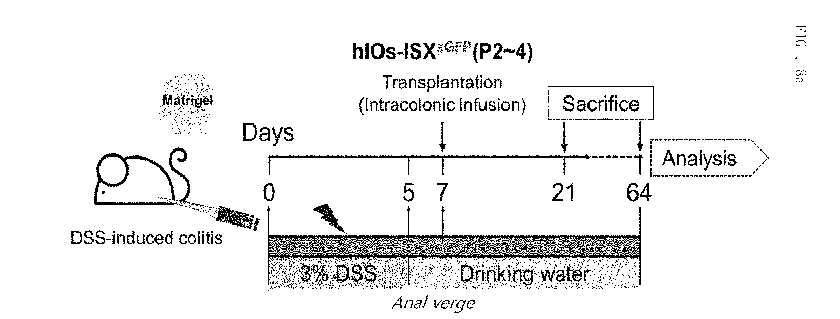

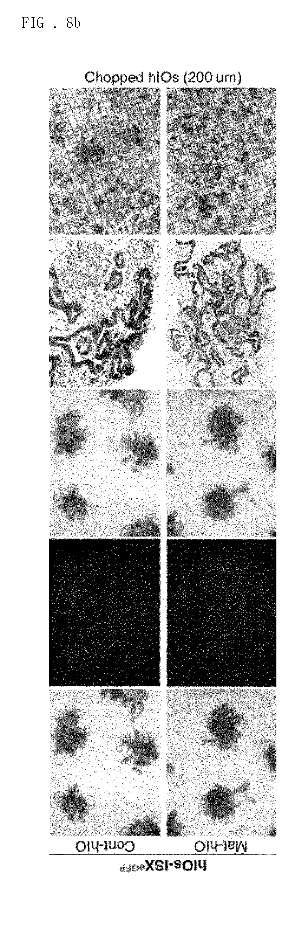

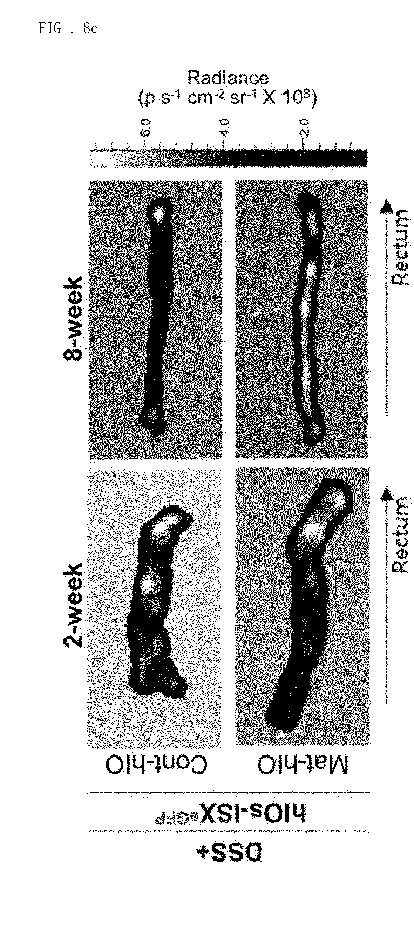

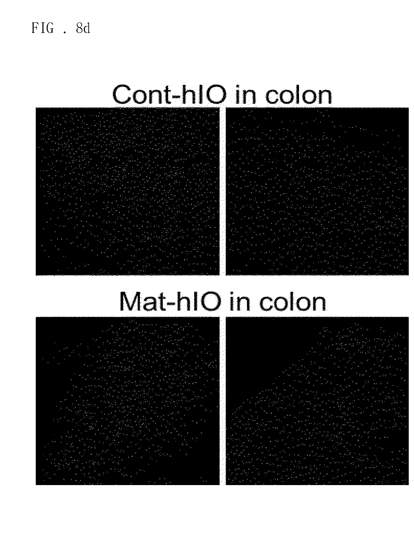

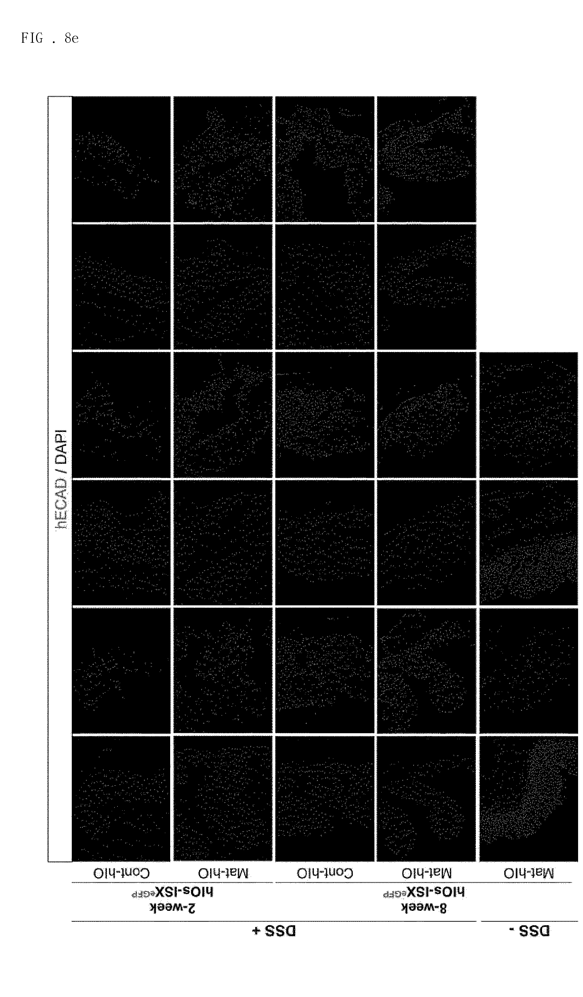

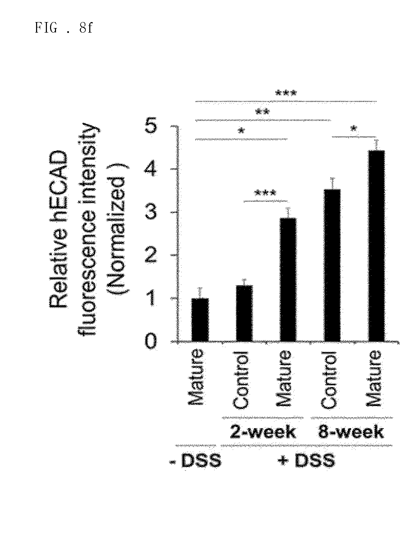

To verify the transplantation and engraftment ability of high-performance mature intestinal organoids (Mat-hIO), DSS-induced inflammatory bowel disease immunodeficient mice were transplanted with induced pluripotent stem cell-derived Cont-hIOs or Mat-hIOs, and observed after 2 or 8 weeks (FIG. 8a). After implantation of reporter intestinal organoids expressing GFP fluorescence, the location and distribution of the reporter intestinal organoids could be tracked via IVIS images after 2 or 8 weeks (FIGS. 8b, and 8c). After 8 weeks, fluorescence was detected throughout the intestine in the Mat-hIO-implanted colon, and GFP fluorescence identification of excised colon sections showed a greater distribution of Mat-hIOs compared to the Cont-hIO-implanted group (FIG. 8d). The colons of Matrigel, Cont-hIO, or Mat-hIO transplantation groups were sectioned and stained with human-specific antibodies (hECAD), and consequently, the expression of reporter Mat-hIO cells was significantly expressed in the transplanted intestinal tissues (FIGS. 8e and 8f). This means that the transplantable Mat-hIO has excellent engraftment ability through follow-up observation for 2 and 8 weeks after in vivo transplantation.

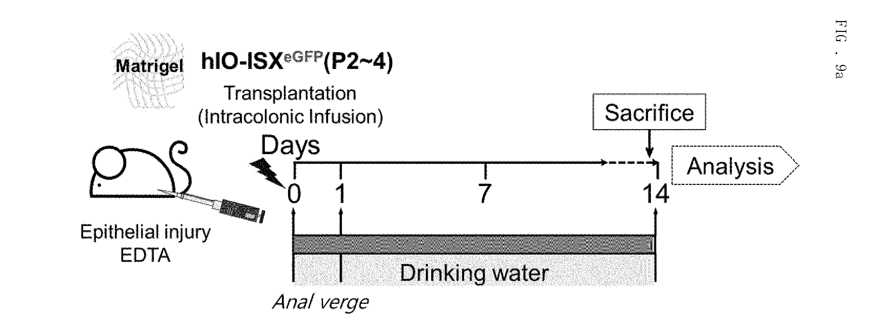

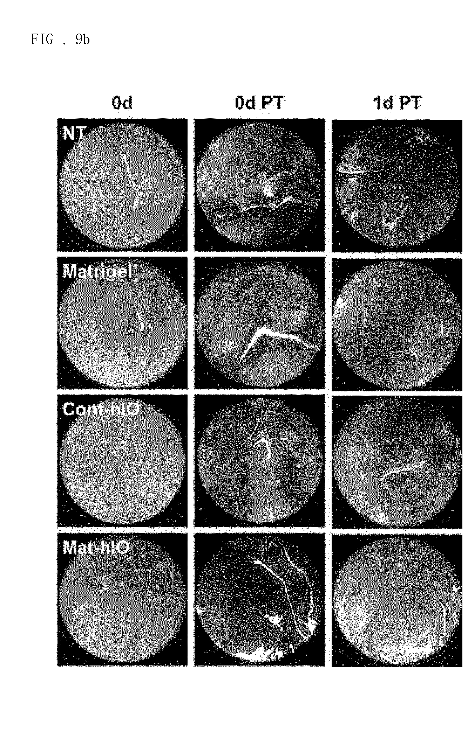

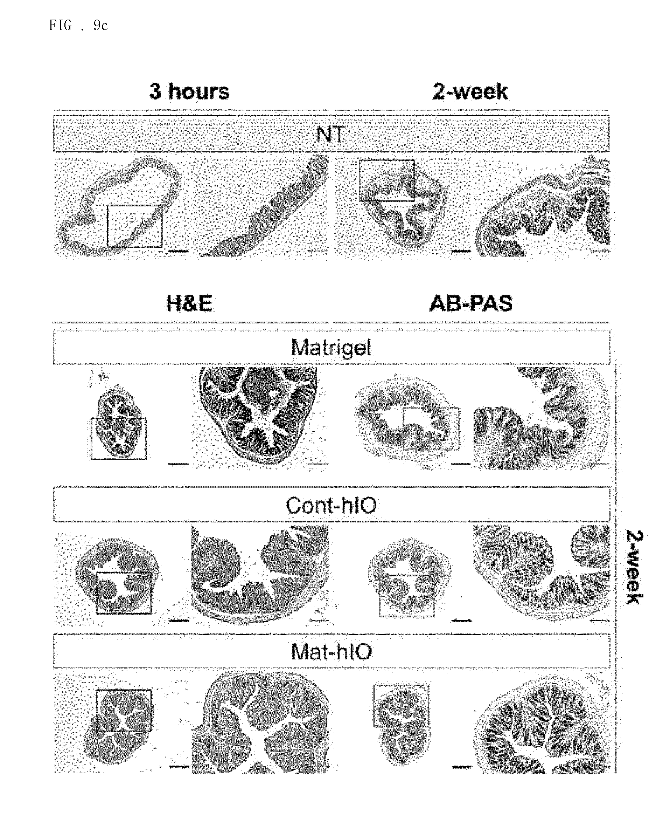

In addition, in order to verify the initial engraftment once more, transplantation was performed with immunodeficient mice with EDTA-induced intestinal epithelial injury, which allows for directly injury to the rectum in the anal region (FIG. 9a). Colonoscopy on day 1 after transplantation of no treatment (NT), Matrigel, Cont-hIO, or Mat-hIO showed that wounds and mucosal bleeding were alleviated in the Mat-hIO transplantation group (FIG. 9b). Histological and functional analyses showed that the Mat-hIO transplantation group showed restoration of the intestinal epithelium structure and enhanced Mucin functionality compared to the no treatment, Matrigel, and Cont-hIO transplantation groups 2 weeks after xenografting (FIG. 9c).

### Example 6. Transplantation efficacy of Matrigel alternative biomaterial (Fibrin) for clinical applications

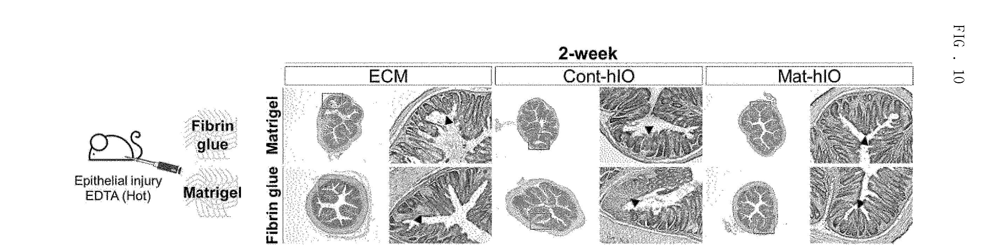

In addition to Matrigel, which is widely used in mouse transplantation, a clinically applicable biomaterial (Fibrin) was applied to transplant ECM alone and immature or mature intestinal organoids derived from induced pluripotent stem cells into immunodeficient mice with EDTA-induced intestinal epithelial injury (FIG. 10). Histological examination of the colon transplant site 2 weeks after transplantation showed little difference in transplantation efficacy between Matrigel and Fibrin when transplanted alone or together with high-performance mature intestinal organoids.

### Example 7. Optimization of the size of regenerative therapeutic intestinal organoids for improved transplantation efficacy

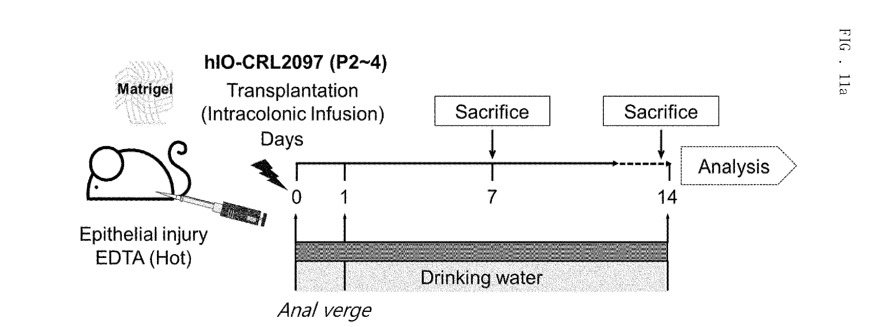

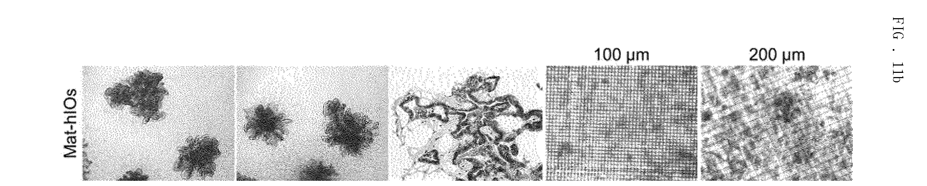

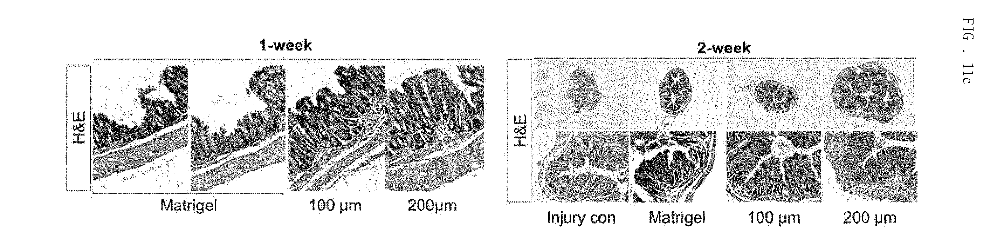

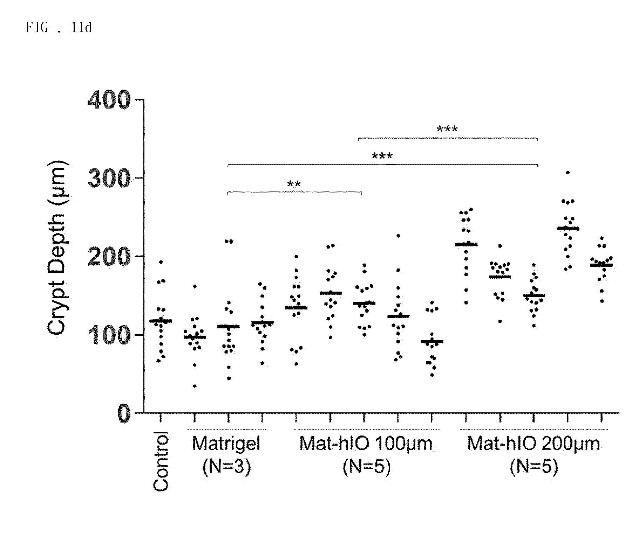

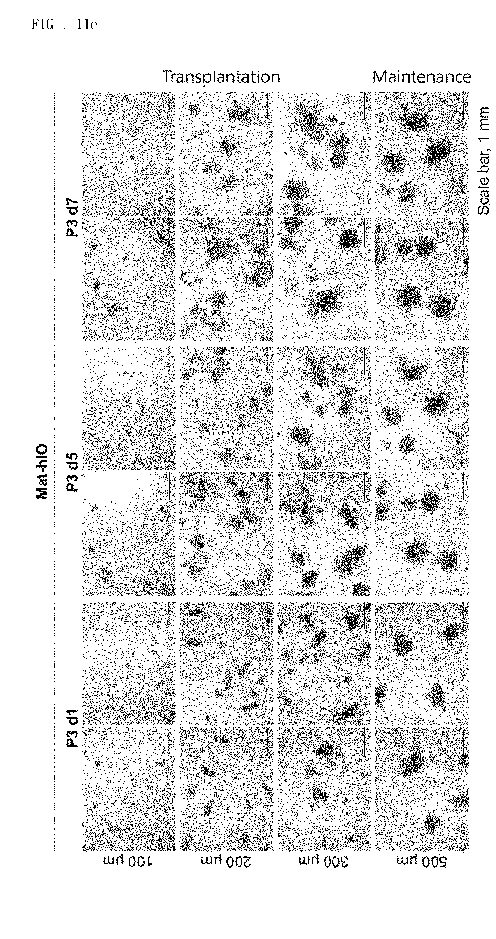

The size of the organoids was determined by measuring the size that did not interfere with their growth during in vitro subculture. When transplanted, oversized cells may not engraft into the intestinal wound, and thus the whole organoid cannot be transplanted as it is. Therefore, since high-performance mature intestinal organoids of appropriate size for transplantation should help rapid engraftment and regeneration including mesenchymal stromal cells, sizes of 100 um, 200 um, 300 um, and 500 um were tested respectively (FIG. 11e). The size of the 500 um organoid was established as the size for passage culture because it maintains homogeneity in size and quickly recovers mesenchymal stromal cells and crypt-villus structures. In addition, it was confirmed that in vitro culture was possible without growth inhibition at the size of 200 um, and the transplantation ability and crypt depth were verified after transplantation of 100 or 200 um into the immunodeficient mice with EDTA-induced intestinal epithelial injury (FIG. 11). Two weeks after transplantation, the 200 um Mat-hIO transplantation group showed the recovery of the transplant site in both longitudinal and transverse sections (FIG. 11c). Human intestinal organoids are known to have a higher crypt depth compared to mouse intestinal organoids, resulting in high crypt depth at the transplantation site upon successful transplantation. Thus, the crypt depth was measured and showed a significant increase in the 200 um Mat-hIO xenografts (FIG. 11d).

### Example 8. Engraftment and regeneration efficacy of high-performance mature intestinal organoids derived from induced pluripotent stem cells

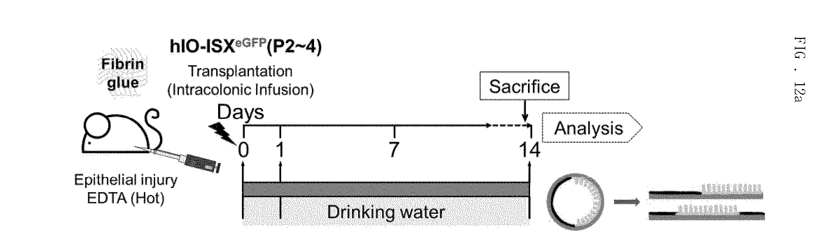

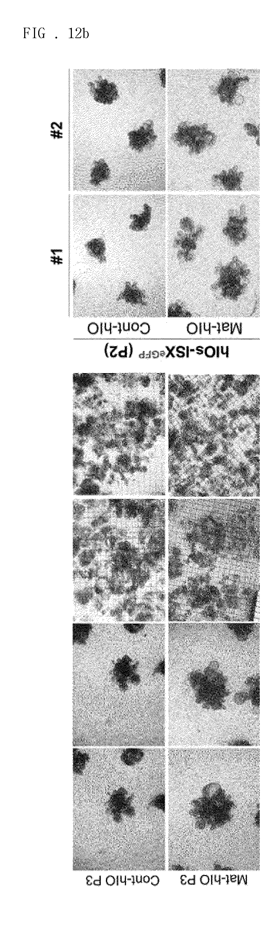

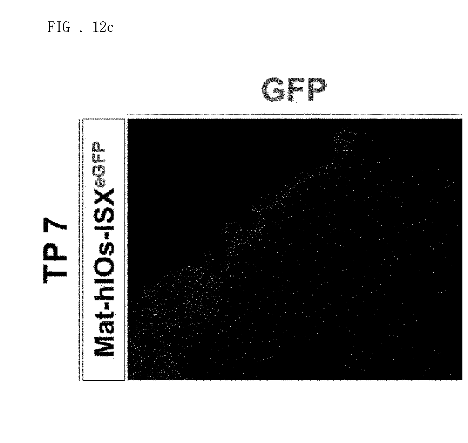

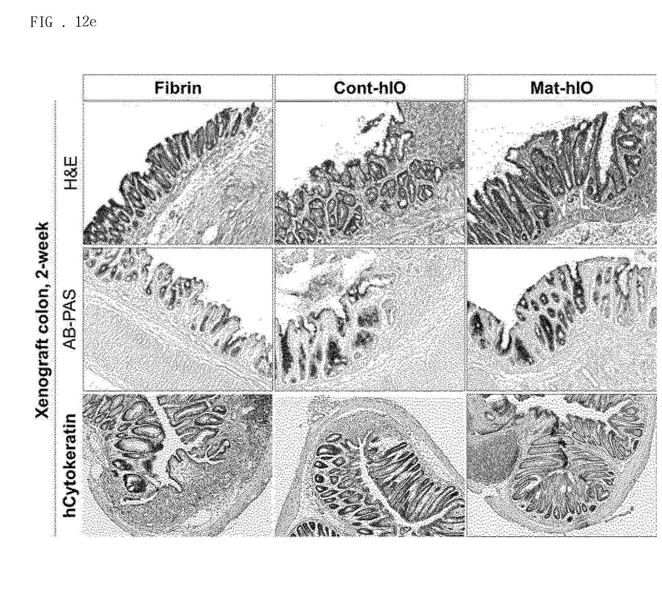

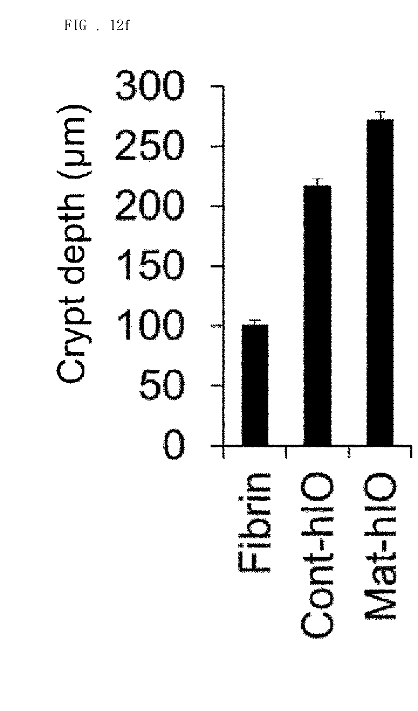

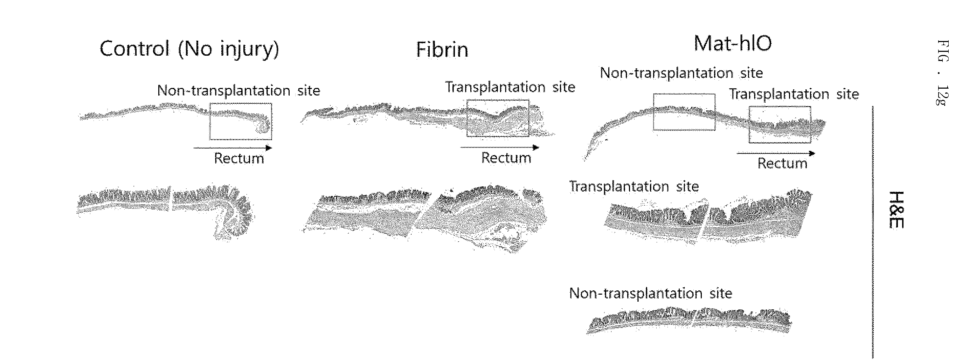

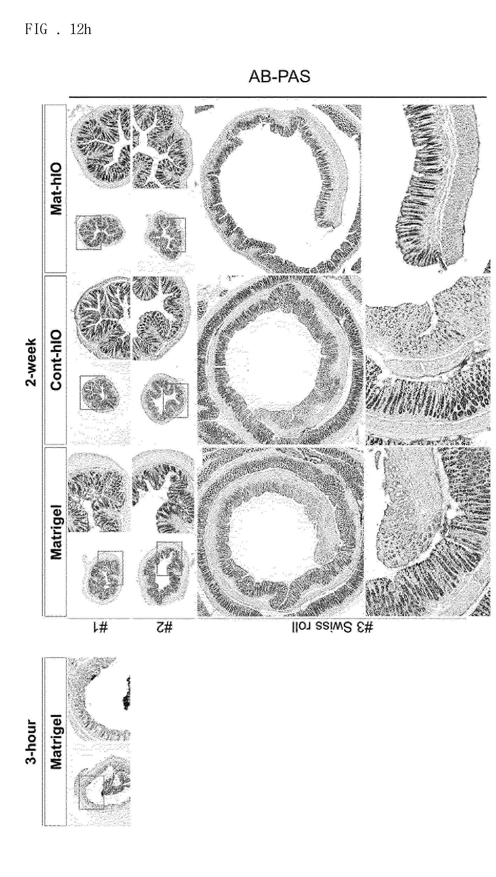

To verify the engraftment and regeneration efficacy of reporter high-performance mature intestinal organoids derived from induced pluripotent stem cells in mice, immunodeficient mice with EDTA-induced intestinal epithelial injury were prepared. In addition, based on the previous results, mature intestinal organoids of 200 µm in size were prepared for transplantation, and fibrin was adopted instead of Matrigel as the ECM (FIG. 12a). To directly verify engraftment, the structure and GFP fluorescence expression of the reporter organoids were confirmed, and transplantation was performed (FIG. 12b). The normal intestinal structure and environment were confirmed before transplantation through a colonoscopy for experimental animals, the degree of epithelial injury was measured again immediately after the epithelial injury, and transplantation was performed. At this time, it was observed through tissue fluorescence confirmation 1 week after transplantation that the transplanted Mat-hIO was located on the surface of the intestinal epithelium (FIG. 12c). In addition, colonoscopy 2 weeks after transplantation showed that colonic epithelial tissue was developed without abnormal tissue expression in the Mat-hIO transplantation group. Furthermore, quantitative evaluation of colonoscopy confirmed a significant reduction in epithelial injury in the Mat-hIO group. Fluorescence dissection microscopy verified that GFP expression of the transplanted reporter intestinal organoids was high in Mat-hIO (FIG. 12d). Histological staining of the colon area of the xenograft showed that the Mat-hIO transplantation group had significantly higher crypt depth and formation (restoration) rate than the groups transplanted with Matrigel or Cont-hIO, and in particular, the secretion of Mucin was functionally active (FIGS. 12e and 12f). In addition, histological staining of longitudinal sections was performed to confirm the overall pattern in the colon of the transplanted mouse, and it was re-verified that the transplantation and reconstitution were performed in Mat-hIO xenografts compared to the control and Fibrin transplantation groups (FIG. 12g). Further, AB-PAS staining of Swiss-roll xenografts showed that the structure of the epithelium in the rectum area was restored and that mucin secretion was active in Mat-hIO (FIG. 12h).

### Example 9. Engraftment and regeneration efficacy of high-performance mature intestinal organoids derived from embryonic stem cells differentiated from xeno-free ECM

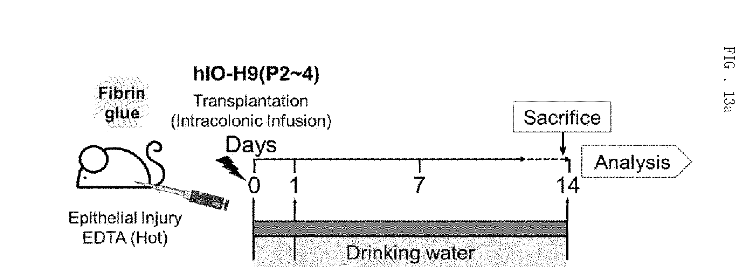

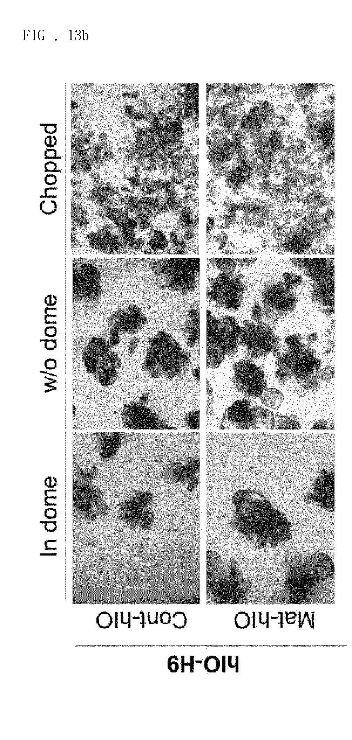

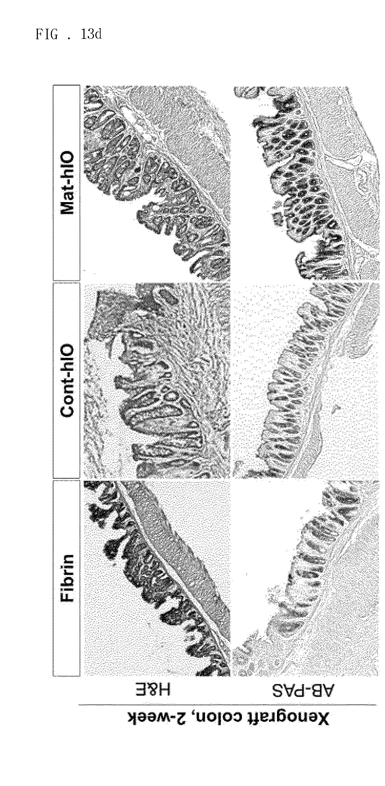

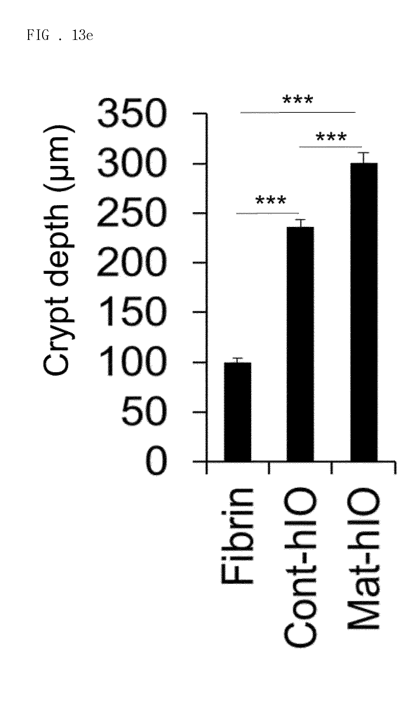

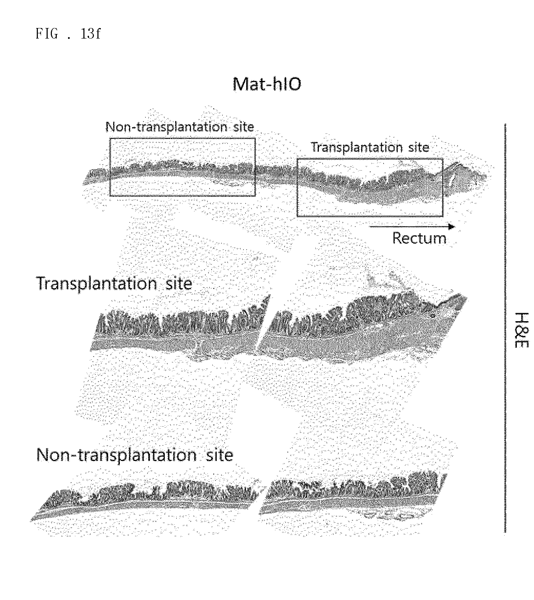

Embryonic stem cell-derived intestinal organoids differentiated from xeno-free ECM (ECMatrixTM-511) and cultured in VitroGel for 1 passage before transplantation were transplanted into immunodeficient mice with EDTA-induced intestinal epithelial injury to verify engraftment and regeneration efficacy (FIGS. 13a and 13b). The colonic environment of each group was observed before transplantation, immediately after epithelial injury, and 2 weeks after transplantation via a colonoscopy for experimental animals, and the condition of the excised intestine was confirmed via a fluorescence dissecting microscope. As a result, it was confirmed that the colon transplanted with high-performance mature intestinal organoids (Mat-hIO) showed the fastest recovery, which is consistent with the transplantation results of reporter intestinal organoids derived from induced pluripotent stem cells. In addition, 2 weeks after transplantation, colonoscopic evaluation also confirmed that the epithelial injury was significantly reduced in the Mat-hIO transplantation group (FIG. 13c). Furthermore, histologic staining demonstrated restored colon tissue structure, crypt depth, and improved mucin secretion (FIGS. 13d and 13e). Analysis of the entire intestinal structure also confirmed that the Mat-hIO transplanted site had a higher crypt depth than the non-transplanted site (FIG. 13f).

### Example 10. Transplantation efficacy of induced pluripotent stem cell-derived high-performance mature intestinal organoids in inflammatory bowel disease model

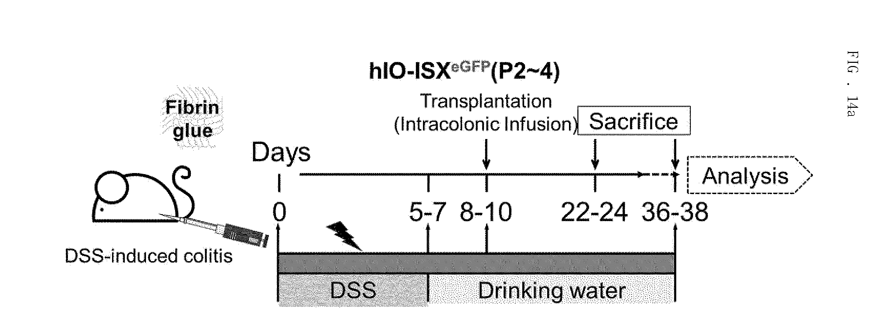

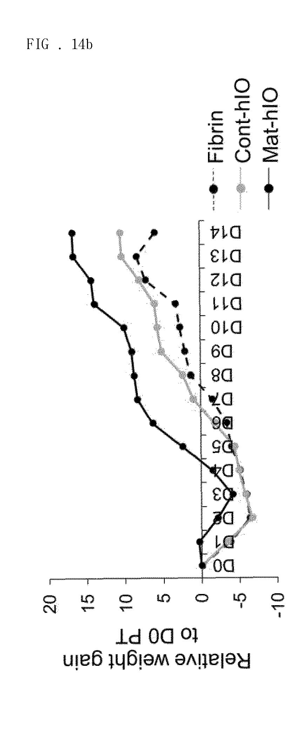

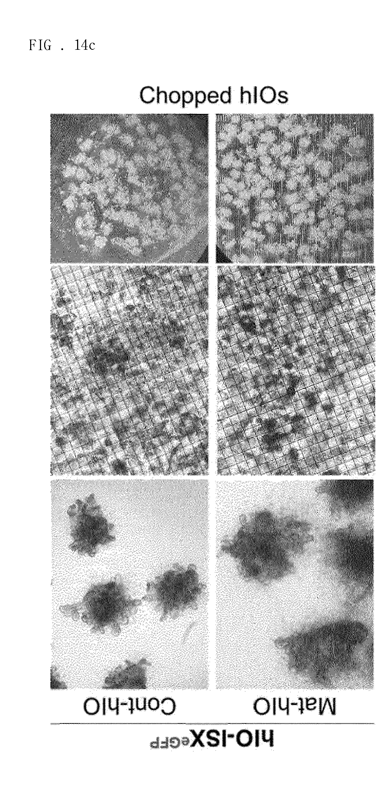

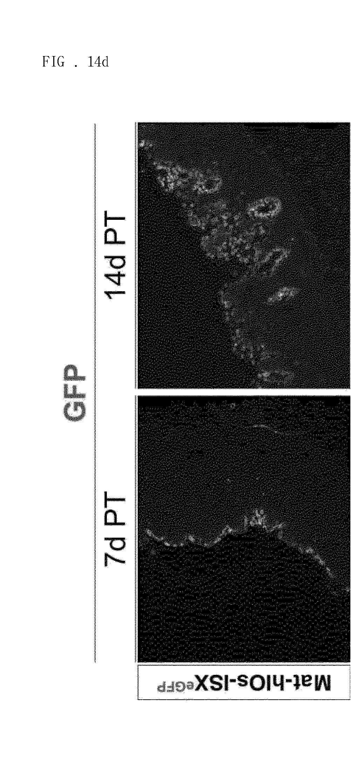

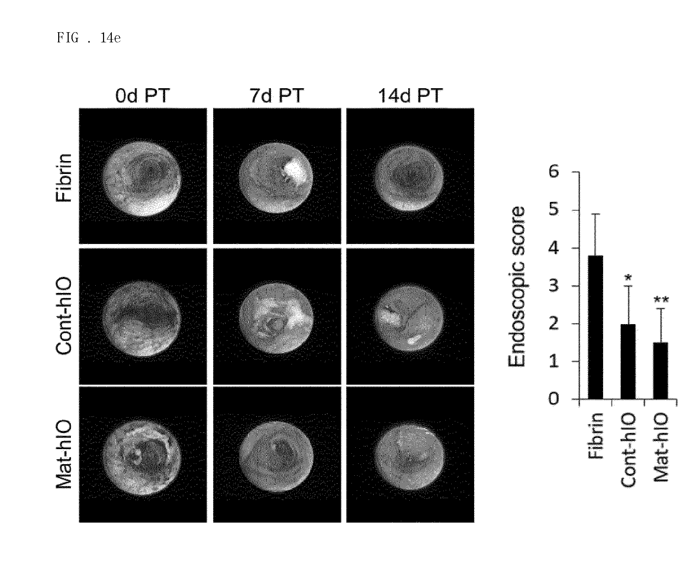

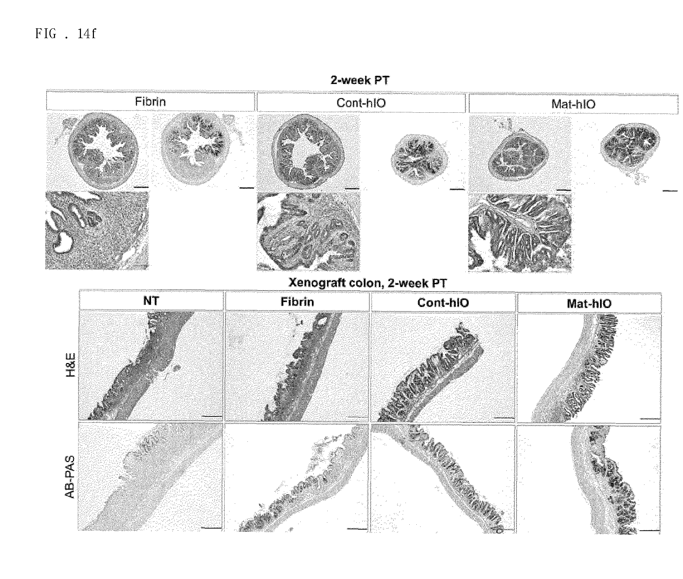

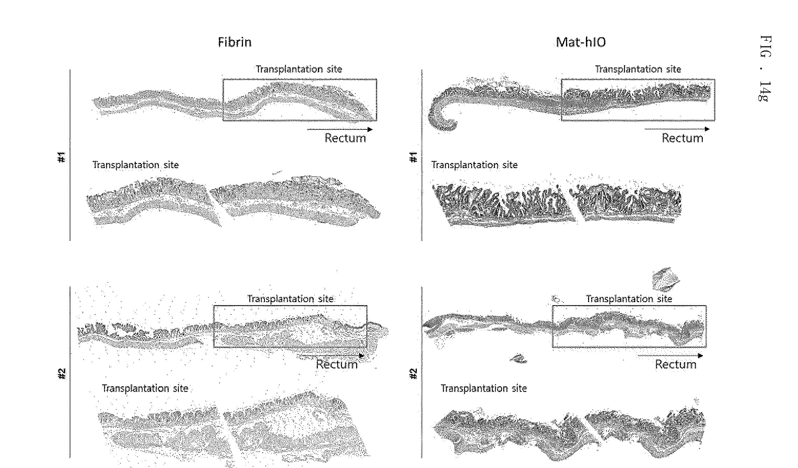

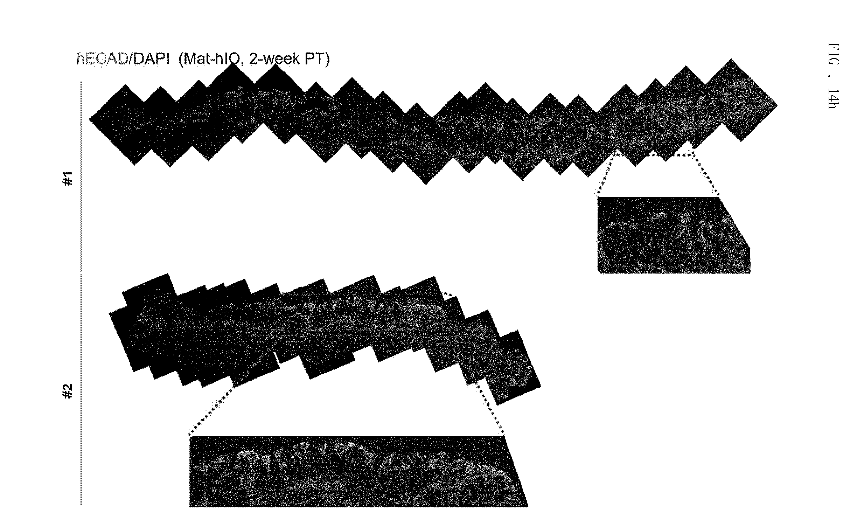

In order to confirm not only the engraftment efficacy of Mat-hIO in intestinal epithelial injury but also the therapeutic efficacy in an actual inflammatory bowel disease model, immunodeficient mice with DSS-induced inflammatory bowel disease were produced and tested (FIGS. 14a and 14c). The relative weight on the day of transplantation was confirmed to be significantly increased in the Mat-hIO transplantation group (FIG. 14b). When the fluorescence of the xenograft colon tissue was observed 1 week after transplantation using reporter intestinal organoids to track transplanted cells, it was confirmed that GFP+ cells were located on the epithelial surface at 1 week, and then high-performance mature intestinal organoids (Mat-hIO) were distributed within the colonic epithelium at 2 weeks (FIG. 14d). Furthermore, colonoscopy showed that Mat-hIO transplantation group had alleviated intestinal inflammation and decreased intestinal mucosal bleeding (FIG. 14e). Quantitative evaluation of these observations was also performed and re-verified that the degree of injury was significantly reduced in the Mat-hIO transplantation group, similar to the results above. Histological analysis of both longitudinal and transverse sectioned tissues revealed that the hIO transplantation group showed better recovery than the no treatment group or the Matrigel-treated group, and the Mat-hIO transplantation group showed a more prominent regeneration effect than the Cont-hIO transplantation group (FIG. 14f). Furthermore, in DSS-induced inflammatory bowel disease, where inflammation occurs throughout the colon, H&E staining confirmed that the Mat-hIO transplantation group exhibited excellent epithelial recovery when transplantation was performed using a catheter at a depth of 3 cm (FIG. 14g). To confirm the presence of human high-performance mature intestinal organoids, immunofluorescence staining with the human-specific antibody (hECAD) was conducted, demonstrating that more cells were present in the rectum area (FIG. 14h).

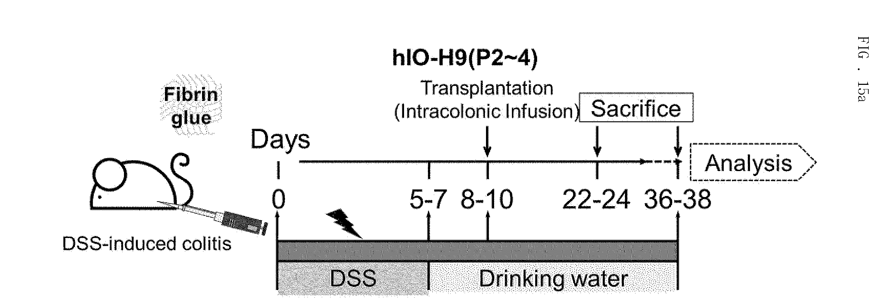

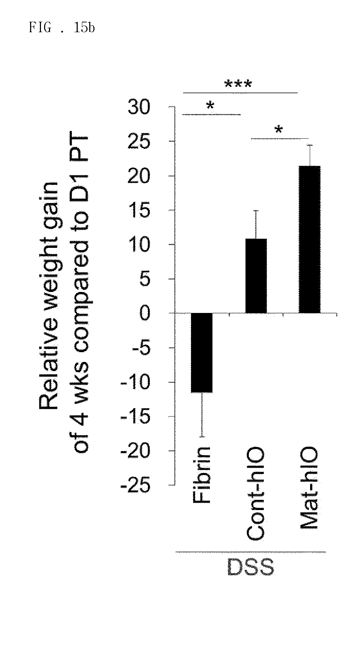

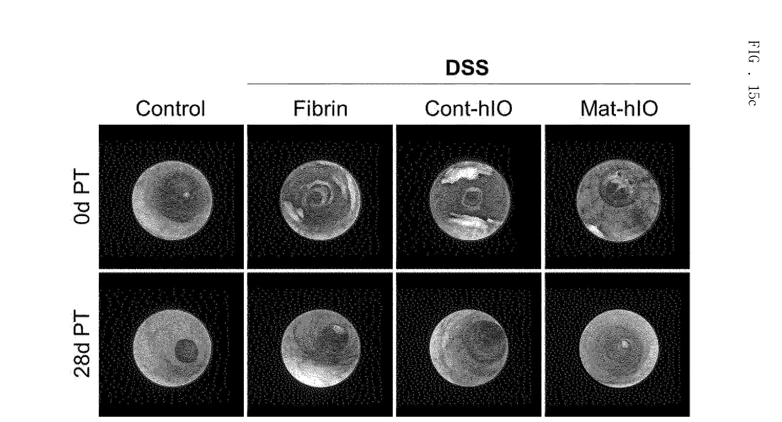

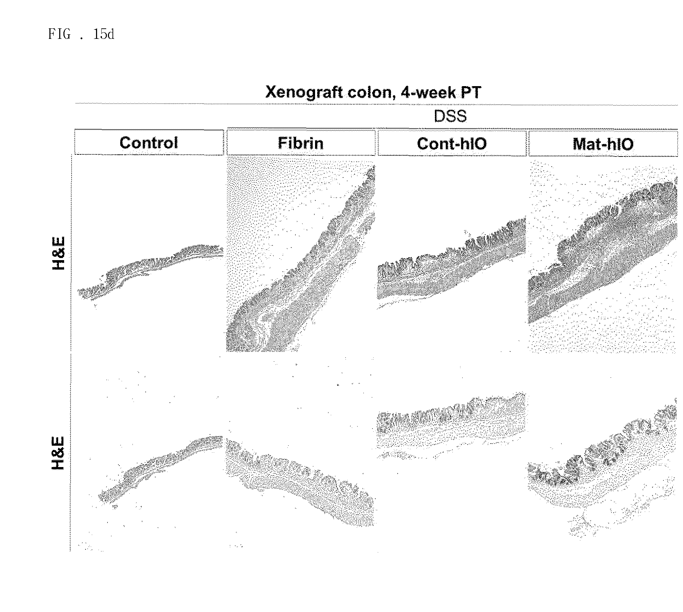

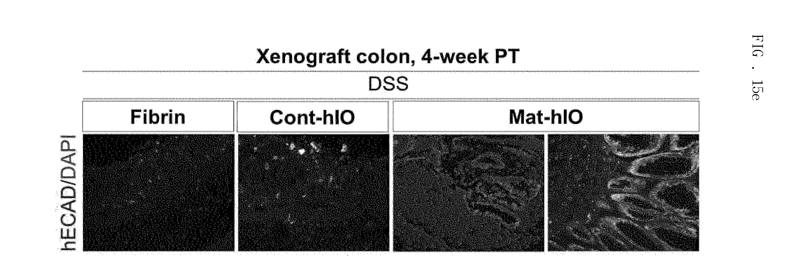

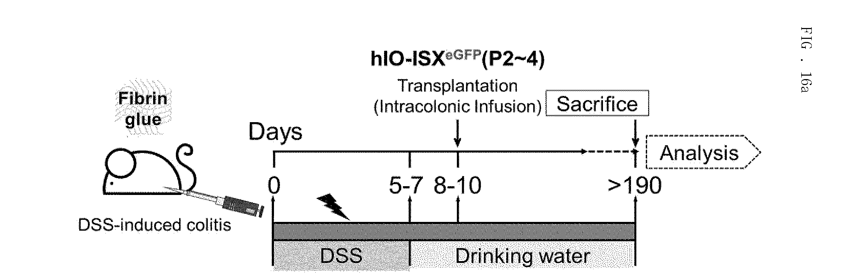

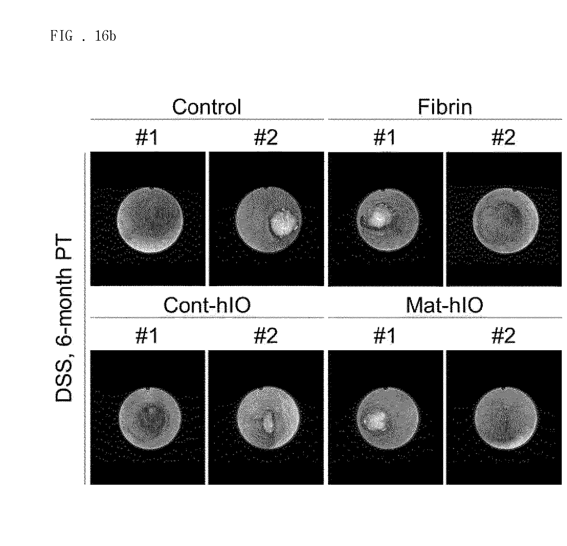

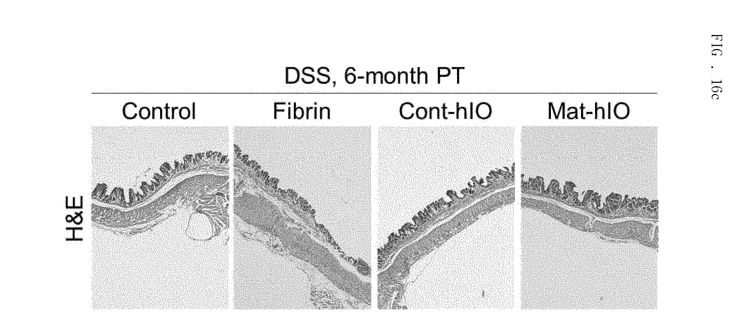

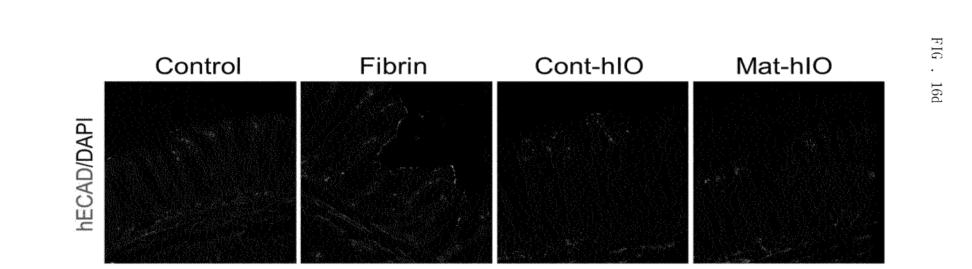

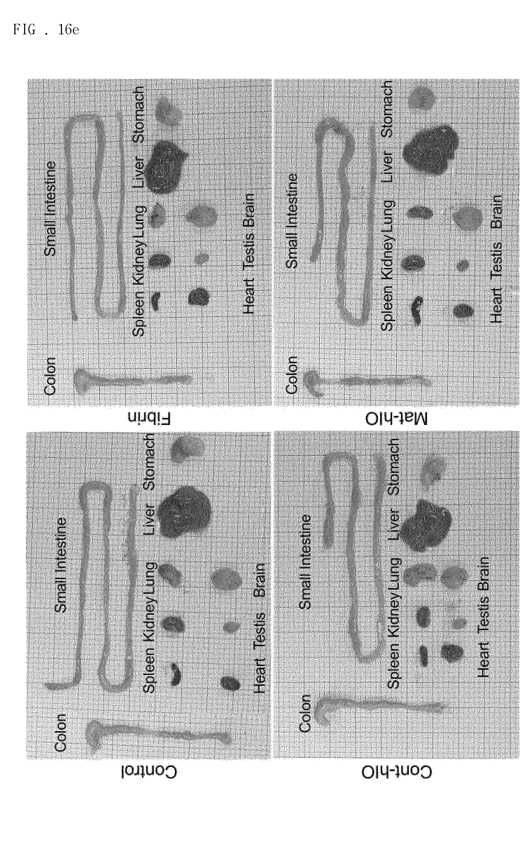

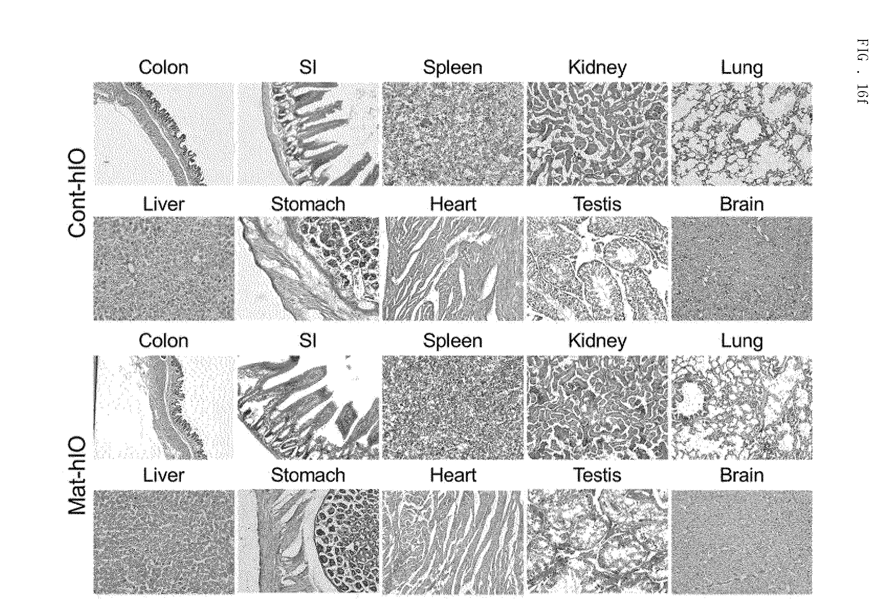

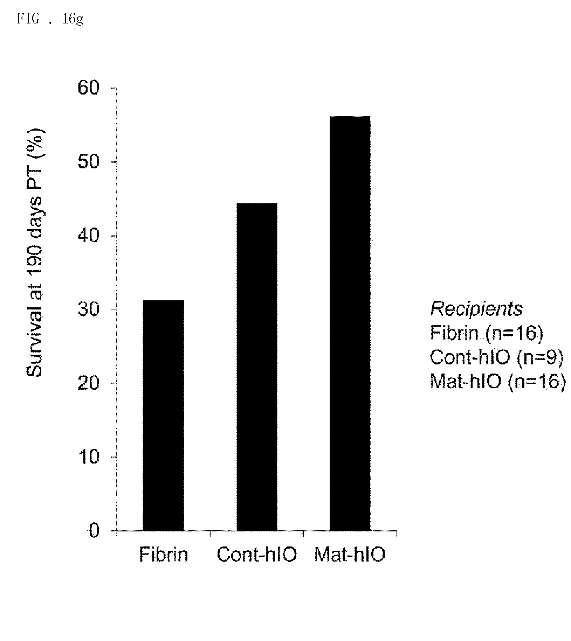

Four weeks after transplantation of intestinal organoids into the same DSS-induced inflammatory bowel disease model, observation of body weight changes, colonoscopy and histological staining confirmed that the Mat-hIO transplantation group had significant therapeutic effects (weight gain compared to day 1 of transplantation, no inflammation observed on endoscopy, recovery of histological structure and intestinal epithelial mucin secretion) (FIGS. 15a to 15d). Further, human-specific antibodies (hECAD) were used to demonstrate that the xenograft Mat-hIO still existed (FIG. 15e). Even months after intestinal organoid transplantation, colonoscopy confirmed no abnormal findings (mass findings) and no inflammation or epithelial injury (FIGS. 16a and 16b). Histological analysis using H&E confirmed that the crypt depth was similar in both the control and transplantation groups (Fibrin, Cont-hIO, and Mat-hIO), and human-specific antibody (hECAD) was used to verify that expression was no longer observed in the xenograft (FIGS. 16c and 16d). Visual inspection and histological examination of the 10 major organs (small intestine, spleen, kidney, lung, liver, stomach, heart, testis, and brain), including the graft colon, confirmed that the organoid transplantation group were identical to normal tissue with no ectopic tissue (FIGS. 16e and 16f). These results demonstrate that transplantation of pluripotent stem cell-derived intestinal organoids plays a role in engraftment and regeneration in an inflammatory bowel disease model while demonstrating safety. In particular, when the survival rate was compared among the transplantation groups (Fibrin, Cont-hIO, and Mat-hIO) at 6 months after transplantation, Mat-hIO showed a significantly higher survival rate of 56.3% than the other transplantation groups (Fibrin 31.3%, and Cont-hIO 44.4%) (FIG. 16g).

### Example 11. Isolation and culture of mesenchymal stromal cells from hPSC-derived hIOs

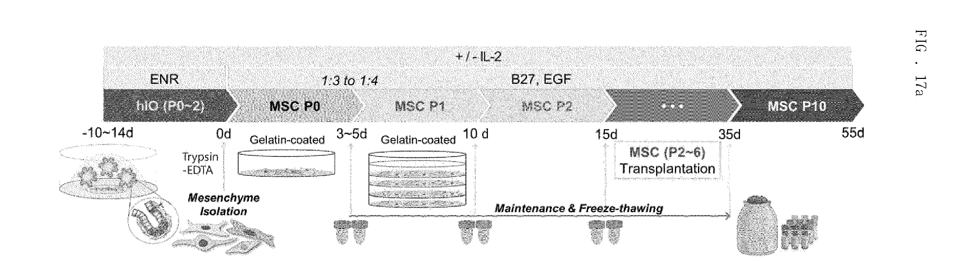

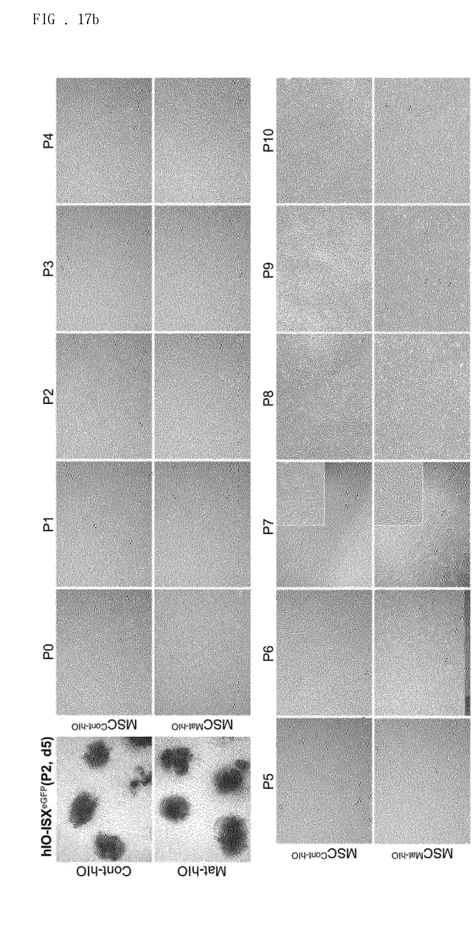

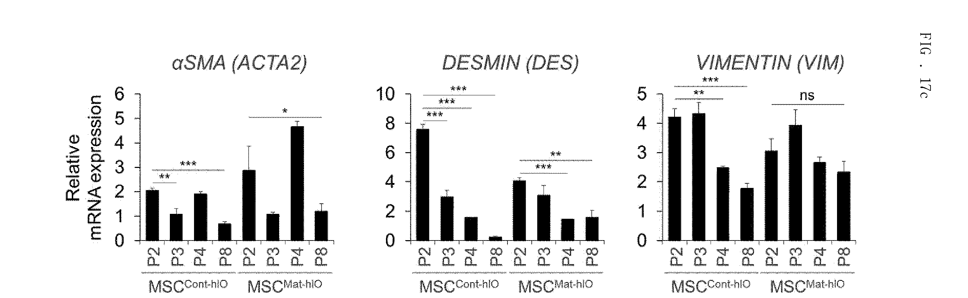

As described above, hIOs were produced from hPSCs containing hESC lines or non-integrating-hiPSC lines, using the hIO differentiation protocol. Then, mesenchymal stromal cells were isolated from mature organoids co-cultured with rhIL-2 (1 ng/ml) from P0 and conventional immature organoids P0 to P2, followed by freezing and thawing process during passage culture, which was shown as a schematic diagram (FIG. 17a). Mesenchymal stromal cells (P0) isolated from Cont-hIO and Mat-hIO (P2) were culturable in hIO medium containing 1X B27 and 100 ng/ml EGF and were maintained until P10 (FIG. 17b). In addition, it was confirmed that the expression of representative markers of mesenchymal stromal cells (αSMA, VIM, and DES) was still maintained during passage culture (FIG. 17c).

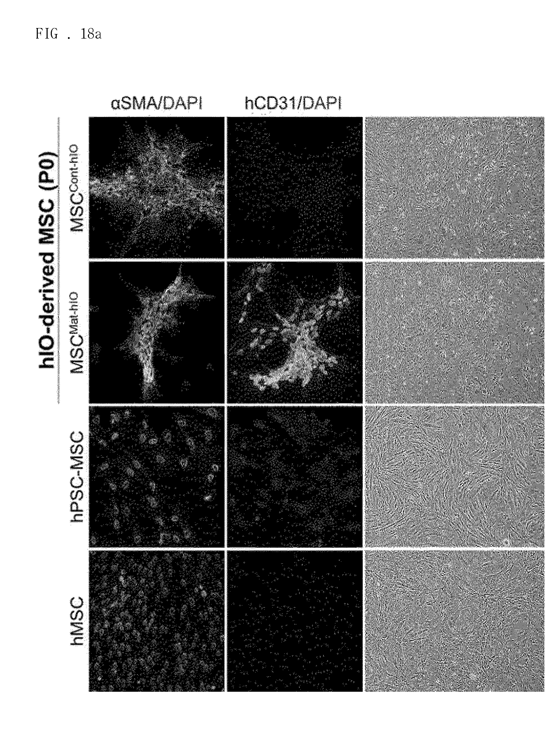

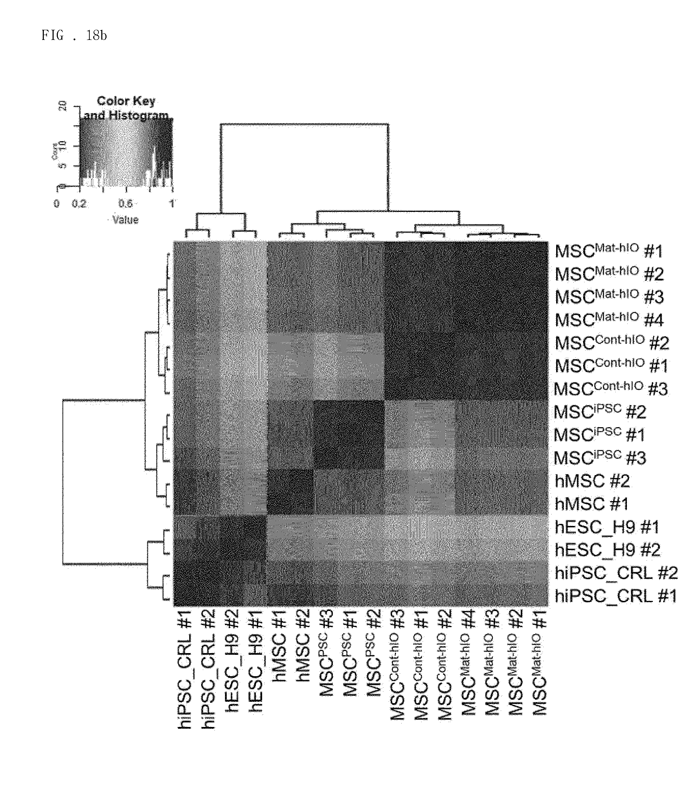

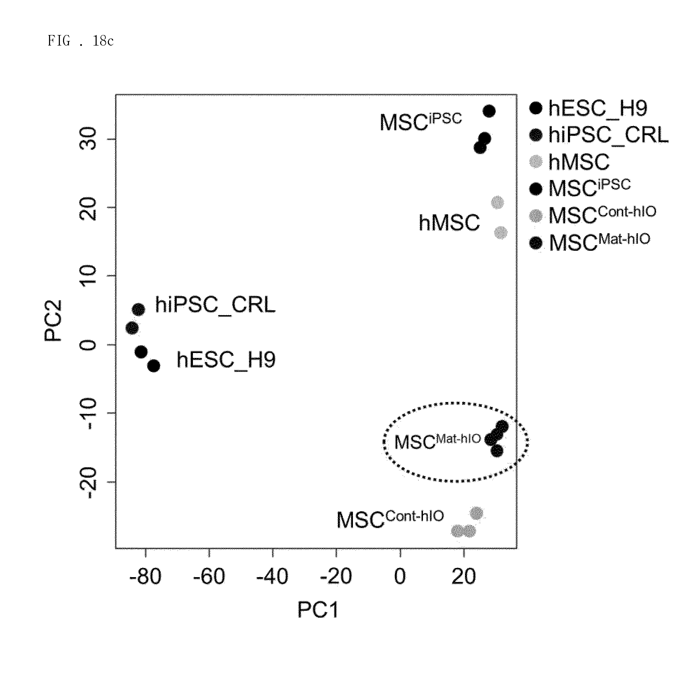

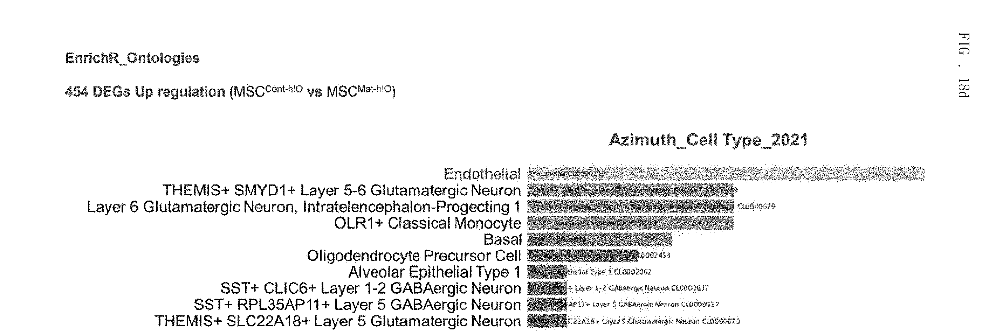

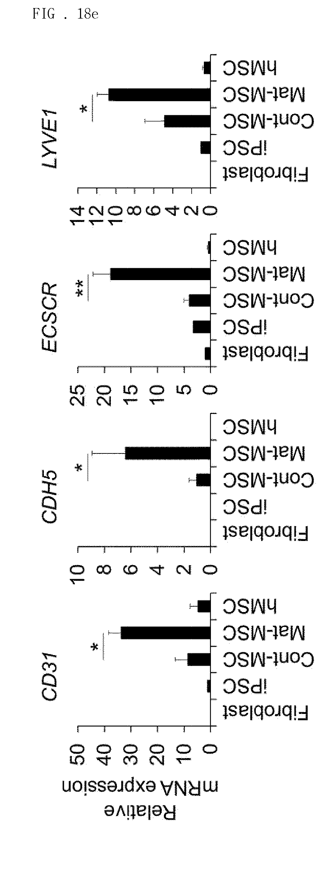

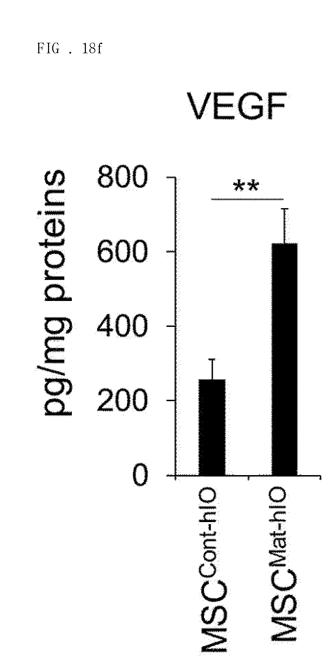

To closely examine the characteristics of the isolated mesenchymal stem cells, commercially available human mesenchymal stem cells and mesenchymal stem cells differentiated from induced pluripotent stem cells were used as control groups. Expression of αSMA, a marker of pericytes, was confirmed in all cells through immunofluorescence staining. However, in mature intestinal organoid-derived mesenchymal stromal cells, expression of the human vascular endothelial cell marker hCD31⁺ was observed, along with αSMA (FIG. 18a). For genome information analysis, RNA sequencing was performed to select differential genes between groups through DEG analysis while showing similarities between samples (FIGS. 18b and 18c). MDS plot confirmed the difference between Mat-hIO-derived MSCs and Cont-hIO-derived MSCs, and GOterm analysis of EnrichR identified endothelial cell type characteristics (FIG. 18d). The difference between the two groups was validated by qPCR to confirm the expression of angiogenic factors (CD31, CDH5, ECSCR, and LYVE1), and by ELISA to detect the vascular secretory factor, VEGF, revealing a novel characteristic of Mat-hIO-derived MSCs (FIGS. 18e and 18f).

These results confirm that Mat-hIO-derived MSCs have different characteristics from immature organoid-derived MSCs, commercially available human MSCs, and MSCs differentiated from induced pluripotent stem cells.

## Claims

1. A pluripotent stem cell-derived intestinal organoid comprising a stromal cell layer around an intestinal organoid,
wherein the stromal cell layer around the intestinal organoid comprises mesenchymal stromal cells.

2. The pluripotent stem cell-derived intestinal organoid comprising the stromal cell layer around the intestinal organoid of claim 1, wherein the stromal cell layer around the intestinal organoid further includes any one or more cells selected from the group consisting of smooth muscle cells, fibroblasts, myofibroblasts, telocytes, and pericytes.

3. The pluripotent stem cell-derived intestinal organoid comprising the stromal cell layer around the intestinal organoid of claim 1, wherein the pluripotent stem cell-derived intestinal organoid comprising the stromal cell layer around the intestinal organoid is cultured by treatment with any one or more factors selected from the group consisting of IL-2, IL-22, IL-6, IL-1β, IL-11, EGF, OSM, IL-10, NRG-1, and colivelin.

4. The pluripotent stem cell-derived intestinal organoid comprising the stromal cell layer around the intestinal organoid of claim 2, wherein the pluripotent stem cell-derived intestinal organoid is cultured by treatment with IL-2.

5. The pluripotent stem cell-derived intestinal organoid comprising the stromal cell layer around the intestinal organoid of claim 3, wherein the treatment with the factor is performed during differentiation into the intestinal organoid from the hindgut.

6. The pluripotent stem cell-derived intestinal organoid comprising the stromal cell layer around the intestinal organoid of claim 1, wherein the pluripotent stem cell-derived intestinal organoid comprising the stromal cell layer around the intestinal organoid, exhibits characteristics of a mature intestinal organoid.

7. The pluripotent stem cell-derived intestinal organoid comprising the stromal cell layer around the intestinal organoid of claim 6, wherein the mature intestinal organoid has higher expression of any one or more factors selected from the group consisting of ASCL2, AXIN2, LGR5, and OLFM4, compared to pluripotent stem cell-derived immature intestinal organoids.

8. The pluripotent stem cell-derived intestinal organoid comprising the stromal cell layer around the intestinal organoid of claim 6, wherein the mature intestinal organoid has higher expression of any one or more factors selected from the group consisting of VIL1, KRT20, CHGA, MUC13, LYZ, OLFM4, SI, LCT, and DPP4, compared to pluripotent stem cell-derived immature intestinal organoids.

9. The pluripotent stem cell-derived intestinal organoid comprising the stromal cell layer around the intestinal organoid of claim 6, wherein the mature intestinal organoid has higher expression of any one or more factors selected from the group consisting of hCD31, CDH5, ECSCR, LYVE1, IGF2, VEGF, ANGPT1, and KDR, known as angiogenic factors and/or vascular endothelium-related factors, compared to adult stem cell-derived organoids or pluripotent stem cell-derived immature intestinal organoids.

10. The pluripotent stem cell-derived intestinal organoid comprising the stromal cell layer around the intestinal organoid of claim 6, wherein the mature intestinal organoid has higher expression of any one or more factors selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, compared to adult stem cell-derived organoids or pluripotent stem cell-derived immature intestinal organoids.

11. The pluripotent stem cell-derived intestinal organoid comprising the stromal cell layer around the intestinal organoid of claim 6, wherein the mature intestinal organoid comprises hCD31⁺ vascular cells in the stromal cell layer around the intestinal organoid.

12. The pluripotent stem cell-derived intestinal organoid comprising the stromal cell layer around the intestinal organoid of claim 6, wherein the stromal cell layer around the intestinal organoid comprises mesenchymal stromal cells, and the mesenchymal stromal cells exhibit at least one characteristic selected from the following:
(a) an enhanced expression level of hCD31 (PECAM1) compared to adult-derived mesenchymal stem cells, pluripotent stem cell-derived mesenchymal stem cells and/or immature intestinal organoid-derived mesenchymal stromal cells;
(b) enhanced expression levels of factors related to cellular vascular endothelial stimulation, angiogenesis, cell adhesion, and differentiation, compared to adult-derived mesenchymal stem cells, pluripotent stem cell-derived mesenchymal stem cells, and/or immature intestinal organoid-derived mesenchymal stromal cells;
(c) enhanced expression levels of endothelial cell types, compared to adult-derived mesenchymal stem cells, pluripotent stem cell-derived mesenchymal stem cells, and/or immature intestinal organoid-derived mesenchymal stromal cells;
(d) enhanced expression levels of endothelial cell characteristic factors on Gene Ontology (GO) term enrichment, compared to adult-derived mesenchymal stem cells, pluripotent stem cell-derived mesenchymal stem cells, and/or immature intestinal organoid-derived mesenchymal stromal cells;
(e) enhanced expression levels of angiogenic factors, vascular endothelial-related factors or vascular endothelial cell-related factors, compared to adult-derived mesenchymal stem cells, pluripotent stem cell-derived mesenchymal stem cells, and/or immature intestinal organoid-derived mesenchymal stromal cells;
wherein the angiogenic factor, vascular endothelial-related factor or vascular endothelial cell-related factor is any one or more selected from the group consisting of hCD31, CDH5, ECSCR, LYVE1, IGF2, VEGF, ANGPT1 and KDR,
(f) enhanced expression levels of any one or more selected from the group consisting of IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, compared to adult-derived mesenchymal stem cells, pluripotent stem cell-derived mesenchymal stem cells, and/or immature intestinal organoid-derived mesenchymal stromal cells; and
(g) maintained expression levels of any one or more selected from the group consisting of αSMA, VIM and DESMIN at the time of subculture.

13. A method for producing a pluripotent stem cell-derived mature intestinal organoid comprising a stromal cell layer around an intestinal organoid, the method comprising:
(a) culturing pluripotent stem cells in a medium containing any one or more selected from the group consisting of Nodal, Activin A, Activin B, BMP4, Wnt3a, and bFGF to perform differentiation into definitive endoderm;
(b) culturing the definitive endoderm in a medium containing: any one or more GSK3 inhibitors selected from the group consisting of BIO (6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII(α,4-dibromoacetophenone), L803-mts(Myr-N-GKEAPPAPPQSpP-NH₂), and CHIR99021; and a fibroblast growth factor (FGF), to perform differentiation into hindgut; and
(c) culturing the hindgut in a medium containing Noggin; epidermal growth factor (EGF); any one or more Wnt agonists selected from the group consisting of R-spondin 1, R-spondin 2, R-spondin 3, and R-spondin 4; and any one or more factors selected from the group consisting of IL-2, IL-22, IL-6, IL-1β, IL-11, EGF, OSM, NRG-1, IL-10, and colivelin to produce a mature intestinal organoid.

14. The method of claim 13, wherein in Step (a), the medium contains any one selected from the group consisting of Activin A, BMP4, bFGF and Wnt3a.

15. The method of claim 13, wherein in Step (b), the medium contains CHIR99021 and FGF.

16. The method of claim 13, wherein in Step (c), the medium contains Noggin; epidermal growth factor (EGF); R-spondin; and IL-2.

17. The method of claim 13, wherein after differentiation into Hindgut in Step (b), in Step (c), any one or more factors selected from the group consisting of IL-2, IL-22, IL-6, IL-1β, IL-11, EGF, OSM, NRG-1, IL-10, and colivelin are immediately added.

18. The method of claim 13, wherein Step (c) is performed under Xeno-free conditions.

19. The method of claim 18, wherein it is cultured with VitroGel.

20. A pluripotent stem cell-derived mature intestinal organoid comprising a stromal cell layer around an intestinal organoid, produced by the method according to any one of claims 13 to 19.

21. An intestinal transplant material, comprising: the pluripotent stem cell-derived intestinal organoid comprising the stromal cell layer around the intestinal organoid according to any one of claims 1 to 12.

22. An intestinal transplant material, comprising: the pluripotent stem cell-derived mature intestinal organoid comprising the stromal cell layer around the intestinal organoid according to claim 20.

23. A pharmaceutical composition for preventing or treating an intestinal disease, comprising: the pluripotent stem cell-derived intestinal organoid comprising the stromal cell layer around the intestinal organoid according to any one of claims 1 to 12.

24. A pharmaceutical composition for preventing or treating an intestinal disease, comprising: the pluripotent stem cell-derived mature intestinal organoid comprising the stromal cell layer around the intestinal organoid according to claim 20.

25. The pharmaceutical composition of claim 23, wherein the intestinal disease is any one or more selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

26. The pharmaceutical composition of claim 24, wherein the intestinal disease is any one or more selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

27. The intestinal transplant material of claim 21, further comprising:
any one or more biodegradable supports selected from the group consisting of fibrin, laminin, collagen, gelatin, chitosan, alginate, hyaluronic acid, dextran, polylactic acid, poly(glycolic acid) (PGA), poly(lactic-co-glycolic acid) (PLGA), poly-ε-(caprolactone), polyanhydrides, polyorthoesters, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof.

28. The intestinal transplant material of claim 22, further comprising:
any one or more biodegradable supports selected from the group consisting of fibrin, laminin, collagen, gelatin, chitosan, alginate, hyaluronic acid, dextran, polylactic acid, poly(glycolic acid) (PGA), poly(lactic-co-glycolic acid) (PLGA), poly-ε-(caprolactone), polyanhydrides, polyorthoesters, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof.
